# NEW EUROPEAN PATENT SPECIFICATION

(11) **EP 3 478 719 B3**
(45) Date of publication of this specification: **18.12.2024**
(45) Mention of the grant of the patent: 20.01.2021
(21) Application number: 17823190.8
(22) Date of filing: 21.12.2017
(51) Int. Cl.: C07K 16/22, A61K 39/395, A61P 19/08, A61K 39/00

(54) **USE OF ANTI-SCLEROSTIN ANTIBODIES IN THE TREATMENT OF OSTEOGENESIS IMPERFECTA**
VERWENDUNG VON ANTI-SCLEROSTIN-ANTIKÖRPERN BEI DER BEHANDLUNG VON OSTEOGENESIS IMPERFECTA
UTILISATION D'ANTICORPS ANTI-SCLÉROSTINE DANS LE TRAITEMENT DE L'OSTÉOGENÈSE IMPARFAITE

(30) Priority: 21.12.2016 US 201662437353 P
(43) Date of publication of application: 08.05.2019
(60) Divisional application: 21152251.1
(73) Proprietor: Mereo Biopharma 3 Limited, London W1G 0QF (GB)
(72) Inventor: JUNKER, Uwe, 4070 Basel (CH); KNEISSEL, Michaela, 4002 Basel (CH); HALL, Anthony Kent, 2341 JB Oegstgeest (NL); EUDY, Rena Joy, West Hartford Connecticut 06117 (US); RIGGS, Matthew Manning, Haddam Connecticut 06438 (US)
(74) Representative: Carpmaels & Ransford LLP
(86) International application number: PCT/GB2017/053850
(87) International publication number: WO 2018/115880

(56) References cited:
- WO-A1-2009/047356
- WO-A1-2016/092101
- ROSCHGER ANDREAS ET AL: "Effect of sclerostin antibody treatment in a mouse model of severe osteogenesis imperfecta", BONE, PERGAMON PRESS., OXFORD, GB, vol. 66, 19 June 2014 (2014-06-19), pages 182 - 188, XP029013400, ISSN: 8756-3282, DOI: 10.1016/J.BONE.2014.06.015
- CLAIRE MACNABB ET AL: "Sclerostin Antibody Therapy for the Treatment of Osteoporosis: Clinical Prospects and Challenges", JOURNAL OF OSTEOPOROSIS, vol. 2016, 26 May 2016 (2016-05-26), pages 1 - 22, XP055449405, ISSN: 2090-8059, DOI: 10.1155/2016/6217286
- FRANCIS H GLORIEUX ET AL: "BPS804 Anti-Sclerostin Antibody in Adults With Moderate Osteogenesis Imperfecta: Results of a Randomized Phase 2a Trial : BPS804 ANTI-SCLEROSTIN ANTIBODY IN ADULTS WITH MODERATE OI", JOURNAL OF BONE AND MINERAL RESEARCH, vol. 32, no. 7, 19 April 2017 (2017-04-19), US, pages 1496 - 1504, XP055448588, ISSN: 0884-0431, DOI: 10.1002/jbmr.3143

## Description

### FIELD OF INVENTION

This invention relates to antibodies and their use as pharmaceutical compositions, more specifically to the use of anti-sclerostin antibodies in the treatment of osteogenesis imperfecta.

### BACKGROUND

Osteogenesis imperfecta (OI) is a rare genetic disorder of the connective tissue characterized by bone fragility and reduced bone mass. OI comprises a group of inherited disorders which primarily, but not always, arise from mutations in the genes encoding type I collagen. About 85% of the cases are linked to mutations in one of the two genes encoding type I collagen (COL1A1 and COL1A2). Clinically, OI is characterized by fragile bones that fracture easily and without any trauma. In addition, patients with OI are often affected by muscle weakness, hearing loss, fatigue, joint laxity, curved bones, scoliosis, blue sclerae, dentinogenesis imperfecta, and short stature.

The clinical classification system divides OI into types I - V. Type I OI patients usually suffer from a mild non-deforming disease that is often associated with a premature stop codon in COL1A1. This defect results in a reduced rate of type I collagen production and quantitatively less collagen in bone.. Patients with type II OI usually die during the perinatal period, as a result of respiratory failure from multiple severe fractures that include the rib cage. Types III and IV OI are often associated with glycine substitution in COL1A1 and COL1A2, which is a qualitative defect that prevents the 3 polypeptide chains of type I collagen to intertwine properly to form a normal triple alpha helical structure. Type III OI is the most severe form of OI in those affected children who survive infancy, whereas patients with type IV have mild to moderate bone deformities. Type V OI is infrequent. MacNabb et al. (Journal of Osteoporosis, 2016) describes sclerostin antibody therapy for the treatment of osteoporosis. WO 2009/047356 A1 describes compositions and methods for use for antibodies against sclerostin. WO 2016/092101 describes anti-sclerostin antibodies and their use to treat bone disorders as part of a regimen. Roschger et al. (Bone, 2014) describes effect of sclerostin antibody treatment in a mouse model of severe osteogenesis impefecta.

There remains a need for therapeutic methods and agents for the treatment of OI.

### BRIEF SUMMARY OF THE INVENTION

The inventors surprisingly found that human patients with osteogenesis imperfecta (OI) can successfully be treated with an anti-sclerostin antibody, as established by the examples, which confirm that an anti-sclerostin antibody can both increase bone formation and reduce bone resorption in patients with OI. We disclosed high affinity, neutralizing, fully human anti-sclerostin monoclonal antibodies (collectively "the human anti-sclerostin monoclonal antibody") and their potent in vitro activity and in vivo activity in our US Patent Nos. 7,879,322, 8,246,953, and 8,486,661.

In one aspect, the invention provides an anti-sclerostin antibody for use in treating osteogenesis imperfecta (OI) in a human patient comprising administering to the human patient a therapeutically effective amount of the anti-sclerostin antibody, wherein: (i) the therapeutically effective amount of the anti-sclerostin antibody is 20 mg per kg body weight of the human patient administered on a monthly basis; and (ii) the anti-sclerostin antibody comprises: (a) a heavy chain variable region CDR1 comprising an amino acid sequence set forth in SEQ ID NO:4; (b) a heavy chain variable region CDR2 comprising an amino acid sequence set forth in SEQ ID NO: 15; (c) a heavy chain variable region CDR3 comprising an amino acid sequence set forth in SEQ ID NO:26; (d) a light chain variable region CDR1 comprising an amino acid sequence set forth in SEQ ID NO:37; (e) a light chain variable region CDR2 comprising an amino acid sequence set forth in SEQ ID NO:48; and (f) a light chain variable region CDR3 comprising an amino acid sequence set forth in SEQ ID NO: 59.

In another aspect, the invention provides a pharmaceutical composition comprising an anti-sclerostin antibody as defined in the invention for use according to the invention.

Disclosed herein is a method for treating a patient suffering from osteogenesis imperfecta comprising administering to the patient a therapeutically effective amount of an anti-sclerostin antibody. In one instance, disclosed herein is a method for increasing bone formation and reducing bone resorption in an osteogenesis imperfecta patient by administering to the patient a therapeutically effective amount of an anti-sclerostin antibody. Disclosed herein is an anti-sclerostin antibody for use in the treatment of osteogenesis imperfecta. The following embodiments apply to both said aspects. In one embodiment the anti-sclerostin antibody increases bone formation and/or reduces bone resorption. In one embodiment, the anti- sclerostin antibody comprises: a) at least one immunoglobulin heavy chain variable domain (VH) which comprises in sequence hypervariable regions a heavy chain variable region CDR1, a heavy chain variable region CDR2, and a heavy chain variable region CDR3, said heavy chain variable region CDR1 comprising an amino acid sequence set forth in SEQ ID NO: 4, said heavy chain variable region CDR2 comprising an amino acid sequence set forth in SEQ ID NO: 15, and said heavy chain variable region CDR3 comprising an amino acid sequence set forth in SEQ ID NO: 26; and b) at least one immunoglobulin light chain variable domain (VL) which comprises in sequence hypervariable regions a light chain variable region CDR1, a light chain variable region CDR2, and a light chain variable region CDR3, said light chain variable region CDR1 comprising an amino acid sequence set forth in SEQ ID NO: 37, said light chain variable region CDR2 comprising an amino acid sequence set forth in SEQ ID NO: 48, and said light chain variable region CDR3 comprising an amino acid sequence set forth in SEQ ID NO: 59. In one instance, the anti-sclerostin antibody comprises at least one or more complementarity determining region (CDR) sequences selected from the group consisting of: (a) heavy chain variable region CDR1 comprising an amino acid sequence set forth in SEQ ID NO:4; (b) heavy chain variable region CDR2 comprising an amino acid sequence set forth in SEQ ID NO: 15; (c) heavy chain variable region CDR3 comprising an amino acid sequence set forth in SEQ ID NO:26; (d) light chain variable region CDR1 comprising an amino acid sequence set forth in SEQ ID NO:37; (e) light chain variable region CDR2 comprising an amino acid sequence set forth in SEQ ID NO:48; and (f) light chain variable region CDR3 comprising an amino acid sequence set forth in SEQ ID NO:59. In one instance, the anti-sclerostin antibody comprises at least the heavy chain variable region CDR3 comprising an amino acid sequence set forth in SEQ ID NO: 26. In one embodiment, the anti-sclerostin antibody comprises all 6 of the aforementioned CDRs.

In another embodiment, the anti-sclerostin antibody comprises:(a) a heavy chain variable region CDR1 comprising an amino acid sequence set forth in SEQ ID NO:4; (b) a heavy chain variable region CDR2 comprising an amino acid sequence set forth in SEQ ID NO: 15; (c) a heavy chain variable region CDR3 comprising an amino acid sequence set forth in SEQ ID NO:26; (d) a light chain variable region CDR1 comprising an amino acid sequence set forth in SEQ ID NO:37; (e) a light chain variable region CDR2 comprising an amino acid sequence set forth in SEQ ID NO:48; and (f) a light chain variable region CDR3 comprising an amino acid sequence set forth in SEQ ID NO:59. In one instance, the anti-sclerostin antibody binds to the same epitope as an anti-sclerostin antibody comprising: (a) a heavy chain variable region CDR1 comprising an amino acid sequence set forth in SEQ ID NO:4; (b) a heavy chain variable region CDR2 comprising an amino acid sequence set forth in SEQ ID NO: 15; (c) a heavy chain variable region CDR3 comprising an amino acid sequence set forth in SEQ ID NO:26; (d) a light chain variable region CDR1 comprising an amino acid sequence set forth in SEQ ID NO:37; (e) a light chain variable region CDR2 comprising an amino acid sequence set forth in SEQ ID NO:48; and (f) a light chain variable region CDR3 comprising an amino acid sequence set forth in SEQ ID NO:59. In a related instance, the anti-sclerostin antibody binds to the same epitope as an anti-sclerostin antibody comprising a VL polypeptide sequence having the amino acid sequences set forth as SEQ ID NO:81 and a VH polypeptide sequence having a the amino acid sequences set forth SEQ ID NO:70. In another instance, the anti-sclerostin antibody binds to the same epitope as an anti-sclerostin antibody comprising a full length light chain amino acid sequence having the amino acid sequence set forth as SEQ ID NO:125 and a full length heavy chain amino acid sequence having the amino acid sequence set forth as SEQ ID NO:114. In one instance, the anti-sclerostin antibody binds to the same epitope as an anti-sclerostin antibody comprising a full length light chain amino acid sequence having the amino acid sequence set forth as SEQ ID NO:173 and a full length heavy chain amino acid sequence having the amino acid sequence set forth as SEQ ID NO:172.

In one instance, the anti-sclerostin antibody binds to an epitope within the following human sclerostin sequences: CGPARLLPNAIGRGKWWRPSGPDFRC (the "loop 2 epitope"; SEQ ID NO: 174) and/or DVSEYCRELHFTR SAKPVTELVCSGQCGPAR WWRPSGPDFRCIPDRYR LVASCKCKRLTR (the "T20.6 epitope" SEQ ID NO: 175). Thus, in one instance the anti-sclerostin antibody binds to/ within the loop 2 epitope. In another instance, the anti sclerostin antibody binds to/ within the T20.6 epitope. Anti-sclerostin antibodies that bind to/ within the loop 2 and T20.6 epitope regions are known in the art - see, for example, WO00/32773, WO2005/003158, WO2005/014650, WO2006/119107, WO2006/119062, WO2009/039175 and WO2008/061013. Antibodies 'Ab-A', 'Ab-B', 'Ab-C', 'Ab-D', and Ab-1 disclosed in WO2006/119062. In one instance the anti-sclerostin antibody binds to the same epitope as an anti-sclerostin antibody comprising a VL polypeptide sequence having the amino acid sequences set forth as SEQ ID NO:176 and a VH polypeptide sequence having the amino acid sequences set forth SEQ ID NO:177. In another instance, the anti-sclerostin antibody binds to the same epitope as Ab-A, Ab-B, Ab-C or Ab-D disclosed in WO2006/119062.

In one instance, the anti-sclerostin antibody is selected from blosozumab.

In another embodiment, the anti-sclerostin antibody comprises a VH having at least 90 (such as at least 95, 98, or 99 or 100) percent identity to the amino acid sequence set forth in SEQ ID NO: 70. In yet another embodiment, the anti-sclerostin antibody comprises a VL having at least 90 (such as at least 95, 98, or 99 or 100) percent identity to the amino acid sequence set forth in SEQ ID NO: 81. In still another embodiment, the anti- sclerostin antibody comprises a VH having at least 90 (such as at least 95, 98, or 99 or 100) percent identity to the amino acid sequence set forth in SEQ ID NO: 70, and a VL having at least 90 (such as at least 95, 98, or 99 or 100) percent identity to the amino acid sequence set forth in SEQ ID NO: 81. In yet another embodiment, the anti-sclerostin antibody comprises a VH having the amino acid sequence set forth in SEQ ID NO: 70, and a VL having the amino acid sequence set forth in SEQ ID NO: 81. In yet still another embodiment, the anti-sclerostin antibody comprises a heavy chain having at least 90 (such as at least 95, 98, or 99 or 100) percent identity to the amino acid sequence set forth in SEQ ID NO: 114 or 172, and/ or at least 90 (such as at least 95, 98, or 99 or 100) percent identity to a light chain having the amino acid sequence set forth in SEQ ID NO: 125 or 173. In one embodiment, the anti-sclerostin antibody comprises a heavy chain having the amino acid sequence set forth in SEQ ID NO: 172 and a light chain having the amino acid sequence set forth in SEQ ID NO: 173.

In one embodiment, the therapeutically effective amount of the anti-sclerostin antibody is about 1-50 mg of said antibody per kg body weight of the human patient.

Another aspect of the invention is a pharmaceutical composition for increasing bone formation and reducing bone resorption in an osteogenesis imperfecta patient, wherein the pharmaceutical composition comprises an anti-sclerostin antibody, wherein the anti-sclerostin antibody comprises: (a) a heavy chain variable region CDR1 comprising an amino acid sequence set forth in SEQ ID NO:4; (b) a heavy chain variable region CDR2 comprising an amino acid sequence set forth in SEQ ID NO: 15; (c) a heavy chain variable region CDR3 comprising an amino acid sequence set forth in SEQ ID NO:26; (d) a light chain variable region CDR1 comprising an amino acid sequence set forth in SEQ ID NO: 37; (e) a light chain variable region CDR2 comprising an amino acid sequence set forth in SEQ ID NO:48; and (f) a light chain variable region CDR3 comprising an amino acid sequence set forth in SEQ ID NO:59. In some embodiments, the composition contains an anti-sclerostin antibody comprising: a) at least one immunoglobulin heavy chain variable domain (VH) which comprises in sequence hypervariable regions a heavy chain variable region CDR1, a heavy chain variable region CDR2, and a heavy chain variable region CDR3, said heavy chain variable region CDR1 comprising an amino acid sequence set forth in SEQ ID NO: 4, said heavy chain variable region CDR2 comprising an amino acid sequence set forth in SEQ ID NO: 15, and said heavy chain variable region CDR3 comprising an amino acid sequence set forth in SEQ ID NO: 26; and b) at least one immunoglobulin light chain variable domain (VL) which comprises in sequence hypervariable regions a light chain variable region CDR1, a light chain variable region CDR2, and a light chain variable region CDR3, said light chain variable region CDR1 comprising an amino acid sequence set forth in SEQ ID NO: 37, said light chain variable region CDR2 comprising an amino acid sequence set forth in SEQ ID NO: 48, and said light chain variable region CDR3 comprising an amino acid sequence set forth in SEQ ID NO: 59.

In another embodiment, the anti-sclerostin antibody in the pharmaceutical composition comprises a VH having at least 95 percent identity to the amino acid sequence set forth in SEQ ID NO: 70. In yet another embodiment, the anti-sclerostin antibody comprises a VL having at least 95 percent identity to the amino acid sequence set forth in SEQ ID NO: 81.

In still another embodiment, the anti-sclerostin antibody comprises a VH having at least 95 percent identity to the amino acid sequence set forth in SEQ ID NO: 70, and a VL having at least 95 percent identity to the amino acid sequence set forth in SEQ ID NO: 81. In yet another embodiment, the anti-sclerostin antibody comprises a VH having the amino acid sequence set forth in SEQ ID NO: 70, and a VL having the amino acid sequence set forth in SEQ ID NO: 81. In yet still another embodiment, the anti-sclerostin antibody comprises a heavy chain having the amino acid sequence set forth in SEQ ID NO: 114 or 172, and/or a light chain having the amino acid sequence set forth in SEQ ID NO: 125 or 173.

In one aspect the invention provides a pharmaceutical composition comprising an anti-sclerostin antibody as defined in the invention for use in treating OI according to the invention. In one instance the pharmaceutical composition can be used in the methods/ uses described herein

### BRIEF DESCRIPTION OF THE DRAWINGS

Figure 1 shows a table with results of the one-sample t-test analysis results for the bone metabolism biomarkers.
Figure 2 shows a table with results of the two-sample t-test analysis of the bone metabolism biomarkers.
Figure 3 shows a graph depicting ratios to baseline in geometric means (plus or minus SD) for PINP.
Figure 4 shows a graph depicting ratios to baseline in geometric means (plus or minus SD) for PICP.
Figure 5 shows a graph depicting ratios to baseline in geometric means (plus or minus SD) for BSAP.
Figure 6 shows a graph depicting ratios to baseline in geometric means (plus or minus SD) for OC.
Figure 7 shows a graph depicting Bayesian posterior probabilities of ratios to baseline for BMD of lumbar spine (measured by DXA) data.
Figure 8 shows a graph depicting ratios of baseline in geometric means (plus or minus SD) for CTX-1.

### DETAILED DESCRIPTION

The present invention is based on the unexpected and surprising findings that human patients with osteogenesis imperfecta (OI) can successfully be treated with an anti-sclerostin antibody. Administration of an anti-sclerostin antibody to human OI patients increases bone formation and reduces bone resorption, as shown in the examples.

By way of background, sclerostin is a naturally occurring protein that in humans is encoded by the SOST gene. Sclerostin is a secreted glycoprotein with a C terminal cysteine knot-like (CTCK) domain and sequence similarity to the DAN (differential screening-selected gene aberrative in neuroblastoma) family of bone morphogenetic protein (BMP) antagonists.

As shown for the first time by the present inventors, inhibiting sclerostin with anti-sclerostin antibodies boosts bone formation and density and provides beneficial effects in treating OI in humans. Unlike previous anabolic treatments using GH or PTH which increase bone turnover, a treatment with an anti-sclerostin antibody offers the advantage of stimulating bone formation while inhibiting bone resorption in OI patients. The clinical utility of anti-sclerostin antibodies therefore includes the treatment of osteogenesis imperfecta in humans where bone formation is of therapeutic benefit in a disease characterized by a significant lack of bone matrix.

As such, the disclosure is directed to methods of using anti-sclerostin antibodies in the treatment of OI, as well as compositions comprising anti-sclerostin antibodies for use in treating OI. In one embodiment the invention is concerned with the treatment of osteogenesis imperfecta types I, III or IV. Accordingly, in one embodiment anti-sclerostin antibodies of the invention and pharmaceutical compositions comprising said antibodies are for use in the treatment of OI, preferably OI types I, III or IV. In one embodiment, the anti-sclerostin antibody of the invention is a monoclonal anti-sclerostin antibody. In one embodiment the anti-sclerostin antibody of the invention is a human or humanized monoclonal anti-sclerostin antibody. Alternatively, the antibody can be, for example, a chimeric antibody.

The uses of the anti-sclerostin antibody in the present invention were unexpected and surprising because the antibody treatment in OI patients results in both an increase of bone formation and a reduction of bone resorption.

In order that the present invention may be more readily understood, certain terms are first defined. Additional definitions are set forth throughout the detailed description.

The term "comprising" encompasses "including" as well as "consisting" e.g. a composition "comprising" X may consist exclusively of X or may include something additional e.g. X+Y.

The term "about" in relation to a numerical value x means, for example, x±10%.

The term sclerostin refers to human sclerostin as defined in SEQ ID NO: 155. Recombinant human sclerostin can be obtained from R&D Systems (Minneapolis, Minn., USA; 2006 cat# 1406-ST-025). Additionally, recombinant mouse sclerostin/SOST is commercially available from R&D Systems (Minneapolis, Minn., USA; 2006 cat# 1589-ST-025). U.S. Pat. Nos. 6,395,511 and 6,803,453, and U.S. Patent Publications 20040009535 and 20050106683 refer to anti-sclerostin antibodies in general.

The term "antibody" as referred to herein includes whole antibodies and any antigen binding fragment (i.e., "antigen-binding portion") or single chains thereof. A naturally occurring "antibody" is a glycoprotein comprising at least two heavy (H) chains and two light (L) chains interconnected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. In one embodiment, reference to an antibody herein embraces isolated, monoclonal, human and humanized monoclonal antibodies.

The term "antigen-binding portion" of an antibody (or simply "antigen portion"), as used herein, refers to full length or one or more fragments of an antibody that retain the ability to specifically bind to an antigen (e.g., sclerostin). It has been shown that the antigen-binding function of an antibody can be performed by fragments of a full-length antibody. Examples of binding fragments encompassed within the term "antigen-binding portion" of an antibody include a Fab fragment, a monovalent fragment consisting of the VL, VH, CL and CH1 domains; a F(ab)₂ fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., 1989 Nature 341 :544-546), which consists of a VH domain; and an isolated complementarity determining region (CDR).

Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al., 1988 Science 242:423-426; and Huston et al., 1988 Proc. Natl. Acad. Sci. 85:5879-5883). Such single chain antibodies are also intended to be encompassed within the term "antigen-binding region" of an antibody. These antibody fragments are obtained using conventional techniques known to those of skill in the art, and the fragments are screened for utility in the same manner as are intact antibodies.

An "isolated antibody", as used herein, refers to an antibody that is substantially free of other antibodies having different antigenic specificities (e.g., an isolated antibody that specifically binds sclerostin is substantially free of antibodies that specifically bind antigens other than sclerostin). An isolated antibody that specifically binds sclerostin may, however, have cross-reactivity to other antigens, such as sclerostin molecules from other species. Moreover, an isolated antibody may be substantially free of other cellular material and/or chemicals. In one embodiment, reference to an antibody herein means an isolated antibody.

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis as described in Knappik, et al. (2000. J Mol Biol 296, 57-86).

The human antibodies may include amino acid residues not encoded by human sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "human monoclonal antibody" refers to antibodies displaying a single binding specificity which have variable regions in which both the framework and CDR regions are derived from human sequences. In one embodiment, the human monoclonal antibodies are produced by a hybridoma which includes a B cell obtained from a transgenic nonhuman animal, e.g., a transgenic mouse, having a genome comprising a human heavy chain transgene and a light chain transgene fused to an immortalized cell.

The term "recombinant human antibody", as used herein, includes all human antibodies that are prepared, expressed, created or isolated by recombinant means, such as antibodies isolated from an animal (e.g., a mouse) that is transgenic or transchromosomal for human immunoglobulin genes or a hybridoma prepared therefrom, antibodies isolated from a host cell transformed to express the human antibody, e.g., from a transfectoma, antibodies isolated from a recombinant, combinatorial human antibody library, and antibodies prepared, expressed, created or isolated by any other means that involve splicing of all or a portion of a human immunoglobulin gene, sequences to other DNA sequences. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

In one embodiment, and as used herein, an antibody that binds sclerostin (e.g. an anti-sclerostin antibody) means that it specifically binds to sclerostin polypeptide. "Specifically binds to sclerostin polypeptide" is intended to refer to an antibody that binds to sclerostin polypeptide with a K_{D} of 1 × 10⁻⁸ M or less, 1 × 10⁻⁹ M or less, or 1 × 10⁻¹⁰ M or less. The term "K_{D}", as used herein, is intended to refer to the dissociation constant, which is obtained from the ratio of K_{d} to Kₐ (i.e. K_{d}/Kₐ) and is expressed as a molar concentration (M). K_{D} values for antibodies can be determined using methods well established in the art. A method for determining the K_{D} of an antibody is by using surface plasmon resonance, or using a biosensor system such as a Biacore^{®} system.

Standard assays to evaluate the binding ability of the antibodies toward sclerostin of various species are known in the art, including for example, ELISAs, western blots and RIAs. Suitable assays are described in detail in WO2009/047356. The binding kinetics (e.g., binding affinity) of the antibodies also can be assessed by standard assays known in the art, such as by Biacore analysis. Assays to evaluate the effects of the antibodies on functional properties of sclerostin (e.g., receptor binding, preventing or ameliorating osteolysis) are described in further detail in WO2009/047356.

As used herein, the percent identity between two sequences is a function of the number of identical positions shared by the sequences (i.e., % identity = # of identical positions/total # of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4: 11-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453, 1970) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6.

Additionally or alternatively, the protein sequences disclosed herein can further be used as a "query sequence" to perform a search against public databases to, for example, identify related sequences. Such searches can be performed using the XBLAST program (version 2.0) of Altschul, et al., 1990 J.Mol. Biol. 215:403-10. BLAST protein searches can be performed with the XBLAST program, score = 50, wordlength = 3 to obtain amino acid sequences homologous to the antibody molecules of the invention. To obtain gapped alignments for comparison purposes, Gapped BLAST can be utilized as described in Altschul et al., 1997 Nucleic Acids Res. 25(17):3389-3402. When utilizing BLAST and Gapped BLAST programs, the default parameters of the respective programs (e.g., XBLAST and NBLAST) can be used. See http:www.ncbi.nhn.nih.gov.

Various aspects of the invention are described in further detail in the following subsections.

### Methods of Treatment with and Uses of Anti-Sclerostin Antibodies in OI Treatment

Disclosed herein are methods of treating OI patients with anti-sclerostin antibodies or uses of anti-sclerostin antibodies in OI treatment. The treatment with anti-sclerostin antibodies may both increase bone formation and reduce bone resorption in the OI patients. In one aspect, the invention provides an anti-sclerostin antibody of the invention for use in the treatment of OI according to the invention. Suitable anti-sclerostin antibodies for use in the treatment of OI are disclosed herein.

Disclosed herein is a method for increasing bone formation and reducing bone resorption in an OI patient and the method comprises administering to the patient a therapeutically effective amount of an anti-sclerostin antibody. In one embodiment, treatment of an OI patient with an anti-sclerostin antibody of the invention increases bone formation and/or reduces bone resorption in said OI patient.

In one instance, the VH amino acid sequences of the anti-sclerostin antibody are shown in SEQ ID NOs: 69-77. The VL amino acid sequences of the anti-sclerostin antibody are shown in SEQ ID NOs: 80-88 respectively. The corresponding full length heavy chain amino acid sequences of antibodies of the anti-sclerostin antibody are shown in SEQ ID NO: 113-121. The corresponding full length light chain amino acid sequences of antibodies of the anti-sclerostin antibody are shown in SEQ ID NO: 124-132 respectively. In another instance, the anti-sclerostin antibody includes amino acids that have been mutated, yet have at least 60, 70, 80, 90 or 95 percent or more identity in the CDR regions with the CDR regions depicted in the sequences described above. In yet another instance, the anti-sclerostin antibody includes mutant amino acid sequences wherein no more than 1, 2, 3, 4 or 5 amino acids have been mutated in the CDR regions when compared with the CDR regions depicted in the sequence described above.

In some instances, variable heavy chain parental nucleotide sequences of the anti-sclerostin antibody are shown in SEQ ID NOs 89-90. Variable heavy chain parental nucleotide sequences of the anti-sclerostin antibody are shown in SEQ ID NOs 100-101. Full length light chain nucleotide sequences optimized for expression in a mammalian cell are shown in SEQ ID NOs 146-154. Full length heavy chain nucleotide sequences optimized for expression in a mammalian cell are shown in SEQ ID NOs 135-143. Full length light chain amino acid sequences encoded by optimized light chain nucleotide sequences are shown in SEQ ID NOs 124-132. Full length heavy chain amino acid sequences encoded by optimized heavy chain nucleotide sequences are shown in SEQ ID NOs 113-121. In other instances, the anti-sclerostin antibody include amino acids or nucleic acids that have been mutated, yet have at least 60, 70, 80, 90 or 95 percent or more identity to the sequences described above. In some instances, the anti-sclerostin antibody includes mutant amino acid sequences wherein no more than 1, 2, 3, 4 or 5 amino acids have been mutated in the variable regions when compared with the variable regions depicted in the sequence described above, while retaining substantially the same therapeutic activity.

In other instances, the above VH, VL, full length light chain, and full length heavy chain sequences (nucleotide sequences and amino acid sequences) can be "mixed and matched". Sclerostin binding of such "mixed and matched" antibodies can be tested using the binding assays described above and in the Examples (e.g., ELISAs). When these chains are mixed and matched, a VH sequence from a particular VH/VL pairing should be replaced with a structurally similar VH sequence. Likewise a full length heavy chain sequence from a particular full length heavy chain/full length light chain pairing should be replaced with a structurally similar full length heavy chain sequence. Likewise, a VL sequence from a particular VH/VL pairing should be replaced with a structurally similar VL sequence. Likewise a full length light chain sequence from a particular full length heavy chain/full length light chain pairing should be replaced with a structurally similar full length light chain sequence. Accordingly, disclosed herein isan isolated monoclonal antibody or antigen binding region thereof having: a heavy chain variable region comprising an amino acid sequence selected from the group consisting of SEQ ID NOs: 69-77; and a light chain variable region comprising an amino acid sequence selected from the group consisting of SEQ ID NOs: 80-88; wherein the antibody specifically binds to sclerostin.

In some instance, the anti-sclerostin antibody is (i) an isolated monoclonal antibody having: a full length heavy chain comprising an amino acid sequence that has been optimized for expression in the cell of a mammal selected from the group consisting of SEQ ID NOs: 113-121; and a full length light chain comprising an amino acid sequence that has been optimized for expression in the cell of a mammal selected from the group consisting of SEQ ID NOs: 124-132; or (ii) a functional protein comprising an antigen binding portion thereof.

In other instances, the anti-sclerostin antibody is (i) an isolated monoclonal antibody having: a full length heavy chain comprising a nucleotide sequence that has been optimized for expression in the cell of a mammal selected from the group consisting of SEQ ID NOs:135-143; and a full length light chain comprising a nucleotide sequence that has been optimized for expression in the cell of a mammal selected from the group consisting of SEQ ID NOs: 146-154; or (ii) a functional protein comprising an antigen binding portion thereof.

The anti-sclerostin antibody disclosed herein comprises the heavy chain and light chain CDR1s, CDR2s and CDR3s of the antibodies, or combinations thereof. The amino acid sequences of the VH CDR1s of the antibodies are shown in SEQ ID NOs: 1-11. The amino acid sequences of the VH CDR2s of the antibodies are shown in SEQ ID NOs: 12-22. The amino acid sequences of the VH CDR3s of the antibodies are shown in SEQ ID NOs: 23-33. The amino acid sequences of the VL CDR1s of the antibodies are shown in SEQ ID NOs: 34-44. The amino acid sequences of the VL CDR2s of the antibodies are shown in SEQ ID NOs: 45-55. The amino acid sequences of the VL CDR3s of the antibodies are shown in SEQ ID NOs: 56-66. The CDR regions are delineated using the Kabat system (Kabat, E. A., et al., 1991 Sequences of Proteins of Immunological Interest, Fifth Edition, U.S. Department of Health and Human Services, NIH Publication No. 91-3242).

In some instances, the anti-sclerostin antibody is created after the VH CDR1, 2 and 3 sequences and VL CDR1, 2 and 3 sequences are "mixed and matched" (i.e., CDRs from different antibodies can be mixed and matched). Sclerostin binding of such "mixed and matched" antibodies can be tested using the binding assays described above and in the Examples (e.g., ELISAs). When VH CDR sequences are mixed and matched, the CDR1, CDR2 and/or CDR3 sequence from a particular VH sequence should be replaced with a structurally similar CDR sequence(s). Likewise, when VL CDR sequences are mixed and matched, the CDR1, CDR2 and/or CDR3 sequence from a particular VL sequence should be replaced with a structurally similar CDR sequence(s). It will be readily apparent to the ordinarily skilled artisan that novel VH and VL sequences can be created by substituting one or more VH and/or VL CDR region sequences with structurally similar sequences from the CDR sequences shown herein for monoclonal antibodies.

For example, the anti-sclerostin antibody may have a heavy chain variable region CDR1 comprising an amino acid sequence selected from the group consisting of SEQ ID NOs: 1-11; a heavy chain variable region CDR2 comprising an amino acid sequence selected from the group consisting of SEQ ID NOs: 12-22; a heavy chain variable region CDR3 comprising an amino acid sequence selected from the group consisting of SEQ ID NOs: 23-33; a light chain variable region CDR1 comprising an amino acid sequence selected from the group consisting of SEQ ID NOs: 34-44; a light chain variable region CDR2 comprising an amino acid sequence selected from the group consisting of SEQ ID NOs: 45-55; and a light chain variable region CDR3 comprising an amino acid sequence selected from the group consisting of SEQ ID NOs: 56-66; wherein the antibody specifically binds sclerostin.

In one instance, the anti-sclerostin antibody comprises: a heavy chain variable region CDR1 of SEQ ID NO: 3; a heavy chain variable region CDR2 of SEQ ID NO: 14; a heavy chain variable region CDR3 of SEQ ID NO: 25; a light chain variable region CDR1 of SEQ ID NO: 36; a light chain variable region CDR2 of SEQ ID NO: 47; and a light chain variable region CDR3 of SEQ ID NO: 58.

In a certain embodiment, the anti-sclerostin antibody comprises: a heavy chain variable region CDR1 of SEQ ID NO: 4; a heavy chain variable region CDR2 of SEQ ID NO: 15; a heavy chain variable region CDR3 of SEQ ID NO: 26; a light chain variable region CDR1 of SEQ ID NO: 37; a light chain variable region CDR2 of SEQ ID NO: 48; and a light chain variable region CDR3 of SEQ ID NO: 59.

In one instance, the anti-sclerostin antibody comprises: a heavy chain variable region CDR1 of SEQ ID NO: 5; a heavy chain variable region CDR2 of SEQ ID NO: 16; a heavy chain variable region CDR3 of SEQ ID NO: 27; a light chain variable region CDR1 of SEQ ID NO: 38; a light chain variable region CDR2 of SEQ ID NO: 49; and a light chain variable region CDR3 of SEQ ID NO: 60.

In one instance, the anti-sclerostin antibody comprises: a heavy chain variable region CDR1 of SEQ ID NO: 6; a heavy chain variable region CDR2 of SEQ ID NO: 17; a heavy chain variable region CDR3 of SEQ ID NO: 28; a light chain variable region CDR1 of SEQ ID NO: 39; a light chain variable region CDR2 of SEQ ID NO: 50; and a light chain variable region CDR3 of SEQ ID NO: 61.

In one instance, the anti-sclerostin antibody comprises: a heavy chain variable region CDR1 of SEQ ID NO: 7; a heavy chain variable region CDR2 of SEQ ID NO: 18; a heavy chain variable region CDR3 of SEQ ID NO: 29; a light chain variable region CDR1 of SEQ ID NO: 40; a light chain variable region CDR2 of SEQ ID NO: 51; and a light chain variable region CDR3 of SEQ ID NO: 62.

In one instance, the anti-sclerostin antibody comprises: a heavy chain variable region CDR1 of SEQ ID NO: 8; a heavy chain variable region CDR2 of SEQ ID NO: 19; a heavy chain variable region CDR3 of SEQ ID NO: 30; a light chain variable region CDR1 of SEQ ID NO: 41; a light chain variable region CDR2 of SEQ ID NO: 52; and a light chain variable region CDR3 of SEQ ID NO: 63.

In one instance, the anti-sclerostin antibody comprises: a heavy chain variable region CDR1 of SEQ ID NO: 9; a heavy chain variable region CDR2 of SEQ ID NO: 20; a heavy chain variable region CDR3 of SEQ ID NO: 31; a light chain variable region CDR1 of SEQ ID NO: 42; a light chain variable region CDR2 of SEQ ID NO: 53; and a light chain variable region CDR3 of SEQ ID NO: 64.

In one instance, the anti-sclerostin antibody comprises: a heavy chain variable region CDR1 of SEQ ID NO: 10; a heavy chain variable region CDR2 of SEQ ID NO: 21; a heavy chain variable region CDR3 of SEQ ID NO: 32; a light chain variable region CDR1 of SEQ ID NO: 43; a light chain variable region CDR2 of SEQ ID NO: 54; and a light chain variable region CDR3 of SEQ ID NO: 65.

In one instance, the anti-sclerostin antibody comprises: a heavy chain variable region CDR1 of SEQ ID NO: 11; a heavy chain variable region CDR2 of SEQ ID NO: 22; a heavy chain variable region CDR3 of SEQ ID NO: 33; a light chain variable region CDR1 of SEQ ID NO: 44; a light chain variable region CDR2 of SEQ ID NO: 55; and a light chain variable region CDR3 of SEQ ID NO: 66.

In a certain embodiment, the anti-sclerostin antibody comprises: a VH polypeptide amino acid sequence having at least 95 percent identity to the amino acid sequence set forth as SEQ ID NO: 70.

In a certain embodiment, the anti-sclerostin antibody comprises: a VL polypeptide amino acid sequence having at least 95 percent identity to the amino acid sequence set forth as SEQ ID NO: 81.

In a certain embodiment, the anti-sclerostin antibody comprises: a VH polypeptide amino acid sequence having at least 95 percent identity to the amino acid sequence set forth as SEQ ID NO: 70 and a VL polypeptide amino acid sequence having at least 95 percent identity to the amino acid sequence set forth as SEQ ID NO: 81.

In a certain embodiment, the anti-sclerostin antibody comprises: the VH polypeptide amino acid sequence set forth as SEQ ID NO: 70 and the VL polypeptide amino acid sequence set forth as SEQ ID NO: 81.

In a certain embodiment, the anti-sclerostin antibody comprises: the heavy chain polypeptide amino acid sequence set forth as SEQ ID NO: 114 or 172 and the light chain polypeptide amino acid sequence set forth as SEQ ID NO: 125 or 173.

In a preferred embodiment, the anti-sclerostin antibody is the antibody BPS804, which is a human anti-sclerostin monoclonal antibody. BPS804 comprises the following CDRs: heavy chain variable region CDR1 of SEQ ID NO: 4; heavy chain variable region CDR2 of SEQ ID NO: 15; heavy chain variable region CDR3 of SEQ ID NO: 26; light chain variable region CDR1 of SEQ ID NO: 37; light chain variable region CDR2 of SEQ ID NO: 48; and light chain variable region CDR3 of SEQ ID NO: 59. The VH and VL sequences of BPS804 comprise: the VH polypeptide amino acid sequence set forth as SEQ ID NO: 70 and the VL polypeptide amino acid sequence set forth as SEQ ID NO: 81. The heavy and light chain sequences of BPS804 comprise: the heavy chain polypeptide amino acid sequence set forth as SEQ ID NO: 172 and the light chain polypeptide amino acid sequence set forth as SEQ ID NO: 173.

Additional characteristics of the anti-sclerostin antibodies of the present invention, such as BPS804 are described in WO2009/047356. By way of example only, the antibodies of the invention may exhibit at least one of the following functional properties: the antibody blocks the inhibitory effect of sclerostin in a cell based wnt signaling assay, the antibody blocks the inhibitory effect of sclerostin in a cell based mineralization assay, the antibody blocks the inhibitory effect of sclerostin in Smad1 phosphorylation assay, the antibody inhibits binding of sclerostin to the LRP-6, and the antibody increases bone formation and mass and density. As noted above, these properties are described in detail in WO2009/047356.

In relation to an antibody that "blocks the inhibitory effect of sclerostin in a cell based wnt signaling assay", this is intended to refer to an antibody that restores wnt induced signaling in the presence of sclerostin in a cell-based super top flash (STF) assay with an IC50 less than 1mM, 100 nM, 20 nM, 10nM or less. WO2009/047356 describes said wnt STF assay.

In relation to an antibody that "blocks the inhibitory effect of sclerostin in a cell based mineralization assay", this is intended to refer to an antibody that restores BMP2 induced mineralisation in the presence of sclerostin in a cell-based assay with an IC50 less than 1mM, 500nM, 100 nM, 10nM, 1nM or less.

In relation to an antibody that "blocks the inhibitory effect of sclerostin in Smad1 phosphorylation assay", this is intended to refer to an antibody that restores BMP6 induced Smad1 phosphorylation in the presence of sclerostin in a cell based assay with an IC50 less than 1mM, 500nM, 100 nM, 10nM, 1nM or less.

In relation to an antibody that "inhibits binding of sclerostin to the LRP-6", this is intended to refer to an antibody that inhibits sclerostin binding to LRP-6 with a IC50 of 1rnM, 500nM, 100nM, 10nM, 5nM, 3nM, 1nM or less.

In relation to an antibody that "increases bone formation and mass and density", this is intended to refer to an antibody that is capable of reaching bone formation, mass and density at the level of daily intermittent treatment with high anabolic dose of PTH, such as a daily intermittent treatment with 100 µg/kg of hPTH.

In one embodiment, the anti-sclerostin antibody of the invention increases bone formation and/or reduces bone resorption.

### Dosage regimen

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms are dictated by and directly dependent on the unique characteristics of the active compound and the particular therapeutic effect to be achieved, and the limitations inherent in the art of compounding such an active compound for the treatment of sensitivity in individuals. In one embodiment, the dosage unit form comprises 10-5000 mg of anti-sclerostin antibody, or 10-4000 mg, 10-3000 mg, 10-2000 mg, 10-1000 mg, 10-500 mg, 10-400 mg, 10-300 mg, 10-200 mg, 10-150 mg, 10-100 mg, 10-80 mg, 10-60 mg, 10-50 mg, 10-40 mg, 10-35 mg, 10-30 mg, 10-25 mg, 10-20 mg, or 10-15 mg. In one embodiment, the dosage unit form comprises 150 mg of anti-sclerostin antibody. In another embodiment, the dosage unit form comprises 210 mg of anti-sclerostin antibody.

In one embodiment, the anti-sclerostin antibody of the dosage unit form is in a lyophilized state which may be in powder form. In another embodiment, the anti-sclerostin antibody of the dosage unit form is in solution. In one embodiment the dosage unit form is contained within a container such as a vial. In another embodiment, the container is a syringe.

In addition to the active substance (i.e. the anti-sclerostin antibody), the dosage unit form may comprise one or more additional substances and/or excipients. In one embodiment, the dosage unit form comprises one or more of the following: sucrose, arginine hydrochloride, L-histidine, polysorbate 80, hydrochloric acid and water for injection (wfi).

Disclosed herein is a kit comprising an anti-sclerostin antibody of the invention or a pharmaceutical composition of the invention, or a lyophilizate of the invention, or a dosage unit form of the invention. Optionally the kit may further comprise instructions in the form of e.g. a patient information leaflet, instructions for reconstitution of the lyophilizate, and/or administration instructions. In one embodiment, the kit includes a syringe comprising one or more therapeutically effective doses of the anti-sclerostin antibody. The anti-sclerostin antibody in the syringe can be present in liquid or lyophilized form. The kit may further comprise a solution for reconstitution of the lyophilizate, and/or an infusion solution (e.g. dextrose 5% in sterile water).

The anti-sclerostin antibody is administered at a dose of 20 mg per kg body weight of a patient.

A subcutaneous, topical or oral administration route may facilitate self-administration of the anti-sclerostin antibody and preclude visits to a doctor/ hospital in order to receive treatment.

Another exemplary treatment regime entails administration of multiple doses of 20 mg/kg, until a treatment target is achieved or reached in the patient. The treatment target is achieved or reached after a certain number of doses are administered. The treatment target may be a complete normalization of bone mineral density, a partial normalization of bone mineral density, or a reduced frequency of bone fracture incidence, an increased level of bone formation markers, or a decreased level of bone resorption markers. Thus, in one embodiment, the invention provides an anti-sclerostin antibody for use in the treatment of OI, wherein the anti-sclerostin antibody reduces the fracture rate in a patient/ patient population compared to a control patient/ patient population. Preferably, the anti-sclerostin antibody reduces the fracture rate by at least 10, 20, 30, 35, 40, 50, 60, 70, 80, or 90 percent. In one embodiment the anti-sclerostin antibody reduces the fracture rate by at least 30 percent. In one embodiment, fractures are defined as peripheral or vertebral fractures (including all major, minor, and vertebral clinical fractures; fractures only detected by means of investigations without clinical symptoms are not included), confirmed by radiologic investigation(s). In one embodiment the fracture rate pertains to a population of patients. The patient population and control patient populations are preferably of a size that allow a statistically significant comparison to be made.

In one aspect of the invention, a treatment regimen entails a first dosing regimen optionally followed by a second dosing regimen. A dosing regimen includes the dose administered and the frequency of administration. These can be selected from any of the aforementioned doses and administration frequencies and can be varied according to the clinical requirements of a patient. The doses and administration frequencies disclosed above are hereby explicitly embraced in this aspect of the invention.

The first dosing regimen is 20 mg per kg body weight of a patient administered on a monthly basis.

In one embodiment and by way of example only, the second dosing regimen 1-50 mg per kg body weight of a patient, or 2-50 mg/kg, or 3-50 mg/kg, or 5-50 mg/kg, or 8-50 mg/kg, or 3-30 mg/kg, 5-30 mg/kg, or 8-30 mg/kg, or 10-30 mg/kg, or 12-30 mg/kg, or 12-25 mg/kg, or 15-30 mg/kg ,or 15-25 mg/kg administered on a monthly basis. In another exemplary embodiment, the second dosing regimen is 10-5000mg administered on a monthly basis.

In one embodiment, the second dosing regimen is 1-50 mg per kg body weight of a patient administered on a monthly basis.

In one embodiment, the second dosing regimen is 2-50 mg per kg body weight of a patient administered on a monthly basis.

In one embodiment, the second dosing regimen is 2-30 mg per kg body weight of a patient administered on a monthly basis.

In one embodiment, the second dosing regimen is 3-50 mg per kg body weight of a patient administered on a monthly basis.

In one embodiment, the second dosing regimen is 3-30 mg per kg body weight of a patient administered on a monthly basis.

In one embodiment, the second dosing regimen is 5-30 mg per kg body weight of a patient administered on a monthly basis.

In one embodiment, the second dosing regimen is 5-25 mg per kg body weight of a patient administered on a monthly basis.

In one embodiment, the second dosing regimen is 12-25 mg per kg body weight of a patient administered on a monthly basis.

In one embodiment, the second dosing regimen is 20 mg per kg body weight of a patient administered on a monthly basis.

In another embodiment, the second dosing regimen 1-50 mg per kg body weight of a patient, or 2-50 mg/kg, or 3-50 mg/kg, or 5-50 mg/kg, or 8-50 mg/kg, or 3-30 mg/kg, 5-30 mg/kg, or 8-30 mg/kg, or 10-30 mg/kg, or 12-30 mg/kg, or 12-25 mg/kg, or 15-30 mg/kg ,or 15-25 mg/kg administered on a bi-monthly basis. In another exemplary embodiment, the second dosing regimen is 10-5000mg administered on a bi-monthly basis.

In one embodiment, the second dosing regimen is 1-50 mg per kg body weight of a patient administered on a bi-monthly basis.

In one embodiment, the second dosing regimen is 2-50 mg per kg body weight of a patient administered on a bi-monthly basis.

In one embodiment, the second dosing regimen is 2-30 mg per kg body weight of a patient administered on a bi-monthly basis.

In one embodiment, the second dosing regimen is 3-50 mg per kg body weight of a patient administered on a bi-monthly basis.

In one embodiment, the second dosing regimen is 3-30 mg per kg body weight of a patient administered on a bi-monthly basis.

In one embodiment, the second dosing regimen is 5-30 mg per kg body weight of a patient administered on a bi-monthly basis.

In one embodiment, the second dosing regimen is 5-25 mg per kg body weight of a patient administered on a bi-monthly basis.

In one embodiment, the second dosing regimen is 12-25 mg per kg body weight of a patient administered on a bi-monthly basis.

In one embodiment, the second dosing regimen is 20 mg per kg body weight of a patient administered on a bi-monthly basis.

In another exemplary embodiment, the second dosing regimen is 1-50 mg per kg body weight of a patient, or 2-50 mg/kg 3-50 mg/kg, or 5-50 mg/kg, or 8-50 mg/kg, or 8-30 mg/kg, or 10-30 mg/kg, or 12-30 mg/kg, or 12-25 mg/kg, or 15-30 mg/kg, or 15-25 mg/kg administered on a quarterly basis. In another exemplary embodiment, the second dosing regimen is 10-5000mg administered on a quarterly basis.

In one embodiment, the second dosing regimen is 2-30 mg per kg body weight of a patient administered on a quarterly basis.

In one embodiment, the second dosing regimen is 3-30 mg per kg body weight of a patient administered on a quarterly basis.

In one embodiment, the second dosing regimen is 5-30 mg per kg body weight of a patient administered on a quarterly basis.

In one embodiment, the second dosing regimen is 5-25 mg per kg body weight of a patient administered on a quarterly basis.

In one embodiment, the second dosing regimen is 12-25 mg per kg body weight of a patient administered on a quarterly basis.

In one embodiment, the second dosing regimen is 20 mg per kg body weight of a patient administered on a quarterly basis.

In a related embodiment, the first dosing regimen is 20 mg per kg body weight of a patient administered on a monthly basis and the second dosing regimen is 20 mg per kg body weight of a patient administered on a bi-monthly or quarterly basis.

The time period of administration of the first and second dosing regimens can be varied according to the clinical requirements of a patient. Thus, the first dosing regimen is administered for a first time period and the second dosing regimen is administered for a second time period.

Thus, and by way of example, the first and second time periods can be 1 month, 6 months, 12 months or any other time period.

In one embodiment, an anti-sclerostin antibody can be initially administered to a patient on a monthly basis for a period of 1 year, followed by administration on a bi-monthly or quarterly basis for a period of at least 1 year (such as 2 or more years).

Thus, in one embodiment, the first dosing regimen can be 20 mg per kg body weight of a human patient administered on a monthly basis for a period of 1 year, and the second dosing regimen can be 20 mg per kg body weight of a human patient administered on a bi-monthly or quarterly basis for a period of at least 1 year (such as 2 or more years).

Off-drug periods i.e. periods in which the anti-sclerostin antibody is not administered are also contemplated by the present invention, depending on the clinical requirements of the patient. Thus, in one embodiment the treatment with the anti-sclerostin antibody is discontinued for one or more (such as 2, 3, 4, 5, 6, 8, 10, 12 or more) months or even one or more years.

Measurements of the targets are known in the art. For example, bone mineral density may be measured by dual-energy x-ray absorptiometry (DXA), single-energy x-ray absorptiometry (SXA), quantitative computed tomography (CT), and ultrasound. DXA is an x- ray technique that has become the standard for measuring bone density in the art. Though it can be used for measurements of any skeletal site, clinical determinations are usually made of the lumbar spine and hip. Portable DXA machines have been developed that measure the heel (calcaneus), forearm (radius and ulna), or finger (phalanges), and DXA can also be used to measure body composition. Consequently, it has become standard practice to relate the results to "normal" values using T-scores, which compare individual results to those in a young population that is matched for race and gender. Alternatively, Z-scores compare individual results to those of an age-matched population that is also matched for race and gender. Thus, for example, a 60- year-old woman with a Z-score of -1 (1 SD below mean for age) could have a T-score of -2.5 (2.5 SD below mean for a young control group).

Yet another exemplary treatment regime entails administration of multiple doses, which may be of the same dosage or different dosages ranging from, e.g., about 5-20 mg/kg, for a long term use without a specific timeline to stop the administration. This may be the treatment regime to follow when a dose is needed to maintain an improved symptom in a continuous basis.

In some embodiments, the anti-sclerostin antibody and one or more monoclonal antibodies with different binding specificities are administered simultaneously or sequentially, in which case the dosage of each antibody administered falls within the ranges indicated. In one embodiment the one or more additional monoclonal antibodies are also anti-sclerostin antibodies. Antibody is usually administered on multiple occasions. Intervals between single dosages can be, for example, weekly, monthly, every two months (i.e. bi-monthly) every three months or yearly. Intervals can also be irregular as indicated by measuring blood levels of antibody to the target antigen in the patient. In some methods, dosage is adjusted to achieve a plasma antibody concentration of about 1-1000 µg/ml and in some methods about 25-300 µg/ml. In one embodiment, dosage of anti-sclerostin antibody of the invention is adjusted to achieve a plasma antibody concentration of about 1-1000 µg/ml or about 25-300 µg/ml.

Alternatively, in some embodiments, the anti-sclerostin antibody can be administered to OI patients as a sustained release formulation, in which case less frequent administration is required. Dosage and frequency vary depending on the half-life of the antibody in the patient. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated or until the patient shows partial or complete amelioration of symptoms of disease. Thereafter, the patient can be administered a prophylactic regime.

Actual dosage levels of the anti-sclerostin antibody can be varied so as to obtain an amount of the anti-sclerostin antibody which is effective to achieve the desired therapeutic response for a particular OI patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular anti-sclerostin antibody being employed, the duration of the treatment, other drugs, compounds and/or materials used in combination with the particular compositions employed, the age, sex, weight, condition, general health and prior medical history of the patient being treated, and like factors well known in the medical arts.

A "therapeutically effective amount" of the anti-sclerostin antibody may result in a decrease in severity of disease symptoms, an increase in frequency and duration of disease symptom-free periods, or a prevention of impairment or disability due to the disease affliction.

A composition of the anti-sclerostin antibody can be administered by one or more routes of administration using one or more of a variety of methods known in the art. As will be appreciated by the skilled artisan, the route and/or mode of administration will vary depending upon the desired results. Routes of administration for antibodies of the invention include intravenous, intramuscular, intradermal, intraperitoneal, subcutaneous, spinal or other parenteral routes of administration, for example by injection or infusion. In one embodiment, administration occurs via the intravenous route. In one embodiment, administration occurs intravenously by way of an infusion. In one embodiment, administration occurs subcutaneously. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Alternatively, the anti-sclerostin antibody can be administered by a nonparenteral route, such as a topical, epidermal or mucosal route of administration, for example, intranasally, orally, vaginally, rectally, sublingually or topically.

The anti-sclerostin antibody can be prepared with carriers that may protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978.

### Patient group

In one embodiment, the uses described herein are for treating osteogenesis imperfecta using anti-sclerostin antibodies described herein. OI is classified by the genetics and severity of disease, and can be classified as type I OI, type II OI, type III OI, type IV OI or type V OI according to the classification of Van Dijk and Sillence (2014, Am J Med Genet Part A 164A:1470-1481 and Van Dijk and Sillence, 2014, Am J Med Genet Part A 167A:1178). Classification relies on a combination of clinical evaluation/ diagnosis, biochemical analysis as well as molecular genetic testing, and is routine for those skilled in the art. The OI nomenclature/ classification as used herein is as proposed by Van Dijk and Sillence, as referenced in the publications above.

In 80%-90% of people with OI, OI is caused by mutations in the COL1A1 and COL1A2 genes (17q21.33 and 7q22.3, respectively) encoding the alpha1 and alpha 2 chains of type-I collagen. A comprehensive database of over 1000 known mutations has been published along with a genotype-phenotype correlation (https://oi.gene.le.ac.uk/home.php; accessed 12 December 2016). Mutations in other genes, such as CRTAP, LEPRE1 or PPIB, are also known. Molecular genetic tests for mutations in i.a. the COL1A1 and COL1A2 genes are known and routine for those skilled in the art. By way of example, Korkko et al. (1998) describe PCR amplification of the COL1A1 gene and the COL1A2 genes followed by mutation scanning by conformation-sensitive gel electrophoresis(CSGE) (Am. J. Hum. Genet. 62:98-110, 1998). van Dijk et al. (2010) describe COL1A1 mutation detection by a multiplex ligation-dependent probe amplification (MLPA) technique (Genet Med 12(11):736-741). More recently, Árvai, K. et al. (2016) describe next-generation sequencing methods (Sci. Rep. 6, 28417).

In one embodiment, the uses described herein are for treating patients who exhibit a deficiency of type-I collagen, *e.g.* OI types I - IV. As a result, the normal architecture of bone, consisting of collagen fibrils and hydroxyapatite crystals, is altered and causes brittleness. In one embodiment, the uses herein are for treating human OI patients characterized by one or more mutations in COL1A1 and/or COL1A2.

In one embodiment, the uses described herein are for treating OI type I, III and/or IV. In one embodiment, OI type I, III and IV are confirmed by DNA testing i.e. detection of COL1A1/ COL1A2 mutations. Thus, in one embodiment the uses herein are for treating OI type I, III and/or IV characterized by one or more mutations in COL1A1 and/or COL1A2.

In some embodiments, the uses of the anti-sclerostin antibody are for treating a mild to moderate form of OI. In other embodiments of the uses of the anti-sclerostin antibody, the patient under treatment has a type I OI, a type II OI, a type III OI, or a type IV OI. In still other embodiments of the uses of the anti-sclerostin antibody, the OI patients are adult patients aged 18 and above. In yet still other embodiments of the uses of the anti-sclerostin antibody, the OI patients are pediatric patients. Pediatric patients as defined herein embraces children aged 0-17 such as those aged 2-17, 3-17, 4-17 or 5-17. The term patients, as used herein, means human patients.

### Pharmaceutical Compositions

In another aspect, the present invention provides a pharmaceutical composition for increasing bone formation and reducing bone resorption in a patient suffering from osteogenesis imperfecta, which composition contains the anti-sclerostin antibody as as defined in the invention. The pharmaceutical composition may be formulated with a pharmaceutically acceptable carrier. Also disclosed herein is a pharmaceutical composition comprising an anti-sclerostin antibody as disclosed herein. In one embodiment, the pharmaceutical composition comprising the anti-sclerostin antibody is for use in treating **OI.**

As used herein, "pharmaceutically acceptable carrier" includes any and all solvents, dispersion media, coatings, antibacterial and antifungal agents, isotonic and absorption delaying agents, and the like that are physiologically compatible. The carrier should be suitable for intravenous, intramuscular, subcutaneous, parenteral, spinal or epidermal administration (e.g., by injection or infusion). Depending on the route of administration, the anti-sclerostin antibody, may be coated in a material to protect the compound from the action of acids and other natural conditions that may inactivate the compound.

These compositions may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as, aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, the methods of preparation are vacuum drying and freeze-drying (lyophilization) that yield a powder of the active ingredient plus any additional desired ingredient from a previously sterile-filtered solution thereof. Thus, in one embodiment, the anti-sclerostin antibody of the invention/ pharmaceutical composition of the invention is formulated as a lyophilizate powder. In a related embodiment, the lyophilizate is reconstituted prior to administration. Suitable liquids for reconstitution include water for injection (wfi).

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred percent, this amount will range from about 0.01 percent to about ninety-nine percent of active ingredient, from about 0.1 percent to about 70 percent, or from about 1 percent to about 30 percent of active ingredient in combination with a pharmaceutically acceptable carrier.

Pharmaceutical compositions of the invention and antibodies of the invention may also be administered in a combination therapy, i.e., combined with other active agents. For example, the combination therapy can include an anti-sclerostin antibody of the present invention combined with at least one other anti-inflammatory or anti-osteoporotic agent. Examples of therapeutic agents that can be used in combination therapy include bisphosphonates (such as alendronate, risedronate sodium, ibandronic acid, zoledronic acid, olpadronate, neridronate, skelid, bonefos), parathyroid hormone (e.g. teriparatide (rdna origin) injection), calcilytics, calcimimetics (e.g., cinacalcet), statins, anabolic steroids, lanthanum and strontium salts, and sodium fluoride. Thus, in one embodiment the anti-sclerostin antibodies of the invention can be administered in combination with one or more agents selected from: a calcitonin or an analogue or derivative thereof, e.g. salmon, eel or human calcitonin, calcilytics, calcimimetics (e.g., cinacalcet), a steroid hormone, e.g. an estrogen, a partial estrogen agonist or estrogen-gestagen combination, a SERM (Selective Estrogen Receptor Modulator) e.g. raloxifene, lasofoxifene, bazedoxifene, arzoxifene, FC1271 , Tibolone (Livial ^{®}), a SARM (Selective Androgen Receptor Modulator), a RANKL antibody (such as denosumab), a cathepsin K inhibitor, vitamin D or an analogue thereof or PTH, a PTH fragment or a PTH derivative e.g. PTH (1-84) (such as Preos^{™}), PTH (1-34) (such as Forteo^{™}), PTH (1-36), PTH (1-38), PTH (1-31)NH2 or PTS 893. According to another embodiment, the antibodies of the invention may be employed in combination with other current osteoporosis therapy approaches, including bisphosphonates (e.g., Fosamax^{™} (alendronate), Actonel^{™} (risedronate sodium), Bonviva^{™} (ibandronic acid), Zometa^{™} (zoledronic acid), Aclasta^{™}/Reclast^{™} (zoledronic acid), olpadronate, neridronate, skelid, bonefos), statins, anabolic steroids, lanthanum and strontium salts, and sodium fluoride.

In one embodiment, the antibodies of the invention may be administered in combination with an LRP4 modulating agent, i.e., an agent modulating the expression or activity of LRP4, e.g, an LRP4 neutralizing antibody.

In one embodiment, the antibodies of the invention may be administered in combination with an LRP5 modulating agent, i.e., an agent modulating the expression or activity of LRP5, e.g, an LRP5 neutralizing antibody.

In another embodiment, the antibodies of the invention may be administered in combination with a DKK1 modulating agent, i.e., an agent that interfere or neutralize Dkk-1 mediated antagonism of Wnt signaling, e.g., a DKK1 neutralizing antibody.

In one embodiment, the antibodies of the invention may be administered in combination with a bisphosphonate e.g. alendronate, risedronate sodium, ibandronic acid, zoledronic acid, zoledronic acid, olpadronate, neridronate, skelid, bonefos.

In one embodiment, the antibodies of the invention may be administered in combination with (i) zoledronic acid, (ii) an anti-DKK1 antibody, (iii) alendronate, (iv) an anti- LRP4 antibody, (v) hPTH and/or (vi) parathyroid hormone releasing agents (calcilytics).

Other agents which can be administered in combination with the anti-sclerostin antibodies of the invention include vitamin D and/or calcium. In one embodiment the vitamin D and/or calcium is administered if the patient has a vitamin D and/or calcium deficiency.

In one embodiment, the antibodies of the invention are administered together with another agent (e.g. the above mentioned agents), in a sequential manner (i.e. one after the other) or simultaneously. In one embodiment, the anti-sclerostin antibody is administered according to the aforementioned doses and frequencies of administration. Suitable doses for the combination therapy agent can be varied according to the clinical requirements of the patient.

The compositions are preferably formulated at physiological pH.

Therapeutic compositions can be administered with medical devices known in the art. For example, in one embodiment, a therapeutic composition of the invention can be administered with a needleless hypodermic injection device, such as the devices shown in U.S. Pat. No. 5,399,163; 5,383,851; 5,312,335; 5,064,413; 4,941,880; 4,790,824 or 4,596,556. Examples of well-known implants and modules useful in the present invention include: U.S. Pat. No. 4,487,603, which shows an implantable micro-infusion pump for dispensing medication at a controlled rate; U.S. Pat. No. 4,486,194, which shows a therapeutic device for administering medicaments through the skin; U.S. Pat. No. 4,447,233, which shows a medication infusion pump for delivering medication at a precise infusion rate; U.S. Pat. No. 4,447,224, which shows a variable flow implantable infusion apparatus for continuous drug delivery; U.S. Pat. No. No.4,439,196, which shows an osmotic drug delivery system having multi-chamber compartments; and U.S. Pat. No. 4,475,196, which shows an osmotic drug delivery system. Many other such implants, delivery systems, and modules are known to those skilled in the art. In one embodiment, the therapeutic composition of the invention can be administered with a syringe.

In certain embodiments, the antibodies of the invention can be formulated to ensure proper distribution in vivo.

Disclosed herein is the use of an anti-sclerostin antibody for the manufacture of a medicament for the treatment of osteogenesis imperfecta. All of the other aspects/ embodiments described herein apply equally to this particular disclosure.

In another aspect the invention provides an anti-sclerostin antibody of the invention for use in the treatment of osteogenesis imperfecta according to the invention. All of the other aspects/ embodiments described herein apply equally to this particular aspect of the invention.

In one aspect, the invention provides an anti-sclerostin antibody of the invention for use in a clinical trial for osteogenesis imperfecta comprising comparing the number of fractures that have occurred in the clinical trial population at an interim time point during the clinical trial period with the number of fractures expected at said interim time point for said clinical trial population.

Also disclosed herein is a method of conducting a clinical trial for osteogenesis imperfecta with an anti-sclerostin antibody comprising comparing the number of fractures that have occurred in a clinical trial population at an interim time point during the clinical trial period with the number of fractures expected at said interim time point for said clinical trial population. In one instance, the disclosure may further comprise recruiting additional patients to the clinical trial population if the number of fractures in the clinical trial population at the interim time point is lower than the expected number of fractures for said clinical trial population. In one instance, the clinical trial period is extended if the number of fractures in the clinical trial population at the interim time point is lower than the expected number of fractures for said clinical trial population. In another instance, the clinical trial period is shortened if the number of fractures in the clinical trial population at the interim time point is higher than the expected number of fractures for said clinical trial population. The clinical trial population is made up of patients receiving the anti-sclerostin antibody and a control group of patients receiving a placebo. An advantage of these instances is that it allows the clinical trial period to be varied in response to developments in the clinic, which ultimately leads to a more efficient and cost-effective clinical trial which also benefits the patients. The expected number of fractures can be calculated from a baseline fracture rate which can be derived from historical data and/or from data from the clinical trial population prior to commencement of the clinical trial period. In one instance, the anti-sclerostin antibody is an anti-sclerostin antibody as defined herein. The interim time point can be varied and selected according to when a statistically significant comparison can be made. The clinical trial period begins with the administration of the anti-sclerostin antibody or placebo and ends once the final dose of anti-sclerostin antibody/ placebo has been administered and the relevant data collected.

Sequence of BPS804 H-chain (SEQ ID NO: 172):

Sequence of BPS804 L-chain (SEQ ID NO: 173):

### MODES FOR CARRYING OUT THE INVENTION

### Example 1

This example describes a clinical trial to assess the use of an anti-sclerostin antibody in the treatment of adult patients with OI. The patients were treated with three sequential intra-patient escalating doses of anti-sclerostin antibody BPS804, given as intravenous infusions separated by 2 weeks from each dose. An untreated reference group was enrolled as well for monitoring and observation of the natural OI disease progression with regard to changes in bone biomarker profiles. This trial was a randomized, open-label, intra-patient dose escalating study with an untreated reference group in 14 adult patients with moderate OI. Patients were randomized to the treatment group or the reference group at a ratio of 2:1.

Patients were administered every two weeks with escalating doses of the anti-sclerostin antibody: Week 1: 5 mg/kg, Week 3: 10 mg/kg and Week 5: 20 mg/kg. The treatment period was followed by an about 3.6 month follow-up period. Patients who were randomized to the untreated reference group at screening were only admitted to the study site at week 7, study Day 43 and at the end of the study.

A description of the study drug BPS804 is presented in Table 1. BPS804 solution for infusion was administered as an infusion at a flow rate of about 2 mL/min until the desired dose has been delivered.

Biomarkers of bone metabolism including procollagen I N-terminal propeptide (PINP), procollagen I C-terminal propeptide (PICP), bone-specific alkaline phosphatase (BSAP), osteocalcin (OC), and bone mineral density (BMD) (measured by DXA) were assayed at baseline, day 43 and day 141.

**Table 1 Study Drug BPS804**

| **Name** | **BPS804** |
|---|---|
| Formulation | Powder for solution for infusion (lyophilizate in vial) |
| Appearance before reconstitution | White lyophilizate cake |
| Appearance after reconstitution | Opalescent to clear, colorless solution |
| Unit dose | 150 mg per vial * |
| Packaging | 6 mL Type I glass vials |
| Diluent for iv administration | Dextrose 5% in water (USP or equivalent) in 250 mL |

| | |
|---|---|
| * The vials contain a 20% overfill to allow a complete withdrawal of the labeled amount of BPS804. | |

Of the 14 patients enrolled in the study, nine patients were exposed to anti-sclerostin antibody treatment. The overall mean age of the patients was marginally higher in the anti-sclerostin antibody group (30.7 years) when compared to the reference group (27.4 years). The overall mean weight and height between the two groups were very similar. Summary of patient demographics is presented in Table 2. The overall median scores of the lumbar spine z-score were comparatively lower in the anti-sclerostin antibody group. The overall years on bisphosphonates cannot be compared between the groups considering the relative size of the number of the patients in each group. See Table 3.

**Table 2 Demographic Summary By Treatment Group**

| | | | **BPS804 N=9** | | | **Reference N=5** | | **Total N=14** |
|---|---|---|---|---|---|---|---|---|
| Age (years) | | | | | | | | |
| Mean (SD) | | | | 30.7 (13.47) | | 27.4 (15.47) | | 29.5(13.71) |
| Median | | | | 25.0 | | 21.0 | | 21.5 |
| Range | | | | 19, 57 | | 19, 55 | | 19, 57 |
| Sex - n (%) | | | | | | | | |
| | Male | | 7 (77.8) | | 3 (60.0) | | 10(71.4) | |
| | Female | | 2 (22.2) | | 2 (40.0) | | 4 (28.6) | |
| Predominant Race - n (%) | | | | | | | | |
| | Caucasian | | 9 (100) | | 5 (100) | | 14 (100) | |
| Ethnicity - n (%) | | | | | | | | |
| | Mixed ethnicity | | 1 (11.1) | | 1 (20.0) | | 2 (14.3) | |
| | Other | | 8 (88.9) | | 4 (80.0) | | 12 (85.7) | |
| Weight (kg)* | | | | | | | | |
| | Mean (SD) | | 61.84 (14.378) | | 58.20 (13.034) | | 60.54 (13.519) | |
| | Median | | 63.90 | | 54.00 | | 59.45 | |
| | Range | | 43.5, 80.1 | | 44.0, 75.0 | | 43.5, 80.1 | |
| Height (cm)* | | | | | | | | |
| | | Mean (SD) | 161.6 (12.19) | | 162.8 (13.85) | | 162.0 (12.28) | |
| | | Median | 162.0 | | 161.0 | | 162.0 | |
| | | Range | 142, 178 | | 142, 176 | | 142, 178 | |

| | | | | | | | | |
|---|---|---|---|---|---|---|---|---|
| *Weight and height are taken from Screening vital signs evaluations. | | | | | | | | |

**Table 3 Disease Characteristics By Treatment Group At Study Entry**

| | | **BPS804 N=9** | **Reference N=5** | **Total N=14** |
|---|---|---|---|---|
| Lumbar spine z-score* | | | | |
| | Mean (SD) | -2.59 (1.191) | -2.18 (0.514) | -2.44 (0.997) |
| | Median | -2.30 | -2.07 | -2.19 |
| | Range | -4.9, -1.1 | -2.9, -1.5 | -4.9, -1.1 |
| Yrs. on bisphosphonates# | | | | |
| | N | 2 | 1 | 3 |
| | Mean (SD) | 8.53 (4.882) | 15.46 (-) | 10.84 (5.283) |
| | Median | 8.53 | 15.46 | 11.99 |
| | Range | 5.1, 12.0 | 15.5, 15.5 | 5.1, 15.5 |
| Subjects with D43 Biomarker data - n (%) | | | | |
| | | 9(100) | 5(100) | 14(100) |
| Subjects with D141 BMD data - n (%) | | | | |
| | | 9(100) | 4(80.0) | 13(92.9) |

| | | | | |
|---|---|---|---|---|
| *Lumbar spine z-score is taken at screening. #Years on bisphosphonates is calculated from the medical history page, by taking the difference between the earliest start date of bisphosphonates medication and date of screening. | | | | |

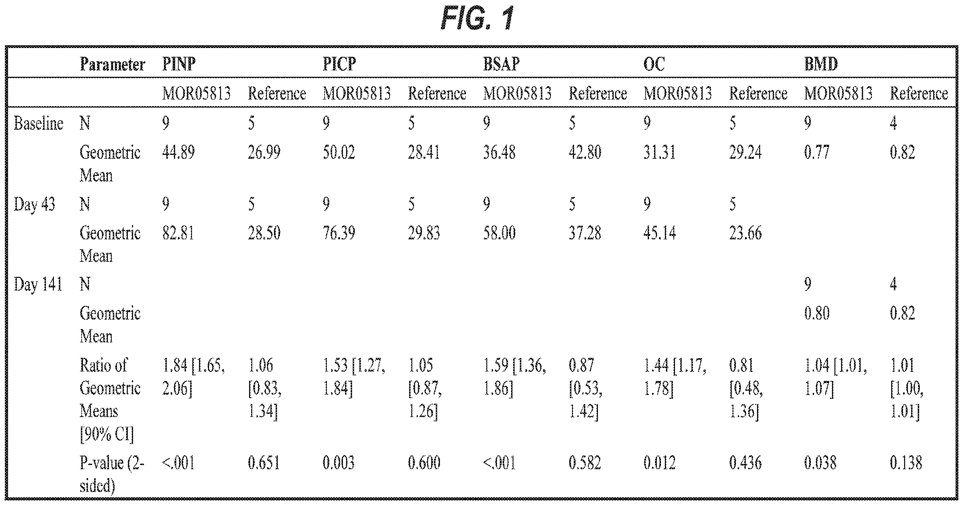

The assay results of the bone metabolism biomarkers for the study are presented in Figure 1. The ratios of geometric means for PINP, PICP, BSAP, and OC at Day 43 were 1.84, 1.53, 1.59 and 1.44 with P-values of <0.001, 0.003, <0.001, and 0.012 in the BPS804 group. The ratio of geometric means for BMD at Day 141 was 1.04 with P-value of 0.038.

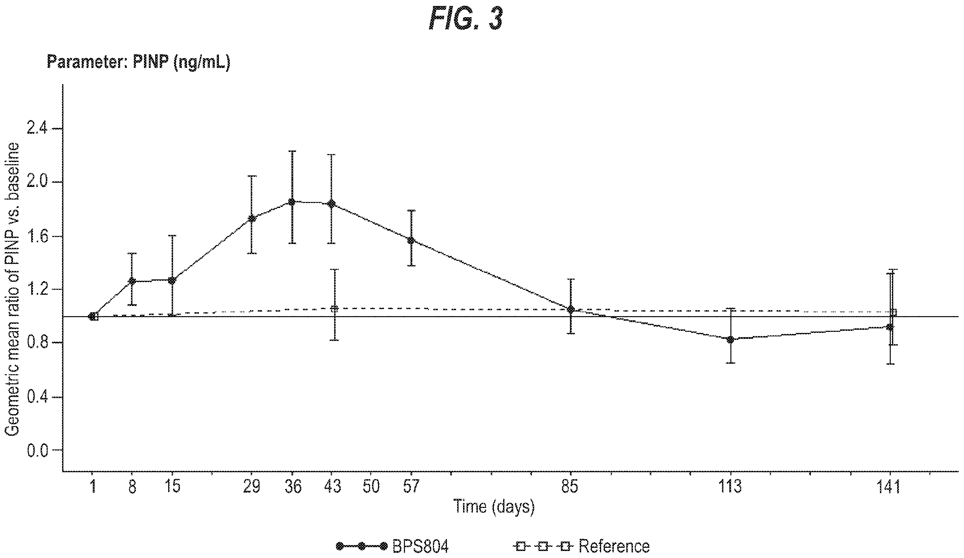

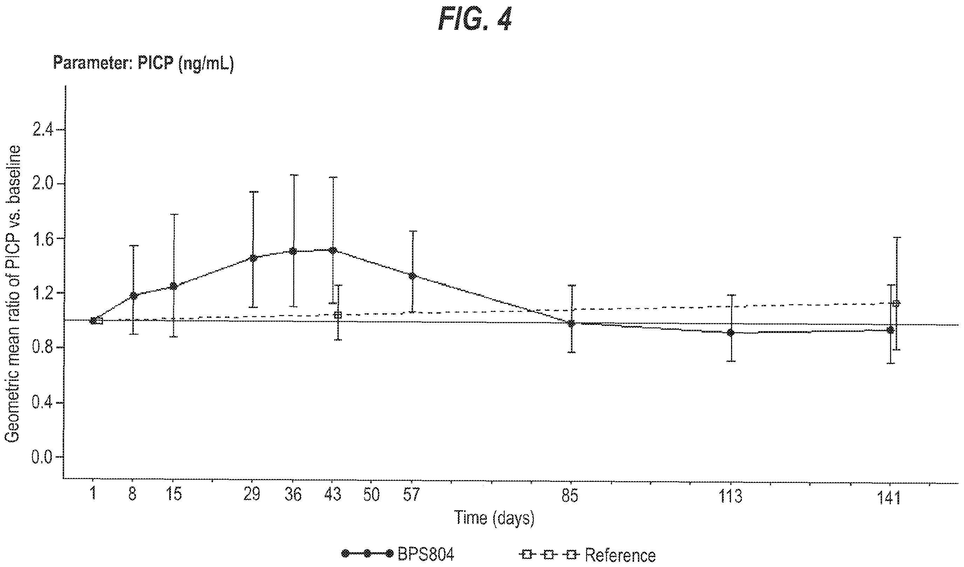

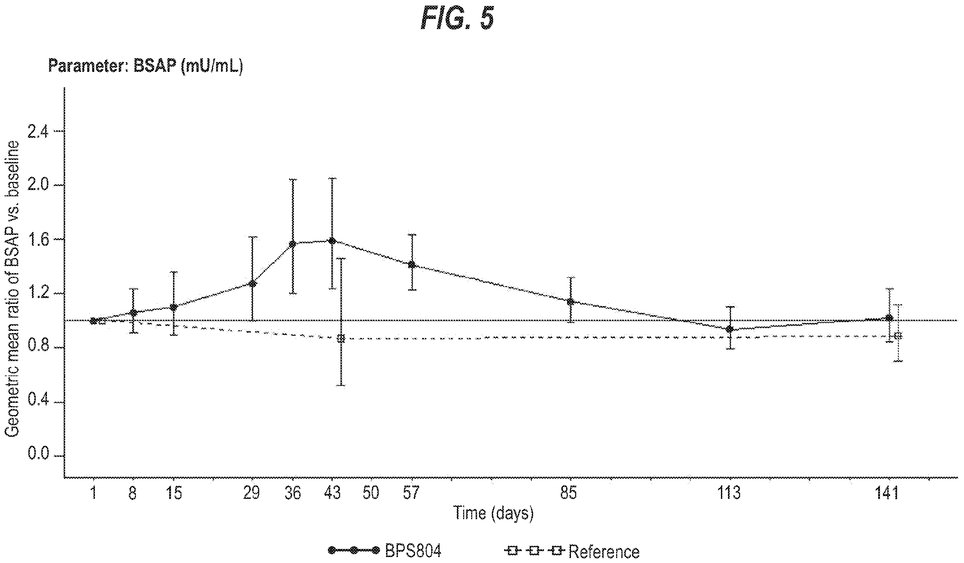

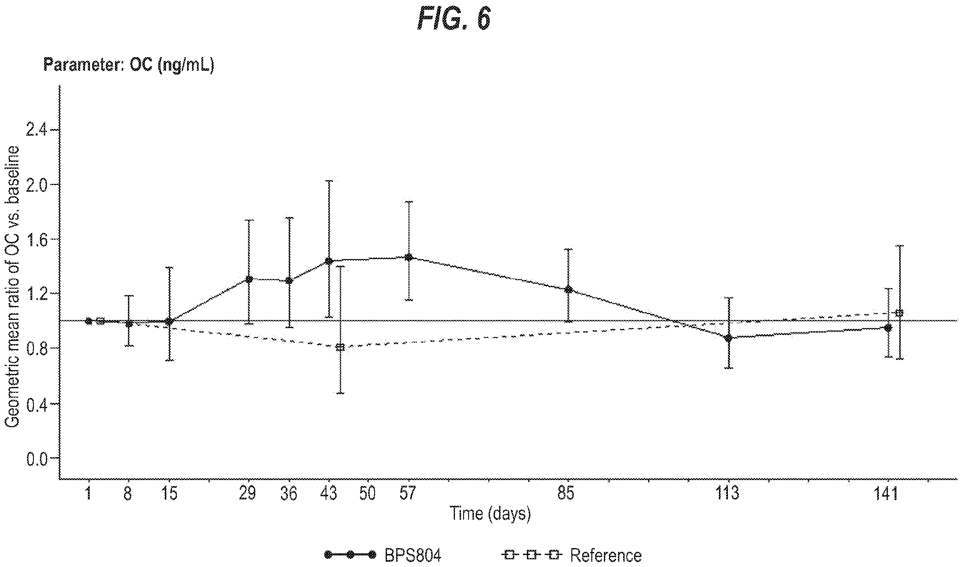

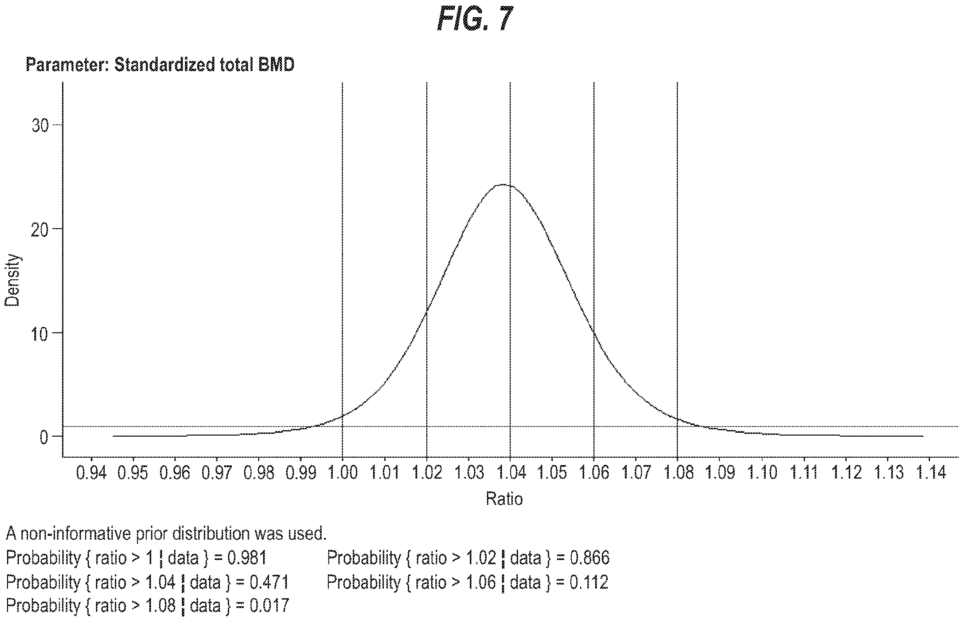

The Bayesian analysis of change from baseline for PINP, PICP, and BSAP showed a posterior probability of around 90% or higher for an increase of at least 70% (PINP) or 30% (PICP, BSAP). The Bayesian analysis for BMD showed a posterior probably of 98% for an increase in BMD, and 87% for an increase of at least 2%. See Figure 3, Figure 4, Figure 5, Figure 6 and Figure 7 for details on the PINP, PICP, BSAP, OC and BMD results, respectively.

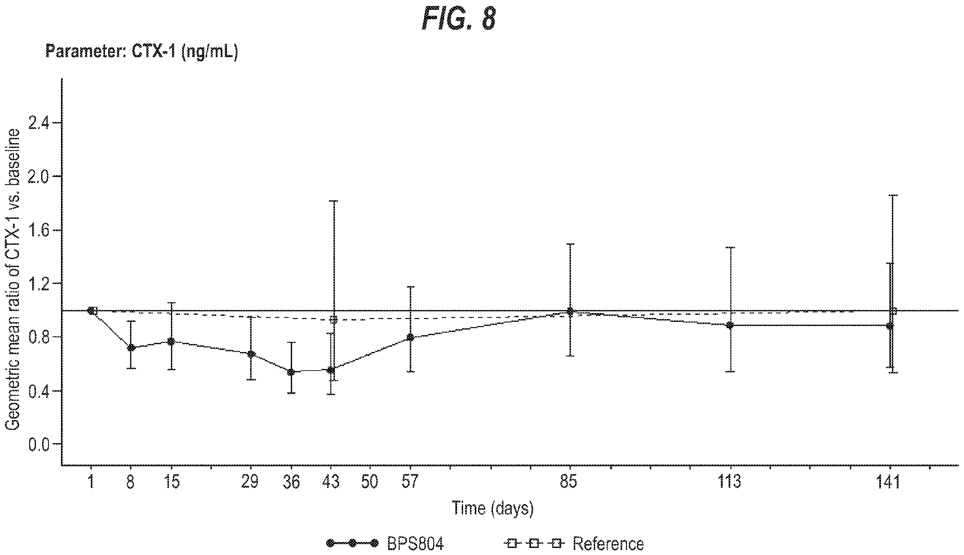

The geometric mean results on CTX-1 from Day 8 through Day43 showed a decrease in CTX-1 concentration levels which together with bone formation biomarker results supports bone anabolic effects. Figure 8 presents the details of the values since Baseline. On Day 43, the ratio from baseline in the BPS804 group was 56% of baseline, thus a reduction by 44%. The concentration levels steadily increased from Day 50 through Day 85 with a marginal decrease on Days 113 and 141.

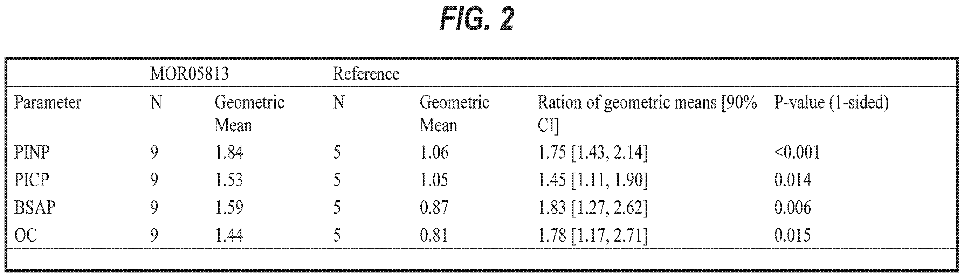

The comparison of ratios with the matching ratios in the reference group were performed as per the planned two sample t-tests (1-sided) and a P-value of below 0.1 in this comparison was considered supportive evidence for efficacy. Based on the two sample t-tests analysis, the P-values were <0.001, 0.014, 0.006, and 0.015 for PINP, PICP, BSAP, and OC, respectively, which supported the evidence for efficacy. The p-value for treatment group comparison of BMD was 0.1, also supporting the evidence that increase was larger under the anti-sclerostin antibody than in the reference group. See Figure 2. These analyses confirmed the evidence of higher increase in the anti-sclerostin antibody group than in the reference group, for all three biomarker data and for the BMD data.

After administration of the anti-sclerostin antibody, median PINP, PICP, BSAP activity, and OC levels were increased at Day 43 by 84%, 53%, 59%, and 44%, respectively, while corresponding biomarkers remained unchanged or declined moderately in the untreated reference group. Further, after administration of the anti-sclerostin antibody, CTX-1 level was decreased by 44% at Day 43 from baseline. The increase of bone formation biomarker (PINP, PICP, BSAP and OC) levels and the reduction of bone resorption biomarker (CTX-1) level were in line with the observed increase by ~ 4% in lumbar spine BMD at Day141 in the anti-sclerostin antibody treatment group, thus confirming the first clinical evidence of bone anabolic effects of an anti-sclerostin antibody in patients with osteogenesis imperfecta.

In addition, the study showed that the anti-sclerostin antibody was safe and well-tolerated in the adult patients with OI. The most commonly reported AEs were headache, influenza, arthralgia, and fatigue. None of the reported AEs were considered related to the study drug. There was one SAE (Goiter) of mild intensity reported in one patient in the reference group. This AE was considered serious because of hospitalization. The SAE resolved prior to end-of-study. The AEs gave no indication of target organ toxicity. Three fractures were reported during the study (subject 5103 - Day 47, subject 5109 - Day 4, Subject 5113 - Day 4). There were also no clinically significant abnormalities of hematological, clinical chemistry, urinalysis, ECG or vital sign data compromising the patients' safety.

According to the clinical study results, with an osteoanabolic treatment like BPS804, bone formation or bone anabolic activity can be stimulated in patients with OI. The increase in BMD results in improved bone quality thereby leading to a reduction in the fracture rate and risk. Genotyping might be warranted to identify or predict OI patients who might benefit most from such a treatment strategy.

### Example 2

A pharmacokinetic (PK) model was developed for BPS804 along with a pharmacokinetic (PK) and pharmacodynamics (PD) (PK-PD) model for circulating sclerostin effects after BPS804 administration based on a combination of clinical trial data and publically available data on other anti-sclerostin antibodies . The PK-PD model was linked to an existing systems pharmacology model to evaluate proposed dosing regimens for BPS804. Model simulations were used to provide guidance for dose selection and dosing interval over 1-2 years of treatment for typical OI patients. These included scenarios with different dosing considerations for the first year (e.g., comparison of dosage amount and QM (i.e. monthly) vs. Q3M (i.e. quarterly) dosing) as well as considerations for subsequent years (e.g., switching from QM to Q3M dosing).

### Results:

The data demonstrated that BPS804 dosing regimens nearing maximal (> 75%) inhibition of sclerostin provide near maximal responses in circulating sclerostin, bone turnover markers (BTM), and lumbar spine bone mineral density (BMD). To demonstrate this, a range of doses from 0.1 mg/kg to 20mg/kg were simulated using QM (i.e. monthly) and Q3M (i.e. quarterly) intervals for a two-year time-course. Results were evaluated assuming full BPS804 exposure effects on sclerostin and relative to maximal inhibition. Overall, the 20 mg/kg QM dose approached the maximal sclerostin response. The maximum level of inhibition was reached by both monthly and quarterly dosing regimens, alike, and both the QM and Q3M 20mg/kg dosing regimens reached the maximum inhibition level.

The modeling results further indicated that a BPS804 20mg/kg QM dose would provide similar or a slightly greater 12-month BMD increase compared with Q3M dosing due to the longer sustained inhibition of sclerostin with BPS804.

Following one-year of treatment with BPS804 20 mg/kg QM, nearly the same peak maximum sclerostin response can also achieved with a 20 mg/kg dose administered quarterly. This potential change in dosing regimen after one-year of dosing could reflect the attainment of an apparent new steady-state in the bone remodeling system after one year of sclerostin inhibition therapy. Therefore, less frequent dosing after the first year of dosing may allow for maintenance of BMD increases from the first year of dosing.

In summary, the modelling data show that BPS804 20 mg/kg QM dosing is expected to provide near maximal inhibition of sclerostin that would translate into maximal BMD response. In addition, extending dosing intervals for longer-term dosing (e.g., switching to dosing every two months (Q2M) or Q3M following one-year of QM dosing) may be advantageous given the observed changes in bone turnover markers following the first year of treatment.

The patents and publications listed herein describe the general skill in the art. In the case of any conflict between a cited reference and this specification, the specification shall control. In describing embodiments of the present application, specific terminology is employed for the sake of clarity. All examples presented are representative and non-limiting.

## Claims

1. An anti-sclerostin antibody for use in treating osteogenesis imperfecta (OI) in a human patient comprising administering to the human patient a therapeutically effective amount of the anti-sclerostin antibody, wherein:
(i) the therapeutically effective amount of the anti-sclerostin antibody is 20 mg per kg body weight of the human patient administered on a monthly basis; and
(ii) the anti-sclerostin antibody comprises:
(a) a heavy chain variable region CDR1 comprising an amino acid sequence set forth in SEQ ID NO:4;
(b) a heavy chain variable region CDR2 comprising an amino acid sequence set forth in SEQ ID NO:15;
(c) a heavy chain variable region CDR3 comprising an amino acid sequence set forth in SEQ ID NO:26;
(d) a light chain variable region CDR1 comprising an amino acid sequence set forth in SEQ ID NO: 37;
(e) a light chain variable region CDR2 comprising an amino acid sequence set forth in SEQ ID NO:48; and
(f) a light chain variable region CDR3 comprising an amino acid sequence set forth in SEQ ID NO: 59.

2. The anti-sclerostin antibody for use according to claim 1, wherein the OI is type I OI, type III OI or type IV OI.

3. The anti-sclerostin antibody for use according to claim 1 or claim 2, wherein the human patient has one or more mutations in the COL1A1 and/ or COL1A2 genes.

4. The anti-sclerostin antibody for use according to any preceding claim, wherein the anti-sclerostin antibody comprises:
a) a VH polypeptide sequence having at least 90 percent sequence identity to the amino acid sequences set forth as SEQ ID NO:70; and/ or
b) a VL polypeptide sequence having at least 90 percent sequence identity to the amino acid sequences set forth as SEQ ID NO:81.

5. The anti-sclerostin antibody for use according to any preceding claim, wherein the anti-sclerostin antibody comprises:
a) a full length heavy chain amino acid sequence having at least 90 percent sequence identity to the amino acid sequence set forth as SEQ ID NO: 172; and/ or
b) a full length light chain amino acid sequence having at least 90 percent sequence identity to the amino acid sequence set forth as SEQ ID NO: 173.

6. The anti-sclerostin antibody for use according to any preceding claim, wherein the anti-sclerostin antibody comprises a VL polypeptide sequence comprising the amino acid sequence set forth as SEQ ID NO:81 and a VH polypeptide sequence comprising the amino acid sequence set forth as SEQ ID NO:70.

7. The anti-sclerostin antibody for use according to any preceding claim, wherein the anti-sclerostin antibody comprises a full length light chain amino acid sequence comprising the amino acid sequence set forth as SEQ ID NO: 173 and a full length heavy chain amino acid sequence comprising the amino acid sequence set forth as SEQ ID NO: 172.

8. The anti-sclerostin antibody for use according to any preceding claim, wherein the treatment regimen comprises a first dosing regimen of 20 mg per kg body weight of the human patient administered on a monthly basis followed by a second dosing regimen.

9. The anti-sclerostin antibody for use according to claim 8, wherein:
(a) the second dosing regimen is 1-50 mg per kg body weight of the human patient administered on a bi-monthly or quarterly basis, for example the second dosing regimen is 20 mg per kg body weight of the human patient administered on a bi-monthly or quarterly basis; or
(b) the first dosing regimen is 20 mg per kg body weight of the human patient administered on a monthly basis for a period of 1 year, and the second dosing regimen is 20 mg per kg body weight of the human patient administered on a bi-monthly or quarterly basis for a period of at least 1 year.

10. The anti-sclerostin antibody for use according to any preceding claim, wherein the anti-sclerostin antibody is administered intravenously.

11. The anti-sclerostin antibody for use according to any preceding claim, comprising administering a further therapeutic agent, such as bisphosphonate, parathyroid hormone, calcilytics, calcimimetics (e.g., cinacalcet), statins, anabolic steroids, lanthanum and strontium salts, and/or sodium fluoride.

12. The anti-sclerostin antibody for use according to any preceding claim, wherein the antibody is formulated in a pharmaceutical composition.

## Patentansprüche

1. Anti-Sclerostin-Antikörper zur Verwendung in der Behandlung von Osteogenesis imperfecta (OI) bei einem menschlichen Patienten, umfassend das Verabreichen einer therapeutisch wirksamen Menge des Anti-Sclerostin-Antikörpers an den menschlichen Patienten, wobei:
(i) die therapeutisch wirksame Menge des Anti-Sclerostin-Antikörpers 20 mg pro kg Körpergewicht des menschlichen Patienten, verabreicht einmal pro Monat, beträgt; und
(ii) der Anti-Sclerostin-Antikörper Folgendes umfasst:
(a) eine variable Region CDR1 der Schwerkette, die eine Aminosäuresequenz gemäß den Angaben in der SEQ ID Nr. 4 umfasst;
(b) eine variable Region CDR2 der Schwerkette, die eine Aminosäuresequenz gemäß den Angaben in der SEQ ID Nr. 15 umfasst;
(c) eine variable Region CDR3 der Schwerkette, die eine Aminosäuresequenz gemäß den Angaben in der SEQ ID Nr. 26 umfasst;
(d) eine variable Region CDR1 der Leichtkette, die eine Aminosäuresequenz gemäß den Angaben in der SEQ ID Nr. 37 umfasst;
(e) eine variable Region CDR2 der Leichtkette, die eine Aminosäuresequenz gemäß den Angaben in der SEQ ID Nr. 48 umfasst; und
(f) eine variable Region CDR3 der Leichtkette, die eine Aminosäuresequenz gemäß den Angaben in der SEQ ID Nr. 59 umfasst.

2. Anti-Sclerostin-Antikörper zur Verwendung gemäß Anspruch 1, wobei es sich bei der OI um OI Typ I, OI Typ III oder OI Typ IV handelt.

3. Anti-Sclerostin-Antikörper zur Verwendung gemäß Anspruch 1 oder Anspruch 2, wobei der menschliche Patient eine oder mehrere Mutationen in den Genen COL1A1 und/oder COL1A2 aufweist.

4. Anti-Sclerostin-Antikörper zur Verwendung gemäß einem beliebigen vorhergehenden Anspruch, wobei der Anti-Sclerostin-Antikörper Folgendes umfasst:
a) eine VH-Polypeptidsequenz, die eine Sequenzübereinstimmung von mindestens 90 Prozent mit den Aminosäuresequenzen gemäß den Angaben in der SEQ ID Nr. 70 aufweist; und/oder
b) eine VL-Polypeptidsequenz, die eine Sequenzübereinstimmung von mindestens 90 Prozent mit den Aminosäuresequenzen gemäß den Angaben in der SEQ ID Nr. 81 aufweist.

5. Anti-Sclerostin-Antikörper zur Verwendung gemäß einem beliebigen vorhergehenden Anspruch, wobei der Anti-Sclerostin-Antikörper Folgendes umfasst:
a) eine Aminosäuresequenz der Schwerkette in voller Länge, die eine Sequenzübereinstimmung von mindestens 90 Prozent mit der Aminosäuresequenz gemäß den Angaben in der SEQ ID Nr. 172 aufweist; und/oder
b) eine Aminosäuresequenz der Leichtkette in voller Länge, die eine Sequenzübereinstimmung von mindestens 90 Prozent mit der Aminosäuresequenz gemäß den Angaben in der SEQ ID Nr. 173 aufweist.

6. Anti-Sclerostin-Antikörper zur Verwendung gemäß einem beliebigen vorhergehenden Anspruch, wobei der Anti-Sclerostin-Antikörper eine VL-Polypeptidsequenz, welche die Aminosäuresequenz gemäß den Angaben in der SEQ ID Nr. 81 umfasst, und eine VH-Polypeptidsequenz umfasst, welche die Aminosäuresequenz gemäß den Angaben in der SEQ ID Nr. 70 umfasst.

7. Anti-Sclerostin-Antikörper zur Verwendung gemäß einem beliebigen vorhergehenden Anspruch, wobei der Anti-Sclerostin-Antikörper eine Aminosäuresequenz der Leichtkette in voller Länge, welche die Aminosäuresequenz gemäß den Angaben in der SEQ ID Nr. 173 umfasst, und eine Aminosäuresequenz der Schwerkette in voller Länge umfasst, welche die Aminosäuresequenz gemäß den Angaben in der SEQ ID Nr. 172 umfasst.

8. Anti-Sclerostin-Antikörper zur Verwendung gemäß einem beliebigen vorhergehenden Anspruch, wobei das Behandlungsschema ein erstes Dosierschema von 20 mg pro kg Körpergewicht des menschlichen Patienten, verabreicht einmal pro Monat, gefolgt von einem zweiten Dosierschema, umfasst.

9. Anti-Sclerostin-Antikörper zur Verwendung gemäß Anspruch 8:
(a) das zweite Dosierschema darin besteht, dass 1 bis 50 mg pro kg Körpergewicht des menschlichen Patienten alle zwei Monate oder einmal pro Quartal verabreicht werden, wobei das zweite Dosierschema beispielsweise darin besteht, dass 20 mg pro kg Körpergewicht des menschlichen Patienten alle zwei Monate oder einmal pro Quartal verabreicht werden; und/oder
(b) das erste Dosierschema darin besteht, dass 20 mg pro kg Körpergewicht des menschlichen Patienten einmal pro Monat über einen Zeitraum von 1 Jahr verabreicht werden, und das zweite Dosierschema darin besteht, dass 20 mg pro kg Körpergewicht des menschlichen Patienten alle zwei Monate oder einmal pro Quartal über einen Zeitraum von mindestens 1 Jahr verabreicht werden.

10. Anti-Sclerostin-Antikörper zur Verwendung gemäß einem beliebigen vorhergehenden Anspruch, wobei der Anti-Sclerostin-Antikörper intravenös verabreicht wird.

11. Anti-Sclerostin-Antikörper zur Verwendung gemäß einem beliebigen vorhergehenden Anspruch, umfassend die Verabreichung eines weiteren therapeutischen Mittels, wie etwa Bisphosphonat, Nebenschilddrüsenhormon, Calcilytika, Calcimimetika (z.B. Cinacalcet), Statine, anabole Steroide, Lanthan- und Strontiumsalze und/oder Natriumfluorid.

12. Anti-Sclerostin-Antikörper zur Verwendung gemäß einem beliebigen vorhergehenden Anspruch, wobei der Antikörper in einer pharmazeutischen Zusammensetzung formuliert ist.

## Revendications

1. Anticorps anti-sclérostine pour une utilisation dans le traitement de l'ostéogenèse imparfaite (OI) chez un patient humain comprenant l'administration au patient humain d'une quantité thérapeutiquement efficace de l'anticorps anti-sclérostine, dans lequel :
(i) la quantité thérapeutiquement efficace de l'anticorps anti-sclérostine est de 20 mg par kg de poids corporel du patient humain administrés sur une base mensuelle ; et
(ii) l'anticorps anti-sclérostine comprenant :
(a)une région variable de chaîne lourde CDR1 comprenant la séquence d'acides aminés présentée dans SEQ ID NO: 4 ;
(b)une région variable de chaîne lourde CDR2 comprenant une séquence d'acides aminés présentée dans SEQ ID NO: 15 ;
(c)une région variable de chaîne lourde CDR3 comprenant une séquence d'acides aminés présentée dans SEQ ID NO: 26 ;
(d)une région variable de chaîne légère CDR1 comprenant une séquence d'acides aminés présentée dans SEQ ID NO: 37 ;
(e)une région variable de chaîne légère CDR2 comprenant une séquence d'acides aminés présentée dans SEQ ID NO: 48 ; et
(f)une région variable de chaîne légère CDR3 comprenant une séquence d'acides aminés présentée dans SEQ ID NO: 59.

2. Anticorps anti-sclérostine pour une utilisation selon la revendication 1, dans lequel l'OI est une OI de type I, une OI de type III ou une OI de type IV.

3. Anticorps anti-sclérostine pour une utilisation selon la revendication 1 ou la revendication 2, dans lequel le patient humain possède une ou plusieurs mutations dans les gènes COL1A1 et/ou COL1A2.

4. Anticorps anti-sclérostine pour une utilisation selon l'une quelconque des revendications précédentes, l'anticorps anti-sclérostine comprenant :
a) une séquence polypeptidique de VH ayant une identité de séquence d'au moins 90 pour cent avec les séquences d'acides aminés représentées par SEQ ID NO: 70 ; et/ou
b) une séquence polypeptidique de VL ayant une identité de séquence d'au moins 90 pour cent avec les séquences d'acides aminés représentées par SEQ ID NO: 81.

5. Anticorps anti-sclérostine pour une utilisation selon l'une quelconque des revendications précédentes, l'anticorps anti-sclérostine comprenant :
a) une séquence d'acides aminés de chaîne lourde pleine longueur ayant une identité de séquence d'au moins 90 pour cent avec la séquence d'acides aminés représentée par SEQ ID NO: 172 ; et/ou
b)une séquence d'acides aminés de chaîne légère pleine longueur ayant une identité de séquence d'au moins 90 pour cent avec la séquence d'acides aminés représentée par SEQ ID NO: 173.

6. Anticorps anti-sclérostine pour une utilisation selon l'une quelconque des revendications précédentes, l'anticorps anti-sclérostine comprenant une séquence polypeptidique de VL comprenant la séquence d'acides aminés représentée par SEQ ID NO: 81 et une séquence polypeptidique de VH comprenant la séquence d'acides aminés représentée par SEQ ID NO: 70.

7. Anticorps anti-sclérostine pour une utilisation selon l'une quelconque des revendications précédentes, l'anticorps anti-sclérostine comprenant une séquence d'acides aminés de chaîne légère pleine longueur comprenant la séquence d'acides aminés représentée par SEQ ID NO: 173 et une séquence d'acides aminés de chaîne lourde pleine longueur comprenant la séquence d'acides aminés représentée par SEQ ID NO: 172.

8. Anticorps anti-sclérostine pour une utilisation selon l'une quelconque des revendications précédentes, le schéma posologique comprenant un premier schéma posologique de 20 mg par kg de poids corporel du patient humain administrés sur une base mensuelle suivi d'un second schéma posologique.

9. Anticorps anti-sclérostine pour une utilisation selon la revendication 8, dans lequel :
(a) le second schéma posologique est de 1 à 50 mg par kg de poids corporel du patient humain administrés sur une base bimestrielle ou trimestrielle, par exemple le second schéma posologique est de 20 mg par kg de poids corporel du patient humain administrés sur une base bimestrielle ou trimestrielle ; ou
(b)le premier schéma posologique est de 20 mg par kg de poids corporel du patient humain administrés sur une base mensuelle sur une période de 1 an, et le second schéma posologique est de 20 mg par kg de poids corporel du patient humain administrés sur une base bimestrielle ou trimestrielle sur une période d'au moins 1 an.

10. Anticorps anti-sclérostine pour une utilisation selon l'une quelconque des revendications précédentes, l'anticorps anti-sclérostine étant administré par voie intraveineuse.

11. Anticorps anti-sclérostine pour une utilisation selon l'une quelconque des revendications précédentes, comprenant l'administration d'un agent thérapeutique supplémentaire, tel que le bisphosphonate, l'hormone parathyroïdienne, les calcilytiques, les calcimimétiques (par exemple le cinacalcet), les statines, les stéroïdes anabolisants, les sels de lanthane et de strontium, et/ou le fluorure de sodium.

12. Anticorps anti-sclérostine pour une utilisation selon l'une quelconque des revendications précédentes, l'anticorps étant formulé dans une composition pharmaceutique.


### SEQUENCE LISTING

<110> MEREO BIOPHARMA 3 LIMITED
<120> USE OF ANTI-SCLEROSTIN ANTIBODIES IN THE TREATMENT OF OSTEOGENESIS IMPERFECTA
<130> P069492-US
<160> 177
<170> SeqWin2010, version 1.0
<210> 1
<211> 10
<212> PRT
<213> Homo sapiens
<400> 1
   Gly Phe Thr Phe Ser Ser Tyr Val Met Asn
   1               5                   10
<210> 2
<211> 10
<212> PRT
<213> Homo sapiens
<400> 2
   Gly Phe Thr Phe Arg Ser His Trp Leu Ser
   1               5                   10
<210> 3
<211> 10
<212> PRT
<213> Homo sapiens
<400> 3
   Gly Phe Thr Phe Ser Ser Tyr Val Met Asn
   1               5                   10
<210> 4
<211> 10
<212> PRT
<213> Homo sapiens
<400> 4
   Gly Phe Thr Phe Arg Ser His Trp Leu Ser
   1               5                   10
<210> 5
<211> 10
<212> PRT
<213> Homo sapiens
<400> 5
   Gly Phe Thr Phe Arg Ser His Trp Leu Ser
   1               5                   10
<210> 6
<211> 10
<212> PRT
<213> Homo sapiens
<400> 6
   Gly Phe Thr Phe Arg Ser His Trp Leu Ser
   1               5                   10
<210> 7
<211> 10
<212> PRT
<213> Homo sapiens
<400> 7
   Gly Phe Thr Phe Arg Ser His Trp Leu Ser
   1               5                   10
<210> 8
<211> 10
<212> PRT
<213> Homo sapiens
<400> 8
   Gly Phe Thr Phe Arg Ser His Trp Leu Ser
   1               5                   10
<210> 9
<211> 10
<212> PRT
<213> Homo sapiens
<400> 9
   Gly Phe Thr Phe Arg Ser His Trp Leu Ser
   1               5                   10
<210> 10
<211> 10
<212> PRT
<213> Homo sapiens
<400> 10
   Gly Phe Thr Phe Arg Ser His Trp Leu Ser
   1               5                   10
<210> 11
<211> 10
<212> PRT
<213> Homo sapiens
<400> 11
   Gly Phe Thr Phe Arg Ser His Trp Leu Ser
   1               5                   10
<210> 12
<211> 20
<212> PRT
<213> Homo sapiens
<400> 12
   Trp Val Ser Phe Ile Ser Gly Asp Ser Ser Asn Thr Tyr Tyr Ala Asp
   1               5                   10                  15
   Ser Val Lys Gly
               20
<210> 13
<211> 20
<212> PRT
<213> Homo sapiens
<400> 13
   Trp Val Ser Asn Ile Asn Tyr Asp Gly Ser Ser Thr Tyr Tyr Ala Asp
   1               5                   10                  15
   Ser Val Lys Gly
               20
<210> 14
<211> 20
<212> PRT
<213> Homo sapiens
<400> 14
   Trp Val Ser Phe Ile Ser Gly Asp Ser Ser Asn Thr Tyr Tyr Ala Asp
   1               5                   10                  15
   Ser Val Lys Gly
               20
<210> 15
<211> 20
<212> PRT
<213> Homo sapiens
<400> 15
   Trp Val Ser Asn Ile Asn Tyr Asp Gly Ser Ser Thr Tyr Tyr Ala Asp
   1               5                   10                  15
   Ser Val Lys Gly
               20
<210> 16
<211> 19
<212> PRT
<213> Homo sapiens
<400> 16
   Trp Val Ser Val Thr Gly Val His Gly Asp Thr Tyr Tyr Ala Asp Ser
   1 5 10 15
   Val Lys Gly
<210> 17
<211> 19
<212> PRT
<213> Homo sapiens
<400> 17
   Trp Val Ser Val Ile Gly Asn Trp Gly Asp Thr Tyr Tyr Ala Asp Ser
   1 5 10 15
   Val Lys Gly
<210> 18
<211> 19
<212> PRT
<213> Homo sapiens
<400> 18
   Trp Val Ser Val Thr Thr His Gln Gly Tyr Thr Tyr Tyr Ala Asp Ser
   1 5 10 15
   Val Lys Gly
<210> 19
<211> 19
<212> PRT
<213> Homo sapiens
<400> 19
   Trp Val Ser Ala Thr Asn Arg Tyr Gly Tyr Thr Tyr Tyr Ala Asp Ser
   1 5 10 15
   Val Lys Gly
<210> 20
<211> 20
<212> PRT
<213> Homo sapiens
<400> 20
   Trp Val Ser Asn Ile Asn Tyr Asp Gly Ser Ser Thr Tyr Tyr Ala Asp
   1 5 10 15
   Ser Val Lys Gly
   20
<210> 21
<211> 19
<212> PRT
<213> Homo sapiens
<400> 21
   Trp Val Ser Val Ile Thr Pro Tyr Gly Asp Thr Tyr Tyr Ala Asp Ser
   1               5                   10                  15
   Val Lys Gly
<210> 22
<211> 19
<212> PRT
<213> Homo sapiens
<400> 22
   Trp Val Ser Val Ile Thr Pro Tyr Gly Asp Thr Tyr Tyr Ala Asp Ser
   1               5                   10                  15
   Val Lys Gly
<210> 23
<211> 15
<212> PRT
<213> Homo sapiens
<400> 23
   Thr Phe Met His Gly His Leu Gly Gly Gly Leu Ser Met Asp Phe
   1               5                   10                  15
<210> 24
<211> 8
<212> PRT
<213> Homo sapiens
<400> 24
   Asp Thr Tyr Leu His Phe Asp Tyr
   1               5
<210> 25
<211> 15
<212> PRT
<213> Homo sapiens
<400> 25
   Thr Phe Met His Gly His Leu Gly Gly Gly Leu Ser Met Asp Phe
   1               5                   10                  15
<210> 26
<211> 8
<212> PRT
<213> Homo sapiens
<400> 26
   Asp Thr Tyr Leu His Phe Asp Tyr
   1               5
<210> 27
<211> 8
<212> PRT
<213> Homo sapiens
<400> 27
   Asp Thr Tyr Leu His Phe Asp Tyr
   1               5
<210> 28
<211> 8
<212> PRT
<213> Homo sapiens
<400> 28
   Asp Thr Tyr Leu His Phe Asp Tyr
   1               5
<210> 29
<211> 8
<212> PRT
<213> Homo sapiens
<400> 29
   Asp Thr Tyr Leu His Phe Asp Tyr
   1               5
<210> 30
<211> 8
<212> PRT
<213> Homo sapiens
<400> 30
   Asp Thr Tyr Leu His Phe Asp Tyr
   1               5
<210> 31
<211> 8
<212> PRT
<213> Homo sapiens
<400> 31
   Asp Thr Tyr Leu His Phe Asp Tyr
   1               5
<210> 32
<211> 8
<212> PRT
<213> Homo sapiens
<400> 32
   Asp Thr Tyr Leu His Phe Asp Tyr
   1               5
<210> 33
<211> 8
<212> PRT
<213> Homo sapiens
<400> 33
   Asp Thr Tyr Leu His Phe Asp Tyr
   1               5
<210> 34
<211> 11
<212> PRT
<213> Homo sapiens
<400> 34
   Ser Gly Asp Asn Ile Gly Ser Phe Tyr Val His
   1               5                   10
<210> 35
<211> 20
<212> PRT
<213> Homo sapiens
<400> 35
   Trp Val Ser Asn Ile Asn Tyr Asp Gly Ser Ser Thr Tyr Tyr Ala Asp
   1               5                   10                  15
   Ser Val Lys Gly
               20
<210> 36
<211> 11
<212> PRT
<213> Homo sapiens
<400> 36
   Ser Gly Asp Asn Ile Gly Ser Phe Tyr Val His
   1               5                   10
<210> 37
<211> 14
<212> PRT
<213> Homo sapiens
<400> 37
   Thr Gly Thr Ser Ser Asp Val Gly Asp Ile Asn Asp Val Ser
   1               5                   10
<210> 38
<211> 14
<212> PRT
<213> Homo sapiens
<400> 38
   Thr Gly Thr Ser Ser Asp Val Gly Asp Ile Asn Asp Val Ser
   1               5                   10
<210> 39
<211> 14
<212> PRT
<213> Homo sapiens
<400> 39
   Thr Gly Thr Ser Ser Asp Val Gly Asp Ile Asn Asp Val Ser
   1               5                   10
<210> 40
<211> 14
<212> PRT
<213> Homo sapiens
<400> 40
   Thr Gly Thr Ser Ser Asp Val Gly Asp Ile Asn Asp Val Ser
   1               5                   10
<210> 41
<211> 14
<212> PRT
<213> Homo sapiens
<400> 41
   Thr Gly Thr Ser Ser Asp Val Gly Asp Ile Asn Asp Val Ser
   1               5                   10
<210> 42
<211> 14
<212> PRT
<213> Homo sapiens
<400> 42
   Thr Gly Thr Ser Ser Asp Val Gly Asp Ile Asn Asp Val Ser
   1               5                   10
<210> 43
<211> 14
<212> PRT
<213> Homo sapiens
<400> 43
   Thr Gly Thr Ser Ser Asp Val Gly Asp Ile Asn Asp Val Ser
   1               5                   10
<210> 44
<211> 14
<212> PRT
<213> Homo sapiens
<400> 44
   Thr Gly Thr Ser Ser Asp Val Gly Asp Ile Asn Asp Val Ser
   1               5                   10
<210> 45
<211> 11
<212> PRT
<213> Homo sapiens
<400> 45
   Leu Val Ile Tyr Asp Asp Asn Asn Arg Pro Ser
   1               5                   10
<210> 46
<211> 11
<212> PRT
<213> Homo sapiens
<400> 46
   Leu Met Ile Tyr Asp Val Asn Asn Arg Pro Ser
   1 5 10
<210> 47
<211> 11
<212> PRT
<213> Homo sapiens
<400> 47
   Leu Val Ile Tyr Asp Asp Asn Asn Arg Pro Ser
   1 5 10
<210> 48
<211> 11
<212> PRT
<213> Homo sapiens
<400> 48
   Leu Met Ile Tyr Asp Val Asn Asn Arg Pro Ser
   1 5 10
<210> 49
<211> 11
<212> PRT
<213> Homo sapiens
<400> 49
   Leu Met Ile Tyr Asp Val Asn Asn Arg Pro Ser
   1 5 10
<210> 50
<211> 11
<212> PRT
<213> Homo sapiens
<400> 50
   Leu Met Ile Tyr Asp Val Asn Asn Arg Pro Ser
   1 5 10
<210> 51
<211> 11
<212> PRT
<213> Homo sapiens
<400> 51
   Leu Met Ile Tyr Asp Val Asn Asn Arg Pro Ser
   1 5 10
<210> 52
<211> 11
<212> PRT
<213> Homo sapiens
<400> 52
   Leu Met Ile Tyr Asp Val Asn Asn Arg Pro Ser
   1 5 10
<210> 53
<211> 11
<212> PRT
<213> Homo sapiens
<400> 53
   Leu Met Ile Tyr Asp Val Asn Asn Arg Pro Ser
   1 5 10
<210> 54
<211> 11
<212> PRT
<213> Homo sapiens
<400> 54
   Leu Met Ile Tyr Asp Val Asn Asn Arg Pro Ser
   1 5 10
<210> 55
<211> 11
<212> PRT
<213> Homo sapiens
<400> 55
   Leu Met Ile Tyr Asp Val Asn Asn Arg Pro Ser
   1 5 10
<210> 56
<211> 10
<212> PRT
<213> Homo sapiens
<400> 56
   Gly Ser Trp Ala Gly Ser Ser Gly Ser Tyr
   1 5 10
<210> 57
<211> 10
<212> PRT
<213> Homo sapiens
<400> 57
   Ser Ser Tyr Gly Glu Ser Leu Thr Ser Tyr
   1 5 10
<210> 58
<211> 10
<212> PRT
<213> Homo sapiens
<400> 58
   Ala Ser Trp Thr Gly Val Glu Pro Asp Tyr
   1 5 10
<210> 59
<211> 10
<212> PRT
<213> Homo sapiens
<400> 59
   Gln Ser Tyr Ala Gly Ser Tyr Leu Ser Glu
   1 5 10
<210> 60
<211> 10
<212> PRT
<213> Homo sapiens
<400> 60
   Ser Ser Tyr Gly Glu Ser Leu Thr Ser Tyr
   1 5 10
<210> 61
<211> 10
<212> PRT
<213> Homo sapiens
<400> 61
   Ser Ser Tyr Gly Glu Ser Leu Thr Ser Tyr
   1 5 10
<210> 62
<211> 10
<212> PRT
<213> Homo sapiens
<400> 62
   Ser Ser Tyr Gly Glu Ser Leu Thr Ser Tyr
   1 5 10
<210> 63
<211> 10
<212> PRT
<213> Homo sapiens
<400> 63
   Ser Ser Tyr Gly Glu Ser Leu Thr Ser Tyr
   1 5 10
<210> 64
<211> 10
<212> PRT
<213> Homo sapiens
<400> 64
   Ser Thr Tyr Asp Gly Pro Gly Leu Ser Glu
   1 5 10
<210> 65
<211> 10
<212> PRT
<213> Homo sapiens
<400> 65
   Ser Ser Tyr Gly Glu Ser Leu Thr Ser Tyr
   1 5 10
<210> 66
<211> 10
<212> PRT
<213> Homo sapiens
<400> 66
   Ser Ser Tyr Gly Glu Ser Leu Thr Ser Tyr
   1               5                   10
<210> 67
<211> 124
<212> PRT
<213> Homo sapiens
<400> 67
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Val Met Asn Trp Val Arg Gln Ala Pro Gly Lys Gly Leu Glu Trp Val
           35                  40                  45
   Ser Phe Ile Ser Gly Asp Ser Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Thr Phe Met His Gly His Leu Gly Gly Gly Leu Ser Met Asp
               100                 105                 110
   Phe Trp Gly Gln Gly Thr Leu Val Thr Val Ser Ser
           115                 120
<210> 68
<211> 117
<212> PRT
<213> Homo sapiens
<400> 68
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Asn Ile Asn Tyr Asp Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Thr Tyr Leu His Phe Asp Tyr Trp Gly Gln Gly Thr Leu
               100                 105                 110
   Val Thr Val Ser Ser
           115
<210> 69
<211> 124
<212> PRT
<213> Homo sapiens
<400> 69
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Val Met Asn Trp Val Arg Gln Ala Pro Gly Lys Gly Leu Glu Trp Val
           35                  40                  45
   Ser Phe Ile Ser Gly Asp Ser Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Thr Phe Met His Gly His Leu Gly Gly Gly Leu Ser Met Asp
               100                 105                 110
   Phe Trp Gly Gln Gly Thr Leu Val Thr Val Ser Ser
           115                 120
<210> 70
<211> 117
<212> PRT
<213> Homo sapiens
<400> 70
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Asn Ile Asn Tyr Asp Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Thr Tyr Leu His Phe Asp Tyr Trp Gly Gln Gly Thr Leu
               100                 105                 110
   Val Thr Val Ser Ser
           115
<210> 71
<211> 116
<212> PRT
<213> Homo sapiens
<400> 71
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Val Thr Gly Val His Gly Asp Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Thr Tyr Leu His Phe Asp Tyr Trp Gly Gln Gly Thr Leu Val
               100                 105                 110
   Thr Val Ser Ser
           115
<210> 72
<211> 116
<212> PRT
<213> Homo sapiens
<400> 72
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Val Ile Gly Asn Trp Gly Asp Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Thr Tyr Leu His Phe Asp Tyr Trp Gly Gln Gly Thr Leu Val
               100                 105                 110
   Thr Val Ser Ser
           115
<210> 73
<211> 116
<212> PRT
<213> Homo sapiens
<400> 73
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Val Thr Thr His Gln Gly Tyr Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Thr Tyr Leu His Phe Asp Tyr Trp Gly Gln Gly Thr Leu Val
               100                 105                 110
   Thr Val Ser Ser
           115
<210> 74
<211> 116
<212> PRT
<213> Homo sapiens
<400> 74
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Ala Thr Asn Arg Tyr Gly Tyr Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Thr Tyr Leu His Phe Asp Tyr Trp Gly Gln Gly Thr Leu Val
               100                 105                 110
   Thr Val Ser Ser
           115
<210> 75
<211> 117
<212> PRT
<213> Homo sapiens
<400> 75
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Asn Ile Asn Tyr Asp Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Thr Tyr Leu His Phe Asp Tyr Trp Gly Gln Gly Thr Leu
               100                 105                 110
   Val Thr Val Ser Ser
           115
<210> 76
<211> 116
<212> PRT
<213> Homo sapiens
<400> 76
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Val Ile Thr Pro Tyr Gly Asp Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Thr Tyr Leu His Phe Asp Tyr Trp Gly Gln Gly Thr Leu Val
               100                 105                 110
   Thr Val Ser Ser
           115
<210> 77
<211> 116
<212> PRT
<213> Homo sapiens
<400> 77
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Val Ile Thr Pro Tyr Gly Asp Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Thr Tyr Leu His Phe Asp Tyr Trp Gly Gln Gly Thr Leu Val
               100                 105                 110
   Thr Val Ser Ser
           115
<210> 78
<211> 110
<212> PRT
<213> Homo sapiens
<400> 78
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Gly Ser Phe Tyr Val
               20                  25                  30
   His Trp Tyr Gln Gln Lys Pro Gly Gln Ala Pro Val Leu Val Ile Tyr
           35                  40                  45
   Asp Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Gly Ser Trp Ala Gly Ser Ser Gly Ser
                   85                  90                  95
   Tyr Val Phe Gly Gly Arg Thr Lys Leu Thr Val Leu Gly Gln
               100                 105                 110
<210> 79
<211> 113
<212> PRT
<213> Homo sapiens
<400> 79
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Glu Ser
                   85                  90                  95
   Leu Thr Ser Tyr Val Phe Gly Gly Gly Thr Lys Leu Thr Val Leu Gly
               100                 105                 110
   Gln
<210> 80
<211> 110
<212> PRT
<213> Homo sapiens
<400> 80
   Asp Ile Glu Leu Thr Gln Pro Pro Ser Val Ser Val Ala Pro Gly Gln
   1               5                   10                  15
   Thr Ala Arg Ile Ser Cys Ser Gly Asp Asn Ile Gly Ser Phe Tyr Val
               20                  25                  30
   His Trp Tyr Gln Gln Lys Pro Gly Gln Ala Pro Val Leu Val Ile Tyr
           35                  40                  45
   Asp Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ala Ser Trp Thr Gly Val Glu Pro Asp
                   85                  90                  95
   Tyr Val Phe Gly Gly Gly Thr Lys Leu Thr Val Leu Gly Gln
               100                 105                 110
<210> 81
<211> 113
<212> PRT
<213> Homo sapiens
<400> 81
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Tyr Ala Gly Ser
                   85                  90                  95
   Tyr Leu Ser Glu Val Phe Gly Gly Gly Thr Lys Leu Thr Val Leu Gly
               100                 105                 110
   Gln
<210> 82
<211> 113
<212> PRT
<213> Homo sapiens
<400> 82
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Glu Ser
                   85                  90                  95
   Leu Thr Ser Tyr Val Phe Gly Gly Gly Thr Lys Leu Thr Val Leu Gly
               100                 105                 110
   Gln
<210> 83
<211> 113
<212> PRT
<213> Homo sapiens
<400> 83
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Glu Ser
                   85                  90                  95
   Leu Thr Ser Tyr Val Phe Gly Gly Gly Thr Lys Leu Thr Val Leu Gly
               100                 105                 110
   Gln
<210> 84
<211> 113
<212> PRT
<213> Homo sapiens
<400> 84
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Glu Ser
                   85                  90                  95
   Leu Thr Ser Tyr Val Phe Gly Gly Gly Thr Lys Leu Thr Val Leu Gly
               100                 105                 110
   Gln
<210> 85
<211> 113
<212> PRT
<213> Homo sapiens
<400> 85
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Glu Ser
                   85                  90                  95
   Leu Thr Ser Tyr Val Phe Gly Gly Gly Thr Lys Leu Thr Val Leu Gly
               100                 105                 110
   Gln
<210> 86
<211> 113
<212> PRT
<213> Homo sapiens
<400> 86
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Thr Tyr Asp Gly Pro
                   85                  90                  95
   Gly Leu Ser Glu Val Phe Gly Gly Gly Thr Lys Leu Thr Val Leu Gly
               100                 105                 110
   Gln
<210> 87
<211> 113
<212> PRT
<213> Homo sapiens
<400> 87
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Glu Ser
                   85                  90                  95
   Leu Thr Ser Tyr Val Phe Gly Gly Gly Thr Lys Leu Thr Val Leu Gly
               100                 105                 110
   Gln
<210> 88
<211> 113
<212> PRT
<213> Homo sapiens
<400> 88
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Thr Tyr Asp Gly Pro
                   85                  90                  95
   Gly Leu Ser Glu Val Phe Gly Gly Gly Thr Lys Leu Thr Val Leu Gly
               100                 105                 110
   Gln
<210> 89
<211> 372
<212> DNA
<213> Homo sapiens
<400> 89
   caggtgcaat tggtggaaag cggcggcggc ctggtgcaac cgggcggcag cctgcgtctg 60
   agctgcgcgg cctccggatt tacctttcct tcttatgtta tgaattgggt gcgccaagcc 120
   cctgggaagg gtctcgagtg ggtgagcttt atctctggtg attctagcaa tacctattat 180
   gcggatagcg tgaaaggccg ttttaccatt tcacgtgata attcgaaaaa caccctgtat 240
   ctgcaaatga acagcctgcg tgcggaagat acggccgtgt attattgcgc gcgtactttt 300
   atgcatggtc atcttggtgg tggtctttct atggattttt ggggccaagg caccctggtg 360
   acggttagct ca 372
<210> 90
<211> 351
<212> DNA
<213> Homo sapiens
<400> 90
   caggtgcaat tggtggaaag cggcggcggc ctggtgcaac cgggcggcag cctgcgtctg 60
   agctgcgcgg cctccggatt tacctttcgt tctcattggc tttcttgggt gcgccaagcc 120
   cctgggaagg gtctcgagtg ggtgagcaat atcaattatg atggtagctc tacctattat 180
   gcggatagcg tgaaaggccg ttttaccatt tcacgtgata attcgaaaaa caccctgtat 240
   ctgcaaatga acagcctgcg tgcggaagat acggccgtgt attattgcgc gcgtgatact 300
   tatcttcatt ttgattattg gggccaaggc accctggtga cggttagctc a 351
<210> 91
<211> 372
<212> DNA
<213> Homo sapiens
<400> 91
   caggtgcaat tggtggaaag cggcggcggc ctggtgcaac cgggcggcag cctgcgtctg  60
   agctgcgcgg cctccggatt taccttttct tcttatgtta tgaattgggt gcgccaagcc  120
   cctgggaagg gtctcgagtg ggtgagcttt atctctggtg attctagcaa tacctattat  180
   gcggatagcg tgaaaggccg ttttaccatt tcacgtgata attcgaaaaa caccctgtat  240
   ctgcaaatga acagcctgcg tgcggaagat acggccgtgt attattgcgc gcgtactttt  300
   atgcatggtc atcttggtgg tggtctttct atggattttt ggggccaagg caccctggtg  360
   acggttagct ca                                                      372
<210> 92
<211> 351
<212> DNA
<213> Homo sapiens
<400> 92
   caggtgcaat tggtggaaag cggcggcggc ctggtgcaac cgggcggcag cctgcgtctg  60
   agctgcgcgg cctccggatt tacctttcgt tctcattggc tttcttgggt gcgccaagcc  120
   cctgggaagg gtctcgagtg ggtgagcaat atcaattatg atggtagctc tacctattat  180
   gcggatagcg tgaaaggccg ttttaccatt tcacgtgata attcgaaaaa caccctgtat  240
   ctgcaaatga acagcctgcg tgcggaagat acggccgtgt attattgcgc gcgtgatact  300
   tatcttcatt ttgattattg gggccaaggc accctggtga cggttagctc a           351
<210> 93
<211> 348
<212> DNA
<213> Homo sapiens
<400> 93
   caggtgcaat tggtggaaag cggcggcggc ctggtgcaac cgggcggcag cctgcgtctg  60
   agctgcgcgg cctccggatt tacctttcgt tctcattggc tttcttgggt gcgccaagcc  120
   cctgggaagg gtctcgagtg ggtgagcgtt actggtgttc atggtgatac ttattatgct  180
   gattctgtta agggtcgttt taccatttca cgtgataatt cgaaaaacac cctgtatctg  240
   caaatgaaca gcctgcgtgc ggaagatacg gccgtgtatt attgcgcgcg tgatacttat  300
   cttcattttg attattgggg ccaaggcacc ctggtgacgg ttagctca              348
<210> 94
<211> 348
<212> DNA
<213> Homo sapiens
<400> 94
   caggtgcaat tggtggaaag cggcggcggc ctggtgcaac cgggcggcag cctgcgtctg  60
   agctgcgcgg cctccggatt tacctttcgt tctcattggc tttcttgggt gcgccaagcc  120
   cctgggaagg gtctcgagtg ggtgagcgtt attggtaatt ggggtgatac ttattatgct  180
   gattctgtta agggtcgttt taccatttca cgtgataatt cgaaaaacac cctgtatctg  240
   caaatgaaca gcctgcgtgc ggaagatacg gccgtgtatt attgcgcgcg tgatacttat  300
   cttcattttg attattgggg ccaaggcacc ctggtgacgg ttagctca              348
<210> 95
<211> 348
<212> DNA
<213> Homo sapiens
<400> 95
   caggtgcaat tggtggaaag cggcggcggc ctggtgcaac cgggcggcag cctgcgtctg  60
   agctgcgcgg cctccggatt tacctttcgt tctcattggc tttcttgggt gcgccaagcc  120
   cctgggaagg gtctcgagtg ggtgagcgtt actactcatc agggttatac ttattatgct  180
   gattctgtta agggtcgttt taccatttca cgtgataatt cgaaaaacac cctgtatctg 240
   caaatgaaca gcctgcgtgc ggaagatacg gccgtgtatt attgcgcgcg tgatacttat 300
   cttcattttg attattgggg ccaaggcacc ctggtgacgg ttagctca 348
<210> 96
<211> 348
<212> DNA
<213> Homo sapiens
<400> 96
   caggtgcaat tggtggaaag cggcggcggc ctggtgcaac cgggcggcag cctgcgtctg 60
   agctgcgcgg cctccggatt tacctttcgt tctcattggc tttcttgggt gcgccaagcc 120
   cctgggaagg gtctcgagtg ggtgagcgct actaatcgtt atggttatac ttattatgct 180
   gattctgtta agggtcgttt taccatttca cgtgataatt cgaaaaacac cctgtatctg 240
   caaatgaaca gcctgcgtgc ggaagatacg gccgtgtatt attgcgcgcg tgatacttat 300
   cttcattttg attattgggg ccaaggcacc ctggtgacgg ttagctca 348
<210> 97
<211> 351
<212> DNA
<213> Homo sapiens
<400> 97
   caggtgcaat tggtggaaag cggcggcggc ctggtgcaac cgggcggcag cctgcgtctg 60
   agctgcgcgg cctccggatt tacctttcgt tctcattggc tttcttgggt gcgccaagcc 120
   cctgggaagg gtctcgagtg ggtgagcaat atcaattatg atggtagctc tacctattat 180
   gcggatagcg tgaaaggccg ttttaccatt tcacgtgata attcgaaaaa caccctgtat 240
   ctgcaaatga acagcctgcg tgcggaagat acggccgtgt attattgcgc gcgtgatact 300
   tatcttcatt ttgattattg gggccaaggc accctggtga cggttagctc a 351
<210> 98
<211> 348
<212> DNA
<213> Homo sapiens
<400> 98
   caggtgcaat tggtggaaag cggcggcggc ctggtgcaac cgggcggcag cctgcgtctg 60
   agctgcgcgg cctccggatt tacctttcgt tctcattggc tttcttgggt gcgccaagcc 120
   cctgggaagg gtctcgagtg ggtgagcgtt attactcctt atggtgatac ttattatgct 180
   gattctgtta agggtcgttt taccatttca cgtgataatt cgaaaaacac cctgtatctg 240
   caaatgaaca gcctgcgtgc ggaagatacg gccgtgtatt attgcgcgcg tgatacttat 300
   cttcattttg attattgggg ccaaggcacc ctggtgacgg ttagctca 348
<210> 99
<211> 348
<212> DNA
<213> Homo sapiens
<400> 99
   caggtgcaat tggtggaaag cggcggcggc ctggtgcaac cgggcggcag cctgcgtctg 60
   agctgcgcgg cctccggatt tacctttcgt tctcattggc tttcttgggt gcgccaagcc 120
   cctgggaagg gtctcgagtg ggtgagcgtt attactcctt atggtgatac ttattatgct 180
   gattctgtta agggtcgttt taccatttca cgtgataatt cgaaaaacac cctgtatctg 240
   caaatgaaca gcctgcgtgc ggaagatacg gccgtgtatt attgcgcgcg tgatacttat 300
   cttcattttg attattgggg ccaaggcacc ctggtgacgg ttagctca 348
<210> 100
<211> 330
<212> DNA
<213> Homo sapiens
<400> 100
   gatatcgaac tgacccagcc gccttcagtg agcgttgcac caggtcagac cgcgcgtatc  60
   tcgtgtagcg gcgataatat tggttctttt tatgttcatt ggtaccagca gaaacccggg  120
   caggcgccag ttcttgtgat ttatgatgat aataatcgtc cctcaggcat cccggaacgc  180
   tttagcggat ccaacagcgg caacaccgcg accctgacca ttagcggcac tcaggcggaa  240
   gacgaagcgg attattattg cggttcttgg gctggttctt ctggttctta tgtgtttggc  300
   ggccgcacga agttaaccgt tcttggccag                                   330
<210> 101
<211> 339
<212> DNA
<213> Homo sapiens
<400> 101
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttatg gtgagtctct tacttcttat  300
   gtgtttggcg gcggcacgaa gttaaccgtt cttggccag                          339
<210> 102
<211> 330
<212> DNA
<213> Homo sapiens
<400> 102
   gatatcgaac tgacccagcc gccttcagtg agcgttgcac caggtcagac cgcgcgtatc  60
   tcgtgtagcg gcgataatat tggttctttt tatgttcatt ggtaccagca gaaacccggg  120
   caggcgccag ttcttgtgat ttatgatgat aataatcgtc cctcaggcat cccggaacgc  180
   tttagcggat ccaacagcgg caacaccgcg accctgacca ttagcggcac tcaggcggaa  240
   gacgaagcgg attattattg cgcttcttgg actggtgttg agcctgatta tgtgtttggc  300
   ggcggcacga agttaaccgt tcttggccag                                   330
<210> 103
<211> 339
<212> DNA
<213> Homo sapiens
<400> 103
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc cagtcttatg ctggttctta tctttctgag  300
   gtgtttggcg gcggcacgaa gttaaccgtt cttggccag                          339
<210> 104
<211> 339
<212> DNA
<213> Homo sapiens
<400> 104
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttatg gtgagtctct tacttcttat  300
   gtgtttggcg gcggcacgaa gttaaccgtt cttggccag                         339
<210> 105
<211> 339
<212> DNA
<213> Homo sapiens
<400> 105
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttatg gtgagtctct tacttcttat  300
   gtgtttggcg gcggcacgaa gttaaccgtt cttggccag                         339
<210> 106
<211> 339
<212> DNA
<213> Homo sapiens
<400> 106
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttatg gtgagtctct tacttcttat  300
   gtgtttggcg gcggcacgaa gttaaccgtt cttggccag                         339
<210> 107
<211> 339
<212> DNA
<213> Homo sapiens
<400> 107
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttatg gtgagtctct tacttcttat  300
   gtgtttggcg gcggcacgaa gttaaccgtt cttggccag                         339
<210> 108
<211> 339
<212> DNA
<213> Homo sapiens
<400> 108
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tctacttatg atggtcctgg tctttctgag  300
   gtgtttggcg gcggcacgaa gttaaccgtt cttggccag                         339
<210> 109
<211> 339
<212> DNA
<213> Homo sapiens
<400> 109
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttatg gtgagtctct tacttcttat  300
   gtgtttggcg gcggcacgaa gttaaccgtt cttggccag                         339
<210> 110
<211> 339
<212> DNA
<213> Homo sapiens
<400> 110
   gatatcgcac tgacccagcc agcttcagtg agcggctcac caggtcagag cattaccatc  60
   tcgtgtacgg gtactagcag cgatgttggt gatattaatg atgtgtcttg gtaccagcag  120
   catcccggga aggcgccgaa acttatgatt tatgatgtta ataatcgtcc ctcaggcgtg  180
   agcaaccgtt ttagcggatc caaaagcggc aacaccgcga gcctgaccat tagcggcctg  240
   caagcggaag acgaagcgga ttattattgc tctacttatg atggtcctgg tctttctgag  300
   gtgtttggcg gcggcacgaa gttaaccgtt cttggccag                         339
<210> 111
<211> 469
<212> PRT
<213> Homo sapiens
<400> 111
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Ser Ser Tyr Val Met Asn Trp Val Arg Gln Ala Pro Gly Lys Gly Leu
       50                  55                  60
   Glu Trp Val Ser Phe Ile Ser Gly Asp Ser Ser Asn Thr Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Thr Phe Met His Gly His Leu Gly Gly Gly Leu
           115                 120                 125
   Ser Met Asp Phe Trp Gly Gln Gly Thr Leu Val Thr Val Ser Ser Ala
       130                 135                 140
   Ser Thr Lys Gly Pro Ser Val Phe Pro Leu Ala Pro Cys Ser Arg Ser
   145                 150                 155                 160
   Thr Ser Glu Ser Thr Ala Ala Leu Gly Cys Leu Val Lys Asp Tyr Phe
                   165                 170                 175
   Pro Glu Pro Val Thr Val Ser Trp Asn Ser Gly Ala Leu Thr Ser Gly
               180                 185                 190
   Val His Thr Phe Pro Ala Val Leu Gln Ser Ser Gly Leu Tyr Ser Leu
           195                 200                 205
   Ser Ser Val Val Thr Val Pro Ser Ser Asn Phe Gly Thr Gln Thr Tyr
       210                 215                 220
   Thr Cys Asn Val Asp His Lys Pro Ser Asn Thr Lys Val Asp Lys Thr
   225                 230                 235                 240
   Val Glu Arg Lys Cys Cys Val Glu Cys Pro Pro Cys Pro Ala Pro Pro
                   245                 250                 255
   Val Ala Gly Pro Ser Val Phe Leu Phe Pro Pro Lys Pro Lys Asp Thr
               260                 265                 270
   Leu Met Ile Ser Arg Thr Pro Glu Val Thr Cys Val Val Val Asp Val
           275                 280                 285
   Ser His Glu Asp Pro Glu Val Gln Phe Asn Trp Tyr Val Asp Gly Val
       290                 295                 300
   Glu Val His Asn Ala Lys Thr Lys Pro Arg Glu Glu Gln Phe Asn Ser
   305                 310                 315                 320
   Thr Phe Arg Val Val Ser Val Leu Thr Val Val His Gln Asp Trp Leu
                   325                 330                 335
   Asn Gly Lys Glu Tyr Lys Cys Lys Val Ser Asn Lys Gly Leu Pro Ala
               340                 345                 350
   Pro Ile Glu Lys Thr Ile Ser Lys Thr Lys Gly Gln Pro Arg Glu Pro
           355                 360                 365
   Gln Val Tyr Thr Leu Pro Pro Ser Arg Glu Glu Met Thr Lys Asn Gln
       370                 375                 380
   Val Ser Leu Thr Cys Leu Val Lys Gly Phe Tyr Pro Ser Asp Ile Ala
   385                 390                 395                 400
   Val Glu Trp Glu Ser Asn Gly Gln Pro Glu Asn Asn Tyr Lys Thr Thr
                   405                 410                 415
   Pro Pro Met Leu Asp Ser Asp Gly Ser Phe Phe Leu Tyr Ser Lys Leu
               420                 425                 430
   Thr Val Asp Lys Ser Arg Trp Gln Gln Gly Asn Val Phe Ser Cys Ser
           435                 440                 445
   Val Met His Glu Ala Leu His Asn His Tyr Thr Gln Lys Ser Leu Ser
       450                 455                 460
   Leu Ser Pro Gly Lys
   465
<210> 112
<211> 462
<212> PRT
<213> Homo sapiens
<400> 112
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Asn Ile Asn Tyr Asp Gly Ser Ser Thr Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Thr Tyr Leu His Phe Asp Tyr Trp Gly Gln
           115                 120                 125
   Gly Thr Leu Val Thr Val Ser Ser Ala Ser Thr Lys Gly Pro Ser Val
       130                 135                 140
   Phe Pro Leu Ala Pro Cys Ser Arg Ser Thr Ser Glu Ser Thr Ala Ala
   145                 150                 155                 160
   Leu Gly Cys Leu Val Lys Asp Tyr Phe Pro Glu Pro Val Thr Val Ser
                   165                 170                 175
   Trp Asn Ser Gly Ala Leu Thr Ser Gly Val His Thr Phe Pro Ala Val
               180                 185                 190
   Leu Gln Ser Ser Gly Leu Tyr Ser Leu Ser Ser Val Val Thr Val Pro
           195                 200                 205
   Ser Ser Asn Phe Gly Thr Gln Thr Tyr Thr Cys Asn Val Asp His Lys
       210                 215                 220
   Pro Ser Asn Thr Lys Val Asp Lys Thr Val Glu Arg Lys Cys Cys Val
   225                 230                 235                 240
   Glu Cys Pro Pro Cys Pro Ala Pro Pro Val Ala Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Gln Phe Asn Trp Tyr Val Asp Gly Val Glu Val His Asn Ala Lys Thr
       290                 295                 300
   Lys Pro Arg Glu Glu Gln Phe Asn Ser Thr Phe Arg Val Val Ser Val
   305                 310                 315                 320
   Leu Thr Val Val His Gln Asp Trp Leu Asn Gly Lys Glu Tyr Lys Cys
                   325                 330                 335
   Lys Val Ser Asn Lys Gly Leu Pro Ala Pro Ile Glu Lys Thr Ile Ser
               340                 345                 350
   Lys Thr Lys Gly Gln Pro Arg Glu Pro Gln Val Tyr Thr Leu Pro Pro
           355                 360                 365
   Ser Arg Glu Glu Met Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Met Leu Asp Ser Asp
                   405                 410                 415
   Gly Ser Phe Phe Leu Tyr Ser Lys Leu Thr Val Asp Lys Ser Arg Trp
   420 425 430
   Gln Gln Gly Asn Val Phe Ser Cys Ser Val Met His Glu Ala Leu His
   435 440 445
   Asn His Tyr Thr Gln Lys Ser Leu Ser Leu Ser Pro Gly Lys
   450 455 460
<210> 113
<211> 469
<212> PRT
<213> Homo sapiens
<400> 113
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Ser Ser Tyr Val Met Asn Trp Val Arg Gln Ala Pro Gly Lys Gly Leu
       50                  55                  60
   Glu Trp Val Ser Phe Ile Ser Gly Asp Ser Ser Asn Thr Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Thr Phe Met His Gly His Leu Gly Gly Gly Leu
           115                 120                 125
   Ser Met Asp Phe Trp Gly Gln Gly Thr Leu Val Thr Val Ser Ser Ala
       130                 135                 140
   Ser Thr Lys Gly Pro Ser Val Phe Pro Leu Ala Pro Cys Ser Arg Ser
   145                 150                 155                 160
   Thr Ser Glu Ser Thr Ala Ala Leu Gly Cys Leu Val Lys Asp Tyr Phe
                   165                 170                 175
   Pro Glu Pro Val Thr Val Ser Trp Asn Ser Gly Ala Leu Thr Ser Gly
               180                 185                 190
   Val His Thr Phe Pro Ala Val Leu Gln Ser Ser Gly Leu Tyr Ser Leu
           195                 200                 205
   Ser Ser Val Val Thr Val Pro Ser Ser Asn Phe Gly Thr Gln Thr Tyr
       210                 215                 220
   Thr Cys Asn Val Asp His Lys Pro Ser Asn Thr Lys Val Asp Lys Thr
   225                 230                 235                 240
   Val Glu Arg Lys Cys Cys Val Glu Cys Pro Pro Cys Pro Ala Pro Pro
                   245                 250                 255
   Val Ala Gly Pro Ser Val Phe Leu Phe Pro Pro Lys Pro Lys Asp Thr
               260                 265                 270
   Leu Met Ile Ser Arg Thr Pro Glu Val Thr Cys Val Val Val Asp Val
           275                 280                 285
   Ser His Glu Asp Pro Glu Val Gln Phe Asn Trp Tyr Val Asp Gly Val
       290                 295                 300
   Glu Val His Asn Ala Lys Thr Lys Pro Arg Glu Glu Gln Phe Asn Ser
   305                 310                 315                 320
   Thr Phe Arg Val Val Ser Val Leu Thr Val Val His Gln Asp Trp Leu
                   325                 330                 335
   Asn Gly Lys Glu Tyr Lys Cys Lys Val Ser Asn Lys Gly Leu Pro Ala
               340                 345                 350
   Pro Ile Glu Lys Thr Ile Ser Lys Thr Lys Gly Gln Pro Arg Glu Pro
           355                 360                 365
   Gln Val Tyr Thr Leu Pro Pro Ser Arg Glu Glu Met Thr Lys Asn Gln
       370                 375                 380
   Val Ser Leu Thr Cys Leu Val Lys Gly Phe Tyr Pro Ser Asp Ile Ala
   385                 390                 395                 400
   Val Glu Trp Glu Ser Asn Gly Gln Pro Glu Asn Asn Tyr Lys Thr Thr
                   405                 410                 415
   Pro Pro Met Leu Asp Ser Asp Gly Ser Phe Phe Leu Tyr Ser Lys Leu
               420                 425                 430
   Thr Val Asp Lys Ser Arg Trp Gln Gln Gly Asn Val Phe Ser Cys Ser
           435                 440                 445
   Val Met His Glu Ala Leu His Asn His Tyr Thr Gln Lys Ser Leu Ser
       450                 455                 460
   Leu Ser Pro Gly Lys
   465
<210> 114
<211> 462
<212> PRT
<213> Homo sapiens
<400> 114
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Asn Ile Asn Tyr Asp Gly Ser Ser Thr Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Thr Tyr Leu His Phe Asp Tyr Trp Gly Gln
           115                 120                 125
   Gly Thr Leu Val Thr Val Ser Ser Ala Ser Thr Lys Gly Pro Ser Val
       130                 135                 140
   Phe Pro Leu Ala Pro Cys Ser Arg Ser Thr Ser Glu Ser Thr Ala Ala
   145                 150                 155                 160
   Leu Gly Cys Leu Val Lys Asp Tyr Phe Pro Glu Pro Val Thr Val Ser
                   165                 170                 175
   Trp Asn Ser Gly Ala Leu Thr Ser Gly Val His Thr Phe Pro Ala Val
               180                 185                 190
   Leu Gln Ser Ser Gly Leu Tyr Ser Leu Ser Ser Val Val Thr Val Pro
           195                 200                 205
   Ser Ser Asn Phe Gly Thr Gln Thr Tyr Thr Cys Asn Val Asp His Lys
       210                 215                 220
   Pro Ser Asn Thr Lys Val Asp Lys Thr Val Glu Arg Lys Cys Cys Val
   225                 230                 235                 240
   Glu Cys Pro Pro Cys Pro Ala Pro Pro Val Ala Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Gln Phe Asn Trp Tyr Val Asp Gly Val Glu Val His Asn Ala Lys Thr
       290                 295                 300
   Lys Pro Arg Glu Glu Gln Phe Asn Ser Thr Phe Arg Val Val Ser Val
   305                 310                 315                 320
   Leu Thr Val Val His Gln Asp Trp Leu Asn Gly Lys Glu Tyr Lys Cys
                   325                 330                 335
   Lys Val Ser Asn Lys Gly Leu Pro Ala Pro Ile Glu Lys Thr Ile Ser
               340                 345                 350
   Lys Thr Lys Gly Gln Pro Arg Glu Pro Gln Val Tyr Thr Leu Pro Pro
           355                 360                 365
   Ser Arg Glu Glu Met Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Met Leu Asp Ser Asp
                   405                 410                 415
   Gly Ser Phe Phe Leu Tyr Ser Lys Leu Thr Val Asp Lys Ser Arg Trp
               420                 425                 430
   Gln Gln Gly Asn Val Phe Ser Cys Ser Val Met His Glu Ala Leu His
           435                 440                 445
   Asn His Tyr Thr Gln Lys Ser Leu Ser Leu Ser Pro Gly Lys
       450                 455                 460
<210> 115
<211> 461
<212> PRT
<213> Homo sapiens
<400> 115
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Val Thr Gly Val His Gly Asp Thr Tyr Tyr Ala Asp
   65                  70                  75                  80
   Ser Val Lys Gly Arg Phe Thr Ile Ser Arg Asp Asn Ser Lys Asn Thr
                   85                  90                  95
   Leu Tyr Leu Gln Met Asn Ser Leu Arg Ala Glu Asp Thr Ala Val Tyr
               100                 105                 110
   Tyr Cys Ala Arg Asp Thr Tyr Leu His Phe Asp Tyr Trp Gly Gln Gly
           115                 120                 125
   Thr Leu Val Thr Val Ser Ser Ala Ser Thr Lys Gly Pro Ser Val Phe
       130                 135                 140
   Pro Leu Ala Pro Cys Ser Arg Ser Thr Ser Glu Ser Thr Ala Ala Leu
   145                 150                 155                 160
   Gly Cys Leu Val Lys Asp Tyr Phe Pro Glu Pro Val Thr Val Ser Trp
                   165                 170                 175
   Asn Ser Gly Ala Leu Thr Ser Gly Val His Thr Phe Pro Ala Val Leu
               180                 185                 190
   Gln Ser Ser Gly Leu Tyr Ser Leu Ser Ser Val Val Thr Val Pro Ser
           195                 200                 205
   Ser Asn Phe Gly Thr Gln Thr Tyr Thr Cys Asn Val Asp His Lys Pro
       210                 215                 220
   Ser Asn Thr Lys Val Asp Lys Thr Val Glu Arg Lys Cys Cys Val Glu
   225                 230                 235                 240
   Cys Pro Pro Cys Pro Ala Pro Pro Val Ala Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Gln
           275                 280                 285
   Phe Asn Trp Tyr Val Asp Gly Val Glu Val His Asn Ala Lys Thr Lys
       290                 295                 300
   Pro Arg Glu Glu Gln Phe Asn Ser Thr Phe Arg Val Val Ser Val Leu
   305                 310                 315                 320
   Thr Val Val His Gln Asp Trp Leu Asn Gly Lys Glu Tyr Lys Cys Lys
                   325                 330                 335
   Val Ser Asn Lys Gly Leu Pro Ala Pro Ile Glu Lys Thr Ile Ser Lys
               340                 345                 350
   Thr Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Met Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
       450                 455                 460
<210> 116
<211> 461
<212> PRT
<213> Homo sapiens
<400> 116
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Val Ile Gly Asn Trp Gly Asp Thr Tyr Tyr Ala Asp
   65                  70                  75                  80
   Ser Val Lys Gly Arg Phe Thr Ile Ser Arg Asp Asn Ser Lys Asn Thr
                   85                  90                  95
   Leu Tyr Leu Gln Met Asn Ser Leu Arg Ala Glu Asp Thr Ala Val Tyr
               100                 105                 110
   Tyr Cys Ala Arg Asp Thr Tyr Leu His Phe Asp Tyr Trp Gly Gln Gly
           115                 120                 125
   Thr Leu Val Thr Val Ser Ser Ala Ser Thr Lys Gly Pro Ser Val Phe
       130                 135                 140
   Pro Leu Ala Pro Cys Ser Arg Ser Thr Ser Glu Ser Thr Ala Ala Leu
   145                 150                 155                 160
   Gly Cys Leu Val Lys Asp Tyr Phe Pro Glu Pro Val Thr Val Ser Trp
                   165                 170                 175
   Asn Ser Gly Ala Leu Thr Ser Gly Val His Thr Phe Pro Ala Val Leu
               180                 185                 190
   Gln Ser Ser Gly Leu Tyr Ser Leu Ser Ser Val Val Thr Val Pro Ser
           195                 200                 205
   Ser Asn Phe Gly Thr Gln Thr Tyr Thr Cys Asn Val Asp His Lys Pro
       210                 215                 220
   Ser Asn Thr Lys Val Asp Lys Thr Val Glu Arg Lys Cys Cys Val Glu
   225                 230                 235                 240
   Cys Pro Pro Cys Pro Ala Pro Pro Val Ala Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Gln
           275                 280                 285
   Phe Asn Trp Tyr Val Asp Gly Val Glu Val His Asn Ala Lys Thr Lys
       290                 295                 300
   Pro Arg Glu Glu Gln Phe Asn Ser Thr Phe Arg Val Val Ser Val Leu
   305                 310                 315                 320
   Thr Val Val His Gln Asp Trp Leu Asn Gly Lys Glu Tyr Lys Cys Lys
                   325                 330                 335
   Val Ser Asn Lys Gly Leu Pro Ala Pro Ile Glu Lys Thr Ile Ser Lys
               340                 345                 350
   Thr Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Met Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
       450                 455                 460
<210> 117
<211> 461
<212> PRT
<213> Homo sapiens
<400> 117
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Val Thr Thr His Gln Gly Tyr Thr Tyr Tyr Ala Asp
   65                  70                  75                  80
   Ser Val Lys Gly Arg Phe Thr Ile Ser Arg Asp Asn Ser Lys Asn Thr
                   85                  90                  95
   Leu Tyr Leu Gln Met Asn Ser Leu Arg Ala Glu Asp Thr Ala Val Tyr
               100                 105                 110
   Tyr Cys Ala Arg Asp Thr Tyr Leu His Phe Asp Tyr Trp Gly Gln Gly
           115                 120                 125
   Thr Leu Val Thr Val Ser Ser Ala Ser Thr Lys Gly Pro Ser Val Phe
       130                 135                 140
   Pro Leu Ala Pro Cys Ser Arg Ser Thr Ser Glu Ser Thr Ala Ala Leu
   145                 150                 155                 160
   Gly Cys Leu Val Lys Asp Tyr Phe Pro Glu Pro Val Thr Val Ser Trp
                   165                 170                 175
   Asn Ser Gly Ala Leu Thr Ser Gly Val His Thr Phe Pro Ala Val Leu
               180                 185                 190
   Gln Ser Ser Gly Leu Tyr Ser Leu Ser Ser Val Val Thr Val Pro Ser
           195                 200                 205
   Ser Asn Phe Gly Thr Gln Thr Tyr Thr Cys Asn Val Asp His Lys Pro
       210                 215                 220
   Ser Asn Thr Lys Val Asp Lys Thr Val Glu Arg Lys Cys Cys Val Glu
   225                 230                 235                 240
   Cys Pro Pro Cys Pro Ala Pro Pro Val Ala Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Gln
           275                 280                 285
   Phe Asn Trp Tyr Val Asp Gly Val Glu Val His Asn Ala Lys Thr Lys
       290                 295                 300
   Pro Arg Glu Glu Gln Phe Asn Ser Thr Phe Arg Val Val Ser Val Leu
   305                 310                 315                 320
   Thr Val Val His Gln Asp Trp Leu Asn Gly Lys Glu Tyr Lys Cys Lys
                   325                 330                 335
   Val Ser Asn Lys Gly Leu Pro Ala Pro Ile Glu Lys Thr Ile Ser Lys
               340                 345                 350
   Thr Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Met Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
       450                 455                 460
<210> 118
<211> 465
<212> PRT
<213> Homo sapiens
<400> 118
   Met Lys His Leu Trp Phe Phe Leu Leu Leu Val Ala Ala Pro Arg Trp
   1               5                   10                  15
   Val Leu Ser Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Ala Thr Asn Arg Tyr Gly Tyr Thr Tyr Tyr Ala Asp
   65                  70                  75                  80
   Ser Val Lys Gly Arg Phe Thr Ile Ser Arg Asp Asn Ser Lys Asn Thr
                   85                  90                  95
   Leu Tyr Leu Gln Met Asn Ser Leu Arg Ala Glu Asp Thr Ala Val Tyr
               100                 105                 110
   Tyr Cys Ala Arg Asp Thr Tyr Leu His Phe Asp Tyr Trp Gly Gln Gly
           115                 120                 125
   Thr Leu Val Thr Val Ser Ser Ala Lys Thr Thr Ala Pro Ser Val Tyr
       130                 135                 140
   Pro Leu Ala Pro Val Cys Gly Asp Thr Thr Gly Ser Ser Val Thr Leu
   145                 150                 155                 160
   Gly Cys Leu Val Lys Gly Tyr Phe Pro Glu Pro Val Thr Leu Thr Trp
                   165                 170                 175
   Asn Ser Gly Ser Leu Ser Ser Gly Val His Thr Phe Pro Ala Val Leu
               180                 185                 190
   Gln Ser Asp Leu Tyr Thr Leu Ser Ser Ser Val Thr Val Thr Ser Ser
           195                 200                 205
   Thr Trp Pro Ser Gln Ser Ile Thr Cys Asn Val Ala His Pro Ala Ser
       210                 215                 220
   Ser Thr Lys Val Asp Lys Lys Ile Glu Pro Arg Gly Pro Thr Ile Lys
   225                 230                 235                 240
   Pro Cys Pro Pro Cys Lys Cys Pro Ala Pro Asn Leu Leu Gly Gly Pro
                   245                 250                 255
   Ser Val Phe Ile Phe Pro Pro Lys Ile Lys Asp Val Leu Met Ile Ser
               260                 265                 270
   Leu Ser Pro Ile Val Thr Cys Val Val Val Asp Val Ser Glu Asp Asp
           275                 280                 285
   Pro Asp Val Gln Ile Ser Trp Phe Val Asn Asn Val Glu Val His Thr
       290                 295                 300
   Ala Gln Thr Gln Thr His Arg Glu Asp Tyr Asn Ser Thr Leu Arg Val
   305                 310                 315                 320
   Val Ser Ala Leu Pro Ile Gln His Gln Asp Trp Met Ser Gly Lys Glu
                   325                 330                 335
   Phe Lys Cys Lys Val Asn Asn Lys Asp Leu Pro Ala Pro Ile Glu Arg
               340                 345                 350
   Thr Ile Ser Lys Pro Lys Gly Ser Val Arg Ala Pro Gln Val Tyr Val
           355                 360                 365
   Leu Pro Pro Pro Glu Glu Glu Met Thr Lys Lys Gln Val Thr Leu Thr
       370                 375                 380
   Cys Met Val Thr Asp Phe Met Pro Glu Asp Ile Tyr Val Glu Trp Thr
   385                 390                 395                 400
   Asn Asn Gly Lys Thr Glu Leu Asn Tyr Lys Asn Thr Glu Pro Val Leu
                   405                 410                 415
   Asp Ser Asp Gly Ser Tyr Phe Met Tyr Ser Lys Leu Arg Val Glu Lys
               420                 425                 430
   Lys Asn Trp Val Glu Arg Asn Ser Tyr Ser Cys Ser Val Val His Glu
           435                 440                 445
   Gly Leu His Asn His His Thr Thr Lys Ser Phe Ser Arg Thr Pro Gly
   450 455 460
   Lys
   465
<210> 119
<211> 462
<212> PRT
<213> Homo sapiens
<400> 119
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Asn Ile Asn Tyr Asp Gly Ser Ser Thr Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Asp Thr Tyr Leu His Phe Asp Tyr Trp Gly Gln
           115                 120                 125
   Gly Thr Leu Val Thr Val Ser Ser Ala Ser Thr Lys Gly Pro Ser Val
       130                 135                 140
   Phe Pro Leu Ala Pro Cys Ser Arg Ser Thr Ser Glu Ser Thr Ala Ala
   145                 150                 155                 160
   Leu Gly Cys Leu Val Lys Asp Tyr Phe Pro Glu Pro Val Thr Val Ser
                   165                 170                 175
   Trp Asn Ser Gly Ala Leu Thr Ser Gly Val His Thr Phe Pro Ala Val
               180                 185                 190
   Leu Gln Ser Ser Gly Leu Tyr Ser Leu Ser Ser Val Val Thr Val Pro
           195                 200                 205
   Ser Ser Asn Phe Gly Thr Gln Thr Tyr Thr Cys Asn Val Asp His Lys
       210                 215                 220
   Pro Ser Asn Thr Lys Val Asp Lys Thr Val Glu Arg Lys Cys Cys Val
   225                 230                 235                 240
   Glu Cys Pro Pro Cys Pro Ala Pro Pro Val Ala Gly Pro Ser Val Phe
                   245                 250                 255
   Leu Phe Pro Pro Lys Pro Lys Asp Thr Leu Met Ile Ser Arg Thr Pro
               260                 265                 270
   Glu Val Thr Cys Val Val Val Asp Val Ser His Glu Asp Pro Glu Val
           275                 280                 285
   Gln Phe Asn Trp Tyr Val Asp Gly Val Glu Val His Asn Ala Lys Thr
       290                 295                 300
   Lys Pro Arg Glu Glu Gln Phe Asn Ser Thr Phe Arg Val Val Ser Val
   305                 310                 315                 320
   Leu Thr Val Val His Gln Asp Trp Leu Asn Gly Lys Glu Tyr Lys Cys
                   325                 330                 335
   Lys Val Ser Asn Lys Gly Leu Pro Ala Pro Ile Glu Lys Thr Ile Ser
               340                 345                 350
   Lys Thr Lys Gly Gln Pro Arg Glu Pro Gln Val Tyr Thr Leu Pro Pro
           355                 360                 365
   Ser Arg Glu Glu Met Thr Lys Asn Gln Val Ser Leu Thr Cys Leu Val
       370                 375                 380
   Lys Gly Phe Tyr Pro Ser Asp Ile Ala Val Glu Trp Glu Ser Asn Gly
   385                 390                 395                 400
   Gln Pro Glu Asn Asn Tyr Lys Thr Thr Pro Pro Met Leu Asp Ser Asp
                   405                 410                 415
   Gly Ser Phe Phe Leu Tyr Ser Lys Leu Thr Val Asp Lys Ser Arg Trp
               420                 425                 430
   Gln Gln Gly Asn Val Phe Ser Cys Ser Val Met His Glu Ala Leu His
           435                 440                 445
   Asn His Tyr Thr Gln Lys Ser Leu Ser Leu Ser Pro Gly Lys
       450                 455                 460
<210> 120
<211> 461
<212> PRT
<213> Homo sapiens
<400> 120
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Val Ile Thr Pro Tyr Gly Asp Thr Tyr Tyr Ala Asp
   65                  70                  75                  80
   Ser Val Lys Gly Arg Phe Thr Ile Ser Arg Asp Asn Ser Lys Asn Thr
                   85                  90                  95
   Leu Tyr Leu Gln Met Asn Ser Leu Arg Ala Glu Asp Thr Ala Val Tyr
               100                 105                 110
   Tyr Cys Ala Arg Asp Thr Tyr Leu His Phe Asp Tyr Trp Gly Gln Gly
           115                 120                 125
   Thr Leu Val Thr Val Ser Ser Ala Ser Thr Lys Gly Pro Ser Val Phe
       130                 135                 140
   Pro Leu Ala Pro Cys Ser Arg Ser Thr Ser Glu Ser Thr Ala Ala Leu
   145                 150                 155                 160
   Gly Cys Leu Val Lys Asp Tyr Phe Pro Glu Pro Val Thr Val Ser Trp
                   165                 170                 175
   Asn Ser Gly Ala Leu Thr Ser Gly Val His Thr Phe Pro Ala Val Leu
               180                 185                 190
   Gln Ser Ser Gly Leu Tyr Ser Leu Ser Ser Val Val Thr Val Pro Ser
           195                 200                 205
   Ser Asn Phe Gly Thr Gln Thr Tyr Thr Cys Asn Val Asp His Lys Pro
       210                 215                 220
   Ser Asn Thr Lys Val Asp Lys Thr Val Glu Arg Lys Cys Cys Val Glu
   225                 230                 235                 240
   Cys Pro Pro Cys Pro Ala Pro Pro Val Ala Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Gln
           275                 280                 285
   Phe Asn Trp Tyr Val Asp Gly Val Glu Val His Asn Ala Lys Thr Lys
       290                 295                 300
   Pro Arg Glu Glu Gln Phe Asn Ser Thr Phe Arg Val Val Ser Val Leu
   305                 310                 315                 320
   Thr Val Val His Gln Asp Trp Leu Asn Gly Lys Glu Tyr Lys Cys Lys
                   325                 330                 335
   Val Ser Asn Lys Gly Leu Pro Ala Pro Ile Glu Lys Thr Ile Ser Lys
               340                 345                 350
   Thr Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Met Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
   450 455 460
<210> 121
<211> 461
<212> PRT
<213> Homo sapiens
<400> 121
   Met Ala Trp Val Trp Thr Leu Pro Phe Leu Met Ala Ala Ala Gln Ser
   1               5                   10                  15
   Val Gln Ala Gln Val Gln Leu Val Glu Ser Gly Gly Gly Leu Val Gln
               20                  25                  30
   Pro Gly Gly Ser Leu Arg Leu Ser Cys Ala Ala Ser Gly Phe Thr Phe
           35                  40                  45
   Arg Ser His Trp Leu Ser Trp Val Arg Gln Ala Pro Gly Lys Gly Leu
       50                  55                  60
   Glu Trp Val Ser Val Ile Thr Pro Tyr Gly Asp Thr Tyr Tyr Ala Asp
   65                  70                  75                  80
   Ser Val Lys Gly Arg Phe Thr Ile Ser Arg Asp Asn Ser Lys Asn Thr
                   85                  90                  95
   Leu Tyr Leu Gln Met Asn Ser Leu Arg Ala Glu Asp Thr Ala Val Tyr
               100                 105                 110
   Tyr Cys Ala Arg Asp Thr Tyr Leu His Phe Asp Tyr Trp Gly Gln Gly
           115                 120                 125
   Thr Leu Val Thr Val Ser Ser Ala Ser Thr Lys Gly Pro Ser Val Phe
       130                 135                 140
   Pro Leu Ala Pro Cys Ser Arg Ser Thr Ser Glu Ser Thr Ala Ala Leu
   145                 150                 155                 160
   Gly Cys Leu Val Lys Asp Tyr Phe Pro Glu Pro Val Thr Val Ser Trp
                   165                 170                 175
   Asn Ser Gly Ala Leu Thr Ser Gly Val His Thr Phe Pro Ala Val Leu
               180                 185                 190
   Gln Ser Ser Gly Leu Tyr Ser Leu Ser Ser Val Val Thr Val Pro Ser
           195                 200                 205
   Ser Asn Phe Gly Thr Gln Thr Tyr Thr Cys Asn Val Asp His Lys Pro
       210                 215                 220
   Ser Asn Thr Lys Val Asp Lys Thr Val Glu Arg Lys Cys Cys Val Glu
   225                 230                 235                 240
   Cys Pro Pro Cys Pro Ala Pro Pro Val Ala Gly Pro Ser Val Phe Leu
                   245                 250                 255
   Phe Pro Pro Lys Pro Lys Asp Thr Leu Met Ile Ser Arg Thr Pro Glu
               260                 265                 270
   Val Thr Cys Val Val Val Asp Val Ser His Glu Asp Pro Glu Val Gln
           275                 280                 285
   Phe Asn Trp Tyr Val Asp Gly Val Glu Val His Asn Ala Lys Thr Lys
       290                 295                 300
   Pro Arg Glu Glu Gln Phe Asn Ser Thr Phe Arg Val Val Ser Val Leu
   305                 310                 315                 320
   Thr Val Val His Gln Asp Trp Leu Asn Gly Lys Glu Tyr Lys Cys Lys
                   325                 330                 335
   Val Ser Asn Lys Gly Leu Pro Ala Pro Ile Glu Lys Thr Ile Ser Lys
               340                 345                 350
   Thr Lys Gly Gln Pro Arg Glu Pro Gln Val Tyr Thr Leu Pro Pro Ser
           355                 360                 365
   Arg Glu Glu Met Thr Lys Asn Gln Val Ser Leu Thr Cys Leu Val Lys
       370                 375                 380
   Gly Phe Tyr Pro Ser Asp Ile Ala Val Glu Trp Glu Ser Asn Gly Gln
   385                 390                 395                 400
   Pro Glu Asn Asn Tyr Lys Thr Thr Pro Pro Met Leu Asp Ser Asp Gly
                   405                 410                 415
   Ser Phe Phe Leu Tyr Ser Lys Leu Thr Val Asp Lys Ser Arg Trp Gln
               420                 425                 430
   Gln Gly Asn Val Phe Ser Cys Ser Val Met His Glu Ala Leu His Asn
           435                 440                 445
   His Tyr Thr Gln Lys Ser Leu Ser Leu Ser Pro Gly Lys
       450                 455                 460
<210> 122
<211> 130
<212> PRT
<213> Homo sapiens
<400> 122
   Met Ser Val Leu Thr Gln Val Leu Ala Leu Leu Leu Leu Trp Leu Thr
   1               5                   10                  15
   Gly Thr Arg Cys Asp Ile Glu Leu Thr Gln Pro Pro Ser Val Ser Val
               20                  25                  30
   Ala Pro Gly Gln Thr Ala Arg Ile Ser Cys Ser Gly Asp Asn Ile Gly
           35                  40                  45
   Ser Phe Tyr Val His Trp Tyr Gln Gln Lys Pro Gly Gln Ala Pro Val
       50                  55                  60
   Leu Val Ile Tyr Asp Asp Asn Asn Arg Pro Ser Gly Ile Pro Glu Arg
   65                  70                  75                  80
   Phe Ser Gly Ser Asn Ser Gly Asn Thr Ala Thr Leu Thr Ile Ser Gly
                   85                  90                  95
   Thr Gln Ala Glu Asp Glu Ala Asp Tyr Tyr Cys Gly Ser Trp Ala Gly
               100                 105                 110
   Ser Ser Gly Ser Tyr Val Phe Gly Gly Arg Thr Lys Leu Thr Val Leu
           115                 120                 125
   Gly Gln
       130
<210> 123
<211> 237
<212> PRT
<213> Homo sapiens
<400> 123
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Ser
               100                 105                 110
   Tyr Gly Glu Ser Leu Thr Ser Tyr Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 124
<211> 234
<212> PRT
<213> Homo sapiens
<400> 124
   Met Ser Val Leu Thr Gln Val Leu Ala Leu Leu Leu Leu Trp Leu Thr
   1               5                   10                  15
   Gly Thr Arg Cys Asp Ile Glu Leu Thr Gln Pro Pro Ser Val Ser Val
               20                  25                  30
   Ala Pro Gly Gln Thr Ala Arg Ile Ser Cys Ser Gly Asp Asn Ile Gly
           35                  40                  45
   Ser Phe Tyr Val His Trp Tyr Gln Gln Lys Pro Gly Gln Ala Pro Val
       50                  55                  60
   Leu Val Ile Tyr Asp Asp Asn Asn Arg Pro Ser Gly Ile Pro Glu Arg
   65                  70                  75                  80
   Phe Ser Gly Ser Asn Ser Gly Asn Thr Ala Thr Leu Thr Ile Ser Gly
                   85                  90                  95
   Thr Gln Ala Glu Asp Glu Ala Asp Tyr Tyr Cys Ala Ser Trp Thr Gly
               100                 105                 110
   Val Glu Pro Asp Tyr Val Phe Gly Gly Gly Thr Lys Leu Thr Val Leu
           115                 120                 125
   Gly Gln Pro Lys Ala Ala Pro Ser Val Thr Leu Phe Pro Pro Ser Ser
       130                 135                 140
   Glu Glu Leu Gln Ala Asn Lys Ala Thr Leu Val Cys Leu Ile Ser Asp
   145                 150                 155                 160
   Phe Tyr Pro Gly Ala Val Thr Val Ala Trp Lys Ala Asp Ser Ser Pro
                   165                 170                 175
   Val Lys Ala Gly Val Glu Thr Thr Thr Pro Ser Lys Gln Ser Asn Asn
               180                 185                 190
   Lys Tyr Ala Ala Ser Ser Tyr Leu Ser Leu Thr Pro Glu Gln Trp Lys
           195                 200                 205
   Ser His Arg Ser Tyr Ser Cys Gln Val Thr His Glu Gly Ser Thr Val
       210                 215                 220
   Glu Lys Thr Val Ala Pro Thr Glu Cys Ser
   225                 230
<210> 125
<211> 237
<212> PRT
<213> Homo sapiens
<400> 125
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Gln Ser
               100                 105                 110
   Tyr Ala Gly Ser Tyr Leu Ser Glu Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 126
<211> 237
<212> PRT
<213> Homo sapiens
<400> 126
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Ser
               100                 105                 110
   Tyr Gly Glu Ser Leu Thr Ser Tyr Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 127
<211> 237
<212> PRT
<213> Homo sapiens
<400> 127
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Ser
               100                 105                 110
   Tyr Gly Glu Ser Leu Thr Ser Tyr Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 128
<211> 237
<212> PRT
<213> Homo sapiens
<400> 128
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Ser
               100                 105                 110
   Tyr Gly Glu Ser Leu Thr Ser Tyr Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 129
<211> 237
<212> PRT
<213> Homo sapiens
<400> 129
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Ser
               100                 105                 110
   Tyr Gly Glu Ser Leu Thr Ser Tyr Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 130
<211> 237
<212> PRT
<213> Homo sapiens
<400> 130
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Thr
               100                 105                 110
   Tyr Asp Gly Pro Gly Leu Ser Glu Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 131
<211> 237
<212> PRT
<213> Homo sapiens
<400> 131
   Met Ser Val Leu Thr Gln Val Leu Ala Leu Leu Leu Leu Trp Leu Thr
   1               5                   10                  15
   Gly Thr Arg Cys Asp Ile Ala Leu Thr Gln Pro Ala Ser Val Ser Gly
               20                  25                  30
   Ser Pro Gly Gln Ser Ile Thr Ile Ser Cys Thr Gly Thr Ser Ser Asp
           35                  40                  45
   Val Gly Asp Ile Asn Asp Val Ser Trp Tyr Gln Gln His Pro Gly Lys
       50                  55                  60
   Ala Pro Lys Leu Met Ile Tyr Asp Val Asn Asn Arg Pro Ser Gly Val
   65                  70                  75                  80
   Ser Asn Arg Phe Ser Gly Ser Lys Ser Gly Asn Thr Ala Ser Leu Thr
                   85                  90                  95
   Ile Ser Gly Leu Gln Ala Glu Asp Glu Ala Asp Tyr Tyr Cys Ser Ser
               100                 105                 110
   Tyr Gly Glu Ser Leu Thr Ser Tyr Val Phe Gly Gly Gly Thr Lys Leu
           115                 120                 125
   Thr Val Leu Gly Gln Pro Lys Ala Ala Pro Ser Val Thr Leu Phe Pro
       130                 135                 140
   Pro Ser Ser Glu Glu Leu Gln Ala Asn Lys Ala Thr Leu Val Cys Leu
   145                 150                 155                 160
   Ile Ser Asp Phe Tyr Pro Gly Ala Val Thr Val Ala Trp Lys Ala Asp
                   165                 170                 175
   Ser Ser Pro Val Lys Ala Gly Val Glu Thr Thr Thr Pro Ser Lys Gln
               180                 185                 190
   Ser Asn Asn Lys Tyr Ala Ala Ser Ser Tyr Leu Ser Leu Thr Pro Glu
           195                 200                 205
   Gln Trp Lys Ser His Arg Ser Tyr Ser Cys Gln Val Thr His Glu Gly
       210                 215                 220
   Ser Thr Val Glu Lys Thr Val Ala Pro Thr Glu Cys Ser
   225                 230                 235
<210> 132
<211> 237
<212> PRT
<213> Homo sapiens
<400> 132
   Met Ser Val Leu Thr Gln Val Leu Ala Leu Leu Leu Leu Trp Leu Thr
   1 5 10 15
   Gly Thr Arg Cys Asp Ile Ala Leu Thr Gln Pro Ala Ser Val Ser Gly
   20 25 30
   Ser Pro Gly Gln Ser Ile Thr Ile Ser Cys Thr Gly Thr Ser Ser Asp
   35 40 45
   Val Gly Asp Ile Asn Asp Val Ser Trp Tyr Gln Gln His Pro Gly Lys
   50 55 60
   Ala Pro Lys Leu Met Ile Tyr Asp Val Asn Asn Arg Pro Ser Gly Val
   65 70 75 80
   Ser Asn Arg Phe Ser Gly Ser Lys Ser Gly Asn Thr Ala Ser Leu Thr
   85 90 95
   Ile Ser Gly Leu Gln Ala Glu Asp Glu Ala Asp Tyr Tyr Cys Ser Thr
   100 105 110
   Tyr Asp Gly Pro Gly Leu Ser Glu Val Phe Gly Gly Gly Thr Lys Leu
   115 120 125
   Thr Val Leu Gly Gln Pro Lys Ala Ala Pro Ser Val Thr Leu Phe Pro
   130 135 140
   Pro Ser Ser Glu Glu Leu Gln Ala Asn Lys Ala Thr Leu Val Cys Leu
   145 150 155 160
   Ile Ser Asp Phe Tyr Pro Gly Ala Val Thr Val Ala Trp Lys Ala Asp
   165 170 175
   Ser Ser Pro Val Lys Ala Gly Val Glu Thr Thr Thr Pro Ser Lys Gln
   180 185 190
   Ser Asn Asn Lys Tyr Ala Ala Ser Ser Tyr Leu Ser Leu Thr Pro Glu
   195 200 205
   Gln Trp Lys Ser His Arg Ser Tyr Ser Cys Gln Val Thr His Glu Gly
   210 215 220
   Ser Thr Val Glu Lys Thr Val Ala Pro Thr Glu Cys Ser
   225 230 235
<210> 133
<211> 1410
<212> DNA
<213> Homo sapiens
<400> 133
   atggcttggg tgtggacctt gccattcctg atggcagctg cccaaagcgt gcaggcccag   60
   gtgcagctgg tcgagtctgg cggcggactg gtgcagcctg gcggcagcct gagactgagc  120
   tgcgccgcca gcggcttcac cttcagcagc tacgtgatga actgggtgcg gcaggcccct  180
   ggcaagggcc tggagtgggt gtccttcatc agcggcgaca gcagcaacac ctactacgcc  240
   gacagcgtga agggccggtt caccatcagc cgggacaaca gcaagaacac cctgtacctg  300
   cagatgaaca gcctgcgggc cgaggacacc gccgtgtact actgcgcccg gaccttcatg  360
   cacggccacc tgggcggagg actgagcatg gatttctggg gccagggcac cctggtcacc  420
   gtctcctcag cttccaccaa gggcccatcc gtcttccccc tggcgccctg ctccaggagc  480
   acctccgaga gcacagcggc cctgggctgc ctggtcaagg actacttccc cgaaccggtg  540
   acggtgtcgt ggaactcagg cgctctgacc agcggcgtgc acaccttccc agctgtccta  600
   cagtcctcag gactctactc cctcagcagc gtggtgacag tgccctccag caacttcggc  660
   acccagacct acacctgcaa cgtagatcac aagcccagca acaccaaggt ggacaagaca  720
   gttgagcgca aatgttgtgt cgagtgccca ccgtgcccag caccacctgt ggcaggaccg  780
   tcagtcttcc tcttcccccc aaaacccaag gacaccctca tgatctcccg gacccctgag  840
   gtcacgtgcg tggtggtgga cgtgagccac gaagaccccg aggtccagtt caactggtac  900
   gtggacggcg tggaggtgca taatgccaag acaaagccac gggaggagca gttcaacagc  960
   acgttccgtg tggtcagcgt cctcaccgtt gtgcaccagg actggctgaa cggcaaggag 1020
   tacaagtgca aggtctccaa caaaggcctc ccagccccca tcgagaaaac catctccaaa 1080
   accaaagggc agccccgaga accacaggtg tacaccctgc ccccatcccg ggaggagatg 1140
   accaagaacc aggtcagcct gacctgcctg gtcaaaggct tctaccccag cgacatcgcc 1200
   gtggagtggg agagcaatgg gcagccggag aacaactaca agaccacacc tcccatgctg 1260
   gactccgacg gctccttctt cctctacagc aagctcaccg tggacaagag caggtggcag 1320
   caggggaacg tcttctcatg ctccgtgatg catgaggctc tgcacaacca ctacacgcag 1380
   aagagcctct ccctgtctcc gggtaaatga                                   1410
<210> 134
<211> 1389
<212> DNA
<213> Homo sapiens
<400> 134
   atggcttggg tgtggacctt gccattcctg atggcagctg cccaaggtgt ccaggcccag   60
   gtgcagctgg tcgagagcgg cggagggctg gtgcagcctg gcggcagcct gagactgagc  120
   tgcgccgcca gcggcttcac cttcagaagc cactggctgt cctgggtgcg gcaggcccct  180
   ggcaagggcc tggaatgggt gtccaacatc aactacgacg gcagcagcac ctactacgcc  240
   gacagcgtga agggccggtt caccatcagc cgggacaaca gcaagaacac cctgtacctg  300
   cagatgaaca gcctgcgggc cgaggacacc gccgtgtact actgcgccag ggacacctac  360
   ctgcacttcg actactgggg ccagggcacc ctggtcaccg tctcctcagc ttccaccaag  420
   ggcccatccg tcttccccct ggcgccctgc tccaggagca cctccgagag cacagcggcc  480
   ctgggctgcc tggtcaagga ctacttcccc gaaccggtga cggtgtcgtg gaactcaggc  540
   gctctgacca gcggcgtgca caccttccca gctgtcctac agtcctcagg actctactcc  600
   ctcagcagcg tggtgacagt gccctccagc aacttcggca cccagaccta cacctgcaac  660
   gtagatcaca agcccagcaa caccaaggtg gacaagacag ttgagcgcaa atgttgtgtc  720
   gagtgcccac cgtgcccagc accacctgtg gcaggaccgt cagtcttcct cttcccccca  780
   aaacccaagg acaccctcat gatctcccgg acccctgagg tcacgtgcgt ggtggtggac  840
   gtgagccacg aagaccccga ggtccagttc aactggtacg tggacggcgt ggaggtgcat  900
   aatgccaaga caaagccacg ggaggagcag ttcaacagca cgttccgtgt ggtcagcgtc  960
   ctcaccgttg tgcaccagga ctggctgaac ggcaaggagt acaagtgcaa ggtctccaac 1020
   aaaggcctcc cagcccccat cgagaaaacc atctccaaaa ccaaagggca gccccgagaa 1080
   ccacaggtgt acaccctgcc cccatcccgg gaggagatga ccaagaacca ggtcagcctg 1140
   acctgcctgg tcaaaggctt ctaccccagc gacatcgccg tggagtggga gagcaatggg 1200
   cagccggaga acaactacaa gaccacacct cccatgctgg actccgacgg ctccttcttc 1260
   ctctacagca agctcaccgt ggacaagagc aggtggcagc aggggaacgt cttctcatgc 1320
   tccgtgatgc atgaggctct gcacaaccac tacacgcaga agagcctctc cctgtctccg 1380
   ggtaaatga                                                         1389
<210> 135
<211> 1410
<212> DNA
<213> Homo sapiens
<400> 135
   atggcttggg tgtggacctt gccattcctg atggcagctg cccaaagcgt gcaggcccag   60
   gtgcagctgg tcgagtctgg cggcggactg gtgcagcctg gcggcagcct gagactgagc  120
   tgcgccgcca gcggcttcac cttcagcagc tacgtgatga actgggtgcg gcaggcccct  180
   ggcaagggcc tggagtgggt gtccttcatc agcggcgaca gcagcaacac ctactacgcc  240
   gacagcgtga agggccggtt caccatcagc cgggacaaca gcaagaacac cctgtacctg  300
   cagatgaaca gcctgcgggc cgaggacacc gccgtgtact actgcgcccg gaccttcatg  360
   cacggccacc tgggcggagg actgagcatg gatttctggg gccagggcac cctggtcacc  420
   gtctcctcag cttccaccaa gggcccatcc gtcttccccc tggcgccctg ctccaggagc  480
   acctccgaga gcacagcggc cctgggctgc ctggtcaagg actacttccc cgaaccggtg  540
   acggtgtcgt ggaactcagg cgctctgacc agcggcgtgc acaccttccc agctgtccta  600
   cagtcctcag gactctactc cctcagcagc gtggtgacag tgccctccag caacttcggc  660
   acccagacct acacctgcaa cgtagatcac aagcccagca acaccaaggt ggacaagaca  720
   gttgagcgca aatgttgtgt cgagtgccca ccgtgcccag caccacctgt ggcaggaccg  780
   tcagtcttcc tcttcccccc aaaacccaag gacaccctca tgatctcccg gacccctgag  840
   gtcacgtgcg tggtggtgga cgtgagccac gaagaccccg aggtccagtt caactggtac  900
   gtggacggcg tggaggtgca taatgccaag acaaagccac gggaggagca gttcaacagc  960
   acgttccgtg tggtcagcgt cctcaccgtt gtgcaccagg actggctgaa cggcaaggag 1020
   tacaagtgca aggtctccaa caaaggcctc ccagccccca tcgagaaaac catctccaaa 1080
   accaaagggc agccccgaga accacaggtg tacaccctgc ccccatcccg ggaggagatg 1140
   accaagaacc aggtcagcct gacctgcctg gtcaaaggct tctaccccag cgacatcgcc 1200
   gtggagtggg agagcaatgg gcagccggag aacaactaca agaccacacc tcccatgctg 1260
   gactccgacg gctccttctt cctctacagc aagctcaccg tggacaagag caggtggcag 1320
   caggggaacg tcttctcatg ctccgtgatg catgaggctc tgcacaacca ctacacgcag 1380
   aagagcctct ccctgtctcc gggtaaatga                                  1410
<210> 136
<211> 1389
<212> DNA
<213> Homo sapiens
<400> 136
   atggcttggg tgtggacctt gccattcctg atggcagctg cccaaggtgt ccaggcccag   60
   gtgcagctgg tcgagagcgg cggagggctg gtgcagcctg gcggcagcct gagactgagc  120
   tgcgccgcca gcggcttcac cttcagaagc cactggctgt cctgggtgcg gcaggcccct  180
   ggcaagggcc tggaatgggt gtccaacatc aactacgacg gcagcagcac ctactacgcc  240
   gacagcgtga agggccggtt caccatcagc cgggacaaca gcaagaacac cctgtacctg  300
   cagatgaaca gcctgcgggc cgaggacacc gccgtgtact actgcgccag ggacacctac  360
   ctgcacttcg actactgggg ccagggcacc ctggtcaccg tctcctcagc ttccaccaag  420
   ggcccatccg tcttccccct ggcgccctgc tccaggagca cctccgagag cacagcggcc  480
   ctgggctgcc tggtcaagga ctacttcccc gaaccggtga cggtgtcgtg gaactcaggc  540
   gctctgacca gcggcgtgca caccttccca gctgtcctac agtcctcagg actctactcc  600
   ctcagcagcg tggtgacagt gccctccagc aacttcggca cccagaccta cacctgcaac  660
   gtagatcaca agcccagcaa caccaaggtg gacaagacag ttgagcgcaa atgttgtgtc  720
   gagtgcccac cgtgcccagc accacctgtg gcaggaccgt cagtcttcct cttcccccca  780
   aaacccaagg acaccctcat gatctcccgg acccctgagg tcacgtgcgt ggtggtggac  840
   gtgagccacg aagaccccga ggtccagttc aactggtacg tggacggcgt ggaggtgcat  900
   aatgccaaga caaagccacg ggaggagcag ttcaacagca cgttccgtgt ggtcagcgtc  960
   ctcaccgttg tgcaccagga ctggctgaac ggcaaggagt acaagtgcaa ggtctccaac 1020
   aaaggcctcc cagcccccat cgagaaaacc atctccaaaa ccaaagggca gccccgagaa 1080
   ccacaggtgt acaccctgcc cccatcccgg gaggagatga ccaagaacca ggtcagcctg 1140
   acctgcctgg tcaaaggctt ctaccccagc gacatcgccg tggagtggga gagcaatggg 1200
   cagccggaga acaactacaa gaccacacct cccatgctgg actccgacgg ctccttcttc 1260
   ctctacagca agctcaccgt ggacaagagc aggtggcagc aggggaacgt cttctcatgc 1320
   tccgtgatgc atgaggctct gcacaaccac tacacgcaga agagcctctc cctgtctccg 1380
   ggtaaatga                                                         1389
<210> 137
<211> 1386
<212> DNA
<213> Homo sapiens
<400> 137
   atggcttggg tgtggacctt gccattcctg atggcagctg cccaaagtgt ccaggcccag  60
   gtgcagctgg tcgagagcgg cggagggctg gtgcagcctg gcggcagcct gagactgagc  120
   tgcgccgcca gcggcttcac cttcagaagc cactggctgt cctgggtgcg gcaggcccct  180
   ggcaagggcc tggaatgggt gtccgtgacc ggcgtgcacg gcgacaccta ctacgccgac  240
   agcgtgaagg gccggttcac catcagccgg gacaacagca agaacaccct gtacctgcag  300
   atgaacagcc tgcgggccga ggacaccgcc gtgtactact gcgccaggga cacctacctg  360
   cacttcgact actggggcca gggcaccctg gtcaccgtct cctcagcttc caccaagggc  420
   ccatccgtct tccccctggc gccctgctcc aggagcacct ccgagagcac agcggccctg  480
   ggctgcctgg tcaaggacta cttccccgaa ccggtgacgg tgtcgtggaa ctcaggcgct  540
   ctgaccagcg gcgtgcacac cttcccagct gtcctacagt cctcaggact ctactccctc  600
   agcagcgtgg tgacagtgcc ctccagcaac ttcggcaccc agacctacac ctgcaacgta  660
   gatcacaagc ccagcaacac caaggtggac aagacagttg agcgcaaatg ttgtgtcgag  720
   tgcccaccgt gcccagcacc acctgtggca ggaccgtcag tcttcctctt ccccccaaaa  780
   cccaaggaca ccctcatgat ctcccggacc cctgaggtca cgtgcgtggt ggtggacgtg  840
   agccacgaag accccgaggt ccagttcaac tggtacgtgg acggcgtgga ggtgcataat  900
   gccaagacaa agccacggga ggagcagttc aacagcacgt tccgtgtggt cagcgtcctc  960
   accgttgtgc accaggactg gctgaacggc aaggagtaca agtgcaaggt ctccaacaaa  1020
   ggcctcccag cccccatcga gaaaaccatc tccaaaacca aagggcagcc ccgagaacca  1080
   caggtgtaca ccctgccccc atcccgggag gagatgacca agaaccaggt cagcctgacc  1140
   tgcctggtca aaggcttcta ccccagcgac atcgccgtgg agtgggagag caatgggcag  1200
   ccggagaaca actacaagac cacacctccc atgctggact ccgacggctc cttcttcctc  1260
   tacagcaagc tcaccgtgga caagagcagg tggcagcagg ggaacgtctt ctcatgctcc  1320
   gtgatgcatg aggctctgca caaccactac acgcagaaga gcctctccct gtctccgggt  1380
   aaatga                                                             1386
<210> 138
<211> 1386
<212> DNA
<213> Homo sapiens
<400> 138
   atggcttggg tgtggacctt gccattcctg atggcagctg cccaaagtgt ccaggcccag 60
   gtgcagctgg tcgagagcgg cggagggctg gtgcagcctg gcggcagcct gagactgagc 120
   tgcgccgcca gcggcttcac cttcagaagc cactggctgt cctgggtgcg gcaggcccct 180
   ggcaagggcc tggaatgggt gtccgtgatc ggcaactggg gcgacaccta ctacgccgac 240
   agcgtgaagg gccggttcac catcagccgg gacaacagca agaacaccct gtacctgcag 300
   atgaacagcc tgcgggccga ggacaccgcc gtgtactact gcgccaggga cacctacctg 360
   cacttcgact actggggcca gggcaccctg gtcaccgtct cctcagcttc caccaagggc 420
   ccatccgtct tccccctggc gccctgctcc aggagcacct ccgagagcac agcggccctg 480
   ggctgcctgg tcaaggacta cttccccgaa ccggtgacgg tgtcgtggaa ctcaggcgct 540
   ctgaccagcg gcgtgcacac cttcccagct gtcctacagt cctcaggact ctactccctc 600
   agcagcgtgg tgacagtgcc ctccagcaac ttcggcaccc agacctacac ctgcaacgta 660
   gatcacaagc ccagcaacac caaggtggac aagacagttg agcgcaaatg ttgtgtcgag 720
   tgcccaccgt gcccagcacc acctgtggca ggaccgtcag tcttcctctt ccccccaaaa 780
   cccaaggaca ccctcatgat ctcccggacc cctgaggtca cgtgcgtggt ggtggacgtg 840
   agccacgaag accccgaggt ccagttcaac tggtacgtgg acggcgtgga ggtgcataat 900
   gccaagacaa agccacggga ggagcagttc aacagcacgt tccgtgtggt cagcgtcctc 960
   accgttgtgc accaggactg gctgaacggc aaggagtaca agtgcaaggt ctccaacaaa 1020
   ggcctcccag cccccatcga gaaaaccatc tccaaaacca aagggcagcc ccgagaacca 1080
   caggtgtaca ccctgccccc atcccgggag gagatgacca agaaccaggt cagcctgacc 1140
   tgcctggtca aaggcttcta ccccagcgac atcgccgtgg agtgggagag caatgggcag 1200
   ccggagaaca actacaagac cacacctccc atgctggact ccgacggctc cttcttcctc 1260
   tacagcaagc tcaccgtgga caagagcagg tggcagcagg ggaacgtctt ctcatgctcc 1320
   gtgatgcatg aggctctgca caaccactac acgcagaaga gcctctccct gtctccgggt 1380
   aaatga 1386
<210> 139
<211> 1386
<212> DNA
<213> Homo sapiens
<400> 139
   atggcttggg tgtggacctt gccattcctg atggcagctg cccaaagtgt ccaggcccag 60
   gtgcagctgg tcgagagcgg cggagggctg gtgcagcctg gcggcagcct gagactgagc 120
   tgcgccgcca gcggcttcac cttcagaagc cactggctgt cctgggtgcg gcaggcccct 180
   ggcaagggcc tggaatgggt gtccgtgacc acccaccagg gctacaccta ctacgccgac 240
   agcgtgaagg gccggttcac catcagccgg gacaacagca agaacaccct gtacctgcag 300
   atgaacagcc tgcgggccga ggacaccgcc gtgtactact gcgccaggga cacctacctg 360
   cacttcgact actggggcca gggcaccctg gtcaccgtct cctcagcttc caccaagggc 420
   ccatccgtct tccccctggc gccctgctcc aggagcacct ccgagagcac agcggccctg 480
   ggctgcctgg tcaaggacta cttccccgaa ccggtgacgg tgtcgtggaa ctcaggcgct 540
   ctgaccagcg gcgtgcacac cttcccagct gtcctacagt cctcaggact ctactccctc 600
   agcagcgtgg tgacagtgcc ctccagcaac ttcggcaccc agacctacac ctgcaacgta 660
   gatcacaagc ccagcaacac caaggtggac aagacagttg agcgcaaatg ttgtgtcgag 720
   tgcccaccgt gcccagcacc acctgtggca ggaccgtcag tcttcctctt ccccccaaaa 780
   cccaaggaca ccctcatgat ctcccggacc cctgaggtca cgtgcgtggt ggtggacgtg 840
   agccacgaag accccgaggt ccagttcaac tggtacgtgg acggcgtgga ggtgcataat 900
   gccaagacaa agccacggga ggagcagttc aacagcacgt tccgtgtggt cagcgtcctc 960
   accgttgtgc accaggactg gctgaacggc aaggagtaca agtgcaaggt ctccaacaaa 1020
   ggcctcccag cccccatcga gaaaaccatc tccaaaacca aagggcagcc ccgagaacca 1080
   caggtgtaca ccctgccccc atcccgggag gagatgacca agaaccaggt cagcctgacc 1140
   tgcctggtca aaggcttcta ccccagcgac atcgccgtgg agtgggagag caatgggcag 1200
   ccggagaaca actacaagac cacacctccc atgctggact ccgacggctc cttcttcctc 1260
   tacagcaagc tcaccgtgga caagagcagg tggcagcagg ggaacgtctt ctcatgctcc 1320
   gtgatgcatg aggctctgca caaccactac acgcagaaga gcctctccct gtctccgggt 1380
   aaatga 1386
<210> 140
<211> 1386
<212> DNA
<213> Homo sapiens
<400> 140
   atggcttggg tgtggacctt gccattcctg atggcagctg cccaaagtgt ccaggcccag  60
   gtgcagctgg tcgagagcgg cggagggctg gtgcagcctg gcggcagcct gagactgagc  120
   tgcgccgcca gcggcttcac cttcagaagc cactggctgt cctgggtgcg gcaggcccct  180
   ggcaagggcc tggaatgggt gtccgccacc aacagatacg gctacaccta ctacgccgac  240
   agcgtgaagg gccggttcac catcagccgg gacaacagca agaacaccct gtacctgcag  300
   atgaacagcc tgcgggccga ggacaccgcc gtgtactact gcgccaggga cacctacctg  360
   cacttcgact actggggcca gggcaccctg gtcaccgtct cctcagcttc caccaagggc  420
   ccatccgtct tccccctggc gccctgctcc aggagcacct ccgagagcac agcggccctg  480
   ggctgcctgg tcaaggacta cttccccgaa ccggtgacgg tgtcgtggaa ctcaggcgct  540
   ctgaccagcg gcgtgcacac cttcccagct gtcctacagt cctcaggact ctactccctc  600
   agcagcgtgg tgacagtgcc ctccagcaac ttcggcaccc agacctacac ctgcaacgta  660
   gatcacaagc ccagcaacac caaggtggac aagacagttg agcgcaaatg ttgtgtcgag  720
   tgcccaccgt gcccagcacc acctgtggca ggaccgtcag tcttcctctt ccccccaaaa  780
   cccaaggaca ccctcatgat ctcccggacc cctgaggtca cgtgcgtggt ggtggacgtg  840
   agccacgaag accccgaggt ccagttcaac tggtacgtgg acggcgtgga ggtgcataat  900
   gccaagacaa agccacggga ggagcagttc aacagcacgt tccgtgtggt cagcgtcctc  960
   accgttgtgc accaggactg gctgaacggc aaggagtaca agtgcaaggt ctccaacaaa  1020
   ggcctcccag cccccatcga gaaaaccatc tccaaaacca aagggcagcc ccgagaacca  1080
   caggtgtaca ccctgccccc atcccgggag gagatgacca agaaccaggt cagcctgacc  1140
   tgcctggtca aaggcttcta ccccagcgac atcgccgtgg agtgggagag caatgggcag  1200
   ccggagaaca actacaagac cacacctccc atgctggact ccgacggctc cttcttcctc  1260
   tacagcaagc tcaccgtgga caagagcagg tggcagcagg ggaacgtctt ctcatgctcc  1320
   gtgatgcatg aggctctgca caaccactac acgcagaaga gcctctccct gtctccgggt  1380
   aaatga                                                              1386
<210> 141
<211> 1389
<212> DNA
<213> Homo sapiens
<400> 141
   atggcttggg tgtggacctt gccattcctg atggcagctg cccaaggtgt ccaggcccag  60
   gtgcagctgg tcgagagcgg cggagggctg gtgcagcctg gcggcagcct gagactgagc  120
   tgcgccgcca gcggcttcac cttcagaagc cactggctgt cctgggtgcg gcaggcccct  180
   ggcaagggcc tggaatgggt gtccaacatc aactacgacg gcagcagcac ctactacgcc  240
   gacagcgtga agggccggtt caccatcagc cgggacaaca gcaagaacac cctgtacctg  300
   cagatgaaca gcctgcgggc cgaggacacc gccgtgtact actgcgccag ggacacctac  360
   ctgcacttcg actactgggg ccagggcacc ctggtcaccg tctcctcagc ttccaccaag  420
   ggcccatccg tcttccccct ggcgccctgc tccaggagca cctccgagag cacagcggcc  480
   ctgggctgcc tggtcaagga ctacttcccc gaaccggtga cggtgtcgtg gaactcaggc  540
   gctctgacca gcggcgtgca caccttccca gctgtcctac agtcctcagg actctactcc  600
   ctcagcagcg tggtgacagt gccctccagc aacttcggca cccagaccta cacctgcaac  660
   gtagatcaca agcccagcaa caccaaggtg gacaagacag ttgagcgcaa atgttgtgtc  720
   gagtgcccac cgtgcccagc accacctgtg gcaggaccgt cagtcttcct cttcccccca  780
   aaacccaagg acaccctcat gatctcccgg acccctgagg tcacgtgcgt ggtggtggac  840
   gtgagccacg aagaccccga ggtccagttc aactggtacg tggacggcgt ggaggtgcat  900
   aatgccaaga caaagccacg ggaggagcag ttcaacagca cgttccgtgt ggtcagcgtc  960
   ctcaccgttg tgcaccagga ctggctgaac ggcaaggagt acaagtgcaa ggtctccaac  1020
   aaaggcctcc cagcccccat cgagaaaacc atctccaaaa ccaaagggca gccccgagaa  1080
   ccacaggtgt acaccctgcc cccatcccgg gaggagatga ccaagaacca ggtcagcctg  1140
   acctgcctgg tcaaaggctt ctaccccagc gacatcgccg tggagtggga gagcaatggg  1200
   cagccggaga acaactacaa gaccacacct cccatgctgg actccgacgg ctccttcttc  1260
   ctctacagca agctcaccgt ggacaagagc aggtggcagc aggggaacgt cttctcatgc  1320
   tccgtgatgc atgaggctct gcacaaccac tacacgcaga agagcctctc cctgtctccg  1380
   ggtaaatga                                                           1389
<210> 142
<211> 1386
<212> DNA
<213> Homo sapiens
<400> 142
   atggcttggg tgtggacctt gccattcctg atggcagctg cccaaagtgt ccaggcccag 60
   gtgcagctgg tcgagagcgg cggagggctg gtgcagcctg gcggcagcct gagactgagc 120
   tgcgccgcca gcggcttcac cttcagaagc cactggctgt cctgggtgcg gcaggcccct 180
   ggcaagggcc tggaatgggt gtccgtgatc accccctacg gcgacaccta ctacgccgac 240
   agcgtgaagg gccggttcac catcagccgg gacaacagca agaacaccct gtacctgcag 300
   atgaacagcc tgcgggccga ggacaccgcc gtgtactact gcgccaggga cacctacctg 360
   cacttcgact actggggcca gggcaccctg gtcaccgtct cctcagcttc caccaagggc 420
   ccatccgtct tccccctggc gccctgctcc aggagcacct ccgagagcac agcggccctg 480
   ggctgcctgg tcaaggacta cttccccgaa ccggtgacgg tgtcgtggaa ctcaggcgct 540
   ctgaccagcg gcgtgcacac cttcccagct gtcctacagt cctcaggact ctactccctc 600
   agcagcgtgg tgacagtgcc ctccagcaac ttcggcaccc agacctacac ctgcaacgta 660
   gatcacaagc ccagcaacac caaggtggac aagacagttg agcgcaaatg ttgtgtcgag 720
   tgcccaccgt gcccagcacc acctgtggca ggaccgtcag tcttcctctt ccccccaaaa 780
   cccaaggaca ccctcatgat ctcccggacc cctgaggtca cgtgcgtggt ggtggacgtg 840
   agccacgaag accccgaggt ccagttcaac tggtacgtgg acggcgtgga ggtgcataat 900
   gccaagacaa agccacggga ggagcagttc aacagcacgt tccgtgtggt cagcgtcctc 960
   accgttgtgc accaggactg gctgaacggc aaggagtaca agtgcaaggt ctccaacaaa 1020
   ggcctcccag cccccatcga gaaaaccatc tccaaaacca aagggcagcc ccgagaacca 1080
   caggtgtaca ccctgccccc atcccgggag gagatgacca agaaccaggt cagcctgacc 1140
   tgcctggtca aaggcttcta ccccagcgac atcgccgtgg agtgggagag caatgggcag 1200
   ccggagaaca actacaagac cacacctccc atgctggact ccgacggctc cttcttcctc 1260
   tacagcaagc tcaccgtgga caagagcagg tggcagcagg ggaacgtctt ctcatgctcc 1320
   gtgatgcatg aggctctgca caaccactac acgcagaaga gcctctccct gtctccgggt 1380
   aaatga 1386
<210> 143
<211> 1386
<212> DNA
<213> Homo sapiens
<400> 143
   atggcttggg tgtggacctt gccattcctg atggcagctg cccaaagtgt ccaggcccag  60
   gtgcagctgg tcgagagcgg cggagggctg gtgcagcctg gcggcagcct gagactgagc  120
   tgcgccgcca gcggcttcac cttcagaagc cactggctgt cctgggtgcg gcaggcccct  180
   ggcaagggcc tggaatgggt gtccgtgatc accccctacg gcgacaccta ctacgccgac  240
   agcgtgaagg gccggttcac catcagccgg gacaacagca agaacaccct gtacctgcag  300
   atgaacagcc tgcgggccga ggacaccgcc gtgtactact gcgccaggga cacctacctg  360
   cacttcgact actggggcca gggcaccctg gtcaccgtct cctcagcttc caccaagggc  420
   ccatccgtct tccccctggc gccctgctcc aggagcacct ccgagagcac agcggccctg  480
   ggctgcctgg tcaaggacta cttccccgaa ccggtgacgg tgtcgtggaa ctcaggcgct  540
   ctgaccagcg gcgtgcacac cttcccagct gtcctacagt cctcaggact ctactccctc  600
   agcagcgtgg tgacagtgcc ctccagcaac ttcggcaccc agacctacac ctgcaacgta  660
   gatcacaagc ccagcaacac caaggtggac aagacagttg agcgcaaatg ttgtgtcgag  720
   tgcccaccgt gcccagcacc acctgtggca ggaccgtcag tcttcctctt ccccccaaaa  780
   cccaaggaca ccctcatgat ctcccggacc cctgaggtca cgtgcgtggt ggtggacgtg  840
   agccacgaag accccgaggt ccagttcaac tggtacgtgg acggcgtgga ggtgcataat  900
   gccaagacaa agccacggga ggagcagttc aacagcacgt tccgtgtggt cagcgtcctc  960
   accgttgtgc accaggactg gctgaacggc aaggagtaca agtgcaaggt ctccaacaaa  1020
   ggcctcccag cccccatcga gaaaaccatc tccaaaacca aagggcagcc ccgagaacca  1080
   caggtgtaca ccctgccccc atcccgggag gagatgacca agaaccaggt cagcctgacc  1140
   tgcctggtca aaggcttcta ccccagcgac atcgccgtgg agtgggagag caatgggcag  1200
   ccggagaaca actacaagac cacacctccc atgctggact ccgacggctc cttcttcctc  1260
   tacagcaagc tcaccgtgga caagagcagg tggcagcagg ggaacgtctt ctcatgctcc  1320
   gtgatgcatg aggctctgca caaccactac acgcagaaga gcctctccct gtctccgggt  1380
   aaatga                                                              1386
<210> **144**
<211> 393
<212> DNA
<213> Homo sapiens
<400> 144
   atgaaaaaga cagctatcgc gattgcagtg gcactggctg gtttcgctac cgtagcgcag  60
   gccgatatcg aactgaccca gccgccttca gtgagcgttg caccaggtca gaccgcgcgt  120
   atctcgtgta gcggcgataa tattggttct ttttatgttc attggtacca gcagaaaccc  180
   gggcaggcgc cagttcttgt gatttatgat gataataatc gtccctcagg catcccggaa  240
   cgctttagcg gatccaacag cggcaacacc gcgaccctga ccattagcgg cactcaggcg  300
   gaagacgaag cggattatta ttgcggttct tgggctggtt cttctggttc ttatgtgttt  360
   ggcggccgca cgaagttaac cgttcttggc cag                                 393
<210> 145
<211> 714
<212> DNA
<213> Homo sapiens
<400> 145
   atgagtgtgc tcactcaggt cctggcgttg ctgctgctgt ggcttacagg tacgcgttgc  60
   gacatcgccc tgacccagcc cgccagcgtg agcggcagcc ctggccagag catcaccatc  120
   agctgcaccg gcaccagcag cgacgtgggc gacatcaacg acgtgagctg gtatcagcag  180
   caccccggca aggcccccaa gctgatgatc tacgacgtga acaaccggcc cagcggcgtg  240
   agcaaccggt tcagcggcag caagagcggc aacaccgcca gcctgaccat cagcggcctc  300
   caggccgagg acgaggccga ctactactgc agcagctacg gcgagagcct gaccagctac  360
   gtgtttggcg gcggaaccaa gcttaccgtc ctaggtcagc ccaaggctgc cccctcggtc  420
   actctgttcc cgccctcctc tgaggagctt caagccaaca aggccacact ggtgtgtctc  480
   ataagtgact tctacccggg agccgtgaca gtggcctgga aggcagatag cagccccgtc  540
   aaggcgggag tggagacaac cacaccctcc aaacaaagca acaacaagta cgcggccagc  600
   agctatctga gcctgacgcc tgagcagtgg aagtcccaca gaagctacag ctgccaggtc  660
   acgcatgaag ggagcaccgt ggaaaagaca gtggccccta cagaatgttc atag          714
<210> 146
<211> 705
<212> DNA
<213> Homo sapiens
<400> 146
   atgagtgtgc tcactcaggt cctggcgttg ctgctgctgt ggcttacagg tacgcgttgc  60
   gacatcgagc tgacccagcc ccccagcgtg agcgtggccc ctggccagac cgcccggatc  120
   agctgcagcg gcgacaacat cggcagcttc tacgtgcact ggtatcagca gaagcccggc  180
   caggcccccg tgctggtgat ctacgacgac aacaaccggc ccagcggcat ccccgagcgg  240
   ttcagcggca gcaacagcgg caacaccgcc accctgacca tcagcggcac ccaggccgag  300
   gacgaggccg actactactg cgccagctgg accggcgtgg agcccgacta cgtgtttggc  360
   ggcggaacaa agcttaccgt cctaggtcag cccaaggctg ccccctcggt cactctgttc  420
   ccgccctcct ctgaggagct tcaagccaac aaggccacac tggtgtgtct cataagtgac  480
   ttctacccgg gagccgtgac agtggcctgg aaggcagata gcagccccgt caaggcggga  540
   gtggagacaa ccacaccctc caaacaaagc aacaacaagt acgcggccag cagctatctg  600
   agcctgacgc ctgagcagtg gaagtcccac agaagctaca gctgccaggt cacgcatgaa  660
   gggagcaccg tggaaaagac agtggcccct acagaatgtt catag                 705
<210> 147
<211> 714
<212> DNA
<213> Homo sapiens
<400> 147
   atgagtgtgc tcactcaggt cctggcgttg ctgctgctgt ggcttacagg tacgcgttgc  60
   gacatcgccc tgacccagcc cgccagcgtg agcggcagcc ctggccagag catcaccatc  120
   agctgcaccg gcaccagcag cgacgtgggc gacatcaacg acgtgagctg gtatcagcag  180
   caccccggca aggcccccaa gctgatgatc tacgacgtga acaaccggcc cagcggcgtg  240
   agcaaccggt tcagcggcag caagagcggc aacaccgcca gcctgaccat cagcggcctc  300
   caggccgagg acgaggccga ctactactgc cagagctacg ccggcagcta cctgagcgag  360
   gtgttcggcg gagggaccaa gcttaccgtc ctaggtcagc ccaaggctgc cccctcggtc  420
   actctgttcc cgccctcctc tgaggagctt caagccaaca aggccacact ggtgtgtctc  480
   ataagtgact tctacccggg agccgtgaca gtggcctgga aggcagatag cagccccgtc  540
   aaggcgggag tggagacaac cacaccctcc aaacaaagca acaacaagta cgcggccagc  600
   agctatctga gcctgacgcc tgagcagtgg aagtcccaca gaagctacag ctgccaggtc  660
   acgcatgaag ggagcaccgt ggaaaagaca gtggccccta cagaatgttc atag       714
<210> 148
<211> 714
<212> DNA
<213> Homo sapiens
<400> 148
   atgagtgtgc tcactcaggt cctggcgttg ctgctgctgt ggcttacagg tacgcgttgc  60
   gacatcgccc tgacccagcc cgccagcgtg agcggcagcc ctggccagag catcaccatc  120
   agctgcaccg gcaccagcag cgacgtgggc gacatcaacg acgtgagctg gtatcagcag  180
   caccccggca aggcccccaa gctgatgatc tacgacgtga acaaccggcc cagcggcgtg  240
   agcaaccggt tcagcggcag caagagcggc aacaccgcca gcctgaccat cagcggcctc  300
   caggccgagg acgaggccga ctactactgc agcagctacg gcgagagcct gaccagctac  360
   gtgtttggcg gcggaaccaa gcttaccgtc ctaggtcagc ccaaggctgc cccctcggtc  420
   actctgttcc cgccctcctc tgaggagctt caagccaaca aggccacact ggtgtgtctc  480
   ataagtgact tctacccggg agccgtgaca gtggcctgga aggcagatag cagccccgtc  540
   aaggcgggag tggagacaac cacaccctcc aaacaaagca acaacaagta cgcggccagc  600
   agctatctga gcctgacgcc tgagcagtgg aagtcccaca gaagctacag ctgccaggtc  660
   acgcatgaag ggagcaccgt ggaaaagaca gtggccccta cagaatgttc atag       714
<210> 149
<211> 714
<212> DNA
<213> Homo sapiens
<400> 149
   atgagtgtgc tcactcaggt cctggcgttg ctgctgctgt ggcttacagg tacgcgttgc  60
   gacatcgccc tgacccagcc cgccagcgtg agcggcagcc ctggccagag catcaccatc  120
   agctgcaccg gcaccagcag cgacgtgggc gacatcaacg acgtgagctg gtatcagcag  180
   caccccggca aggcccccaa gctgatgatc tacgacgtga acaaccggcc cagcggcgtg  240
   agcaaccggt tcagcggcag caagagcggc aacaccgcca gcctgaccat cagcggcctc  300
   caggccgagg acgaggccga ctactactgc agcagctacg gcgagagcct gaccagctac  360
   gtgtttggcg gcggaaccaa gcttaccgtc ctaggtcagc ccaaggctgc cccctcggtc  420
   actctgttcc cgccctcctc tgaggagctt caagccaaca aggccacact ggtgtgtctc  480
   ataagtgact tctacccggg agccgtgaca gtggcctgga aggcagatag cagccccgtc  540
   aaggcgggag tggagacaac cacaccctcc aaacaaagca acaacaagta cgcggccagc  600
   agctatctga gcctgacgcc tgagcagtgg aagtcccaca gaagctacag ctgccaggtc  660
   acgcatgaag ggagcaccgt ggaaaagaca gtggccccta cagaatgttc atag        714
<210> 150
<211> 714
<212> DNA
<213> Homo sapiens
<400> 150
   atgagtgtgc tcactcaggt cctggcgttg ctgctgctgt ggcttacagg tacgcgttgc  60
   gacatcgccc tgacccagcc cgccagcgtg agcggcagcc ctggccagag catcaccatc  120
   agctgcaccg gcaccagcag cgacgtgggc gacatcaacg acgtgagctg gtatcagcag  180
   caccccggca aggcccccaa gctgatgatc tacgacgtga acaaccggcc cagcggcgtg  240
   agcaaccggt tcagcggcag caagagcggc aacaccgcca gcctgaccat cagcggcctc  300
   caggccgagg acgaggccga ctactactgc agcagctacg gcgagagcct gaccagctac  360
   gtgtttggcg gcggaaccaa gcttaccgtc ctaggtcagc ccaaggctgc cccctcggtc  420
   actctgttcc cgccctcctc tgaggagctt caagccaaca aggccacact ggtgtgtctc  480
   ataagtgact tctacccggg agccgtgaca gtggcctgga aggcagatag cagccccgtc  540
   aaggcgggag tggagacaac cacaccctcc aaacaaagca acaacaagta cgcggccagc  600
   agctatctga gcctgacgcc tgagcagtgg aagtcccaca gaagctacag ctgccaggtc  660
   acgcatgaag ggagcaccgt ggaaaagaca gtggccccta cagaatgttc atag        714
<210> 151
<211> 714
<212> DNA
<213> Homo sapiens
<400> 151
   atgagtgtgc tcactcaggt cctggcgttg ctgctgctgt ggcttacagg tacgcgttgc  60
   gacatcgccc tgacccagcc cgccagcgtg agcggcagcc ctggccagag catcaccatc  120
   agctgcaccg gcaccagcag cgacgtgggc gacatcaacg acgtgagctg gtatcagcag  180
   caccccggca aggcccccaa gctgatgatc tacgacgtga acaaccggcc cagcggcgtg  240
   agcaaccggt tcagcggcag caagagcggc aacaccgcca gcctgaccat cagcggcctc  300
   caggccgagg acgaggccga ctactactgc agcagctacg gcgagagcct gaccagctac  360
   gtgtttggcg gcggaaccaa gcttaccgtc ctaggtcagc ccaaggctgc cccctcggtc  420
   actctgttcc cgccctcctc tgaggagctt caagccaaca aggccacact ggtgtgtctc  480
   ataagtgact tctacccggg agccgtgaca gtggcctgga aggcagatag cagccccgtc  540
   aaggcgggag tggagacaac cacaccctcc aaacaaagca acaacaagta cgcggccagc  600
   agctatctga gcctgacgcc tgagcagtgg aagtcccaca gaagctacag ctgccaggtc  660
   acgcatgaag ggagcaccgt ggaaaagaca gtggccccta cagaatgttc atag        714
<210> 152
<211> 714
<212> DNA
<213> Homo sapiens
<400> 152
   atgagtgtgc tcactcaggt cctggcgttg ctgctgctgt ggcttacagg tacgcgttgc 60
   gacatcgccc tgacccagcc cgccagcgtg agcggcagcc ctggccagag catcaccatc 120
   agctgcaccg gcaccagcag cgacgtgggc gacatcaacg acgtgagctg gtatcagcag 180
   caccccggca aggcccccaa gctgatgatc tacgacgtga acaaccggcc cagcggcgtg 240
   agcaaccggt tcagcggcag caagagcggc aacaccgcca gcctgaccat cagcggcctc 300
   caggccgagg acgaggccga ctactactgc agcacctacg acggccctgg cctgagcgag 360
   gtgttcggcg gagggaccaa gcttaccgtc ctaggtcagc ccaaggctgc cccctcggtc 420
   actctgttcc cgccctcctc tgaggagctt caagccaaca aggccacact ggtgtgtctc 480
   ataagtgact tctacccggg agccgtgaca gtggcctgga aggcagatag cagccccgtc 540
   aaggcgggag tggagacaac cacaccctcc aaacaaagca acaacaagta cgcggccagc 600
   agctatctga gcctgacgcc tgagcagtgg aagtcccaca gaagctacag ctgccaggtc 660
   acgcatgaag ggagcaccgt ggaaaagaca gtggccccta cagaatgttc atag       714
<210> 153
<211> 714
<212> DNA
<213> Homo sapiens
<400> 153
   atgagtgtgc tcactcaggt cctggcgttg ctgctgctgt ggcttacagg tacgcgttgc 60
   gacatcgccc tgacccagcc cgccagcgtg agcggcagcc ctggccagag catcaccatc 120
   agctgcaccg gcaccagcag cgacgtgggc gacatcaacg acgtgagctg gtatcagcag 180
   caccccggca aggcccccaa gctgatgatc tacgacgtga acaaccggcc cagcggcgtg 240
   agcaaccggt tcagcggcag caagagcggc aacaccgcca gcctgaccat cagcggcctc 300
   caggccgagg acgaggccga ctactactgc agcacctacg acggccctgg cctgagcgag 360
   gtgttcggcg gagggaccaa gcttaccgtc ctaggtcagc ccaaggctgc cccctcggtc 420
   actctgttcc cgccctcctc tgaggagctt caagccaaca aggccacact ggtgtgtctc 480
   ataagtgact tctacccggg agccgtgaca gtggcctgga aggcagatag cagccccgtc 540
   aaggcgggag tggagacaac cacaccctcc aaacaaagca acaacaagta cgcggccagc 600
   agctatctga gcctgacgcc tgagcagtgg aagtcccaca gaagctacag ctgccaggtc 660
   acgcatgaag ggagcaccgt ggaaaagaca gtggccccta cagaatgttc atag       714
<210> 154
<211> 714
<212> DNA
<213> Homo sapiens
<400> 154
   atgagtgtgc tcactcaggt cctggcgttg ctgctgctgt ggcttacagg tacgcgttgc 60
   gacatcgccc tgacccagcc cgccagcgtg agcggcagcc ctggccagag catcaccatc 120
   agctgcaccg gcaccagcag cgacgtgggc gacatcaacg acgtgagctg gtatcagcag 180
   caccccggca aggcccccaa gctgatgatc tacgacgtga acaaccggcc cagcggcgtg 240
   agcaaccggt tcagcggcag caagagcggc aacaccgcca gcctgaccat cagcggcctc 300
   caggccgagg acgaggccga ctactactgc agcacctacg acggccctgg cctgagcgag 360
   gtgttcggcg gagggaccaa gcttaccgtc ctaggtcagc ccaaggctgc cccctcggtc 420
   actctgttcc cgccctcctc tgaggagctt caagccaaca aggccacact ggtgtgtctc 480
   ataagtgact tctacccggg agccgtgaca gtggcctgga aggcagatag cagccccgtc 540
   aaggcgggag tggagacaac cacaccctcc aaacaaagca acaacaagta cgcggccagc 600
   agctatctga gcctgacgcc tgagcagtgg aagtcccaca gaagctacag ctgccaggtc 660
   acgcatgaag ggagcaccgt ggaaaagaca gtggccccta cagaatgttc atag       714
<210> 155
<211> 213
<212> PRT
<213> Homo sapiens
<400> 155
   Met Gln Leu Pro Leu Ala Leu Cys Leu Val Cys Leu Leu Val His Thr
   1               5                   10                  15
   Ala Phe Arg Val Val Glu Gly Gln Gly Trp Gln Ala Phe Lys Asn Asp
               20                  25                  30
   Ala Thr Glu Ile Ile Pro Glu Leu Gly Glu Tyr Pro Glu Pro Pro Pro
           35                  40                  45
   Glu Leu Glu Asn Asn Lys Thr Met Asn Arg Ala Glu Asn Gly Gly Arg
       50                  55                  60
   Pro Pro His His Pro Phe Glu Thr Lys Asp Val Ser Glu Tyr Ser Cys
   65                  70                  75                  80
   Arg Glu Leu His Phe Thr Arg Tyr Val Thr Asp Gly Pro Cys Arg Ser
                   85                  90                  95
   Ala Lys Pro Val Thr Glu Leu Val Cys Ser Gly Gln Cys Gly Pro Ala
               100                 105                 110
   Arg Leu Leu Pro Asn Ala Ile Gly Arg Gly Lys Trp Trp Arg Pro Ser
           115                 120                 125
   Gly Pro Asp Phe Arg Cys Ile Pro Asp Arg Tyr Arg Ala Gln Arg Val
       130                 135                 140
   Gln Leu Leu Cys Pro Gly Gly Glu Ala Pro Arg Ala Arg Lys Val Arg
   145                 150                 155                 160
   Leu Val Ala Ser Cys Lys Cys Lys Arg Leu Thr Arg Phe His Asn Gln
                   165                 170                 175
   Ser Glu Leu Lys Asp Phe Gly Thr Glu Ala Ala Arg Pro Gln Lys Gly
               180                 185                 190
   Arg Lys Pro Arg Pro Arg Ala Arg Ser Ala Lys Ala Asn Gln Ala Glu
           195                 200                 205
   Leu Glu Asn Ala Tyr
       210
<210> 156
<211> 14
<212> PRT
<213> Homo sapiens
<400> 156
   Ala Arg Leu Leu Asn Ala Ile Gly Arg Gly Lys Trp Trp Arg
   1               5                   10
<210> 157
<211> 15
<212> PRT
<213> Homo sapiens
<400> 157
   Arg Leu Val Ala Ser Cys Lys Cys Lys Arg Leu Thr Arg Phe His
   1               5                   10                  15
<210> 158
<211> 13
<212> PRT
<213> Artificial Sequence
<220>
<223> Artificial sequence
<400> 158
   Gly Gly Gly Ser Gly Gly Gly Gly Ser Gly Gly Gly Gly
   1 5 10
<210> 159
<211> 19
<212> PRT
<213> Artificial Sequence
<220>
<223> Artificial sequence
<400> 159
   Gly Gly Gly Gly Ser Gly Gly Gly Gly Ser Gly Gly Gly Gly Ser Gly
   1 5 10 15
   Gly Gly Gly
<210> 160
<211> 21
<212> DNA
<213> Artificial Sequence
<220>
<223> Artificial sequence
<400> 160
   taaattatca taaagtccta a 21
<210> 161
<211> 21
<212> DNA
<213> Artificial Sequence
<220>
<223> Artificial sequence
<400> 161
   aggactttat gataatttat 121
<210> 162
<211> 21
<212> DNA
<213> Artificial Sequence
<220>
<223> Artificial sequence
<400> 162
   atagtggtta aataactcca g 21
<210> 163
<211> 21
<212> DNA
<213> Artificial Sequence
<220>
<223> Artificial sequence
<400> 163
   ggagttattt aaccactatt 121
<210> 164
<211> 21
<212> DNA
<213> Artificial Sequence
<220>
<223> Artificial sequence
<400> 164
   taaattctcg tgatgtgcca 121
<210> 165
<211> 21
<212> DNA
<213> Artificial Sequence
<220>
<223> Artificial sequence
<400> 165
   ggcacatcac gagaatttat 121
<210> 166
<211> 21
<212> DNA
<213> Artificial Sequence
<220>
<223> Artificial sequence
<400> 166
   tttcttatag cacagctggt 121
<210> 167
<211> 21
<212> DNA
<213> Artificial Sequence
<220>
<223> Artificial sequence
<400> 167
   ccagctgtgc tataagaaat 121
<210> 168
<211> 21
<212> DNA
<213> Artificial Sequence
<220>
<223> Artificial sequence
<400> 168
   tagacctttc catccacgct g 21
<210> 169
<211> 21
<212> DNA
<213> Artificial Sequence
<220>
<223> Artificial sequence
<400> 169
   gcgtggatgg aaaggtctat 121
<210> 170
<211> 29
<212> DNA
<213> Artificial Sequence
<220>
<223> Artificial sequence
<400> 170
   atgcagctcc cactggccct gtgtcttgt 29
<210> 171
<211> 30
<212> DNA
<213> Artificial Sequence
<220>
<223> Artificial sequence
<400> 171
   aatcaggccg agctggagaa cgcctactag 30
<210> 172
<211> 443
<212> PRT
<213> Homo sapiens
<400> 172
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser His
               20                  25                  30
   Trp Leu Ser Trp Val Arg Gln Ala Pro Gly Lys Gly Leu Glu Trp Val
           35                  40                  45
   Ser Asn Ile Asn Tyr Asp Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Thr Tyr Leu His Phe Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Cys Ser Arg Ser Thr Ser Glu Ser Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Asn
   180 185 190
   Phe Gly Thr Gln Thr Tyr Thr Cys Asn Val Asp His Lys Pro Ser Asn
   195 200 205
   Thr Lys Val Asp Lys Thr Val Glu Arg Lys Cys Cys Val Glu Cys Pro
   210 215 220
   Pro Cys Pro Ala Pro Pro Val Ala Gly Pro Ser Val Phe Leu Phe Pro
   225 230 235 240
   Pro Lys Pro Lys Asp Thr Leu Met Ile Ser Arg Thr Pro Glu Val Thr
   245 250 255
   Cys Val Val Val Asp Val Ser His Glu Asp Pro Glu Val Gln Phe Asn
   260 265 270
   Trp Tyr Val Asp Gly Val Glu Val His Asn Ala Lys Thr Lys Pro Arg
   275 280 285
   Glu Glu Gln Phe Asn Ser Thr Phe Arg Val Val Ser Val Leu Thr Val
   290 295 300
   Val His Gln Asp Trp Leu Asn Gly Lys Glu Tyr Lys Cys Lys Val Ser
   305 310 315 320
   Asn Lys Gly Leu Pro Ala Pro Ile Glu Lys Thr Ile Ser Lys Thr Lys
   325 330 335
   Gly Gln Pro Arg Glu Pro Gln Val Tyr Thr Leu Pro Pro Ser Arg Glu
   340 345 350
   Glu Met Thr Lys Asn Gln Val Ser Leu Thr Cys Leu Val Lys Gly Phe
   355 360 365
   Tyr Pro Ser Asp Ile Ala Val Glu Trp Glu Ser Asn Gly Gln Pro Glu
   370 375 380
   Asn Asn Tyr Lys Thr Thr Pro Pro Met Leu Asp Ser Asp Gly Ser Phe
   385 390 395 400
   Phe Leu Tyr Ser Lys Leu Thr Val Asp Lys Ser Arg Trp Gln Gln Gly
   405 410 415
   Asn Val Phe Ser Cys Ser Val Met His Glu Ala Leu His Asn His Tyr
   420 425 430
   Thr Gln Lys Ser Leu Ser Leu Ser Pro Gly Lys
   435 440
<210> 173
<211> 217
<212> PRT
<213> Artificial Sequence
<220>
<223> Homo sapiens
<400> 173
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Asp Ile
               20                  25                  30
   Asn Asp Val Ser Trp Tyr Gln Gln His Pro Gly Lys Ala Pro Lys Leu
           35                  40                  45
   Met Ile Tyr Asp Val Asn Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Tyr Ala Gly Ser
                   85                  90                  95
   Tyr Leu Ser Glu Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Cys Ser
       210                 215
<210> 174
<211> 26
<212> PRT
<213> Homo sapiens
<400> 174
   Cys Gly Pro Ala Arg Leu Leu Pro Asn Ala Ile Gly Arg Gly Lys Trp
   1               5                   10                  15
   Trp Arg Pro Ser Gly Pro Asp Phe Arg Cys
               20                  25
<210> 175
<211> 60
<212> PRT
<213> Homo sapiens
<400> 175
   Asp Val Ser Glu Tyr Cys Arg Glu Leu His Phe Thr Arg Ser Ala Lys
   1               5                   10                  15
   Pro Val Thr Glu Leu Val Cys Ser Gly Gln Cys Gly Pro Ala Arg Trp
               20                  25                  30
   Trp Arg Pro Ser Gly Pro Asp Phe Arg Cys Ile Pro Asp Arg Tyr Arg
           35                  40                  45
   Leu Val Ala Ser Cys Lys Cys Lys Arg Leu Thr Arg
       50                  55                  60
<210> 176
<211> 133
<212> PRT
<213> Artificial Sequence
<220>
<223> Synthetic Polypeptide
<220>
<221> MISC_FEATURE
<223> Humanized Antibody Sequence
<400> 176
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Ser Pro Ser Ser
               20                  25                  30
   Leu Ser Ala Ser Val Gly Asp Arg Val Thr Ile Thr Cys Gln Ser Ser
           35                  40                  45
   Gln Ser Val Tyr Asp Asn Asn Trp Leu Ala Trp Tyr Gln Gln Lys Pro
       50                  55                  60
   Gly Lys Ala Pro Lys Leu Leu Ile Tyr Asp Ala Ser Asp Leu Ala Ser
   65                  70                  75                  80
   Gly Val Pro Ser Arg Phe Ser Gly Ser Gly Ser Gly Thr Asp Phe Thr
                   85                  90                  95
   Leu Thr Ile Ser Ser Leu Gln Pro Glu Asp Phe Ala Thr Tyr Tyr Cys
               100                 105                 110
   Gln Gly Ala Tyr Asn Asp Val Ile Tyr Ala Phe Gly Gln Gly Thr Lys
           115                 120                 125
   Val Glu Ile Lys Arg
       130
<210> 177
<211> 131
<212> PRT
<213> Artificial Sequence
<220>
<223> Synthetic Polypeptide
<220>
<221> MISC_FEATURE
<223> Humanized Antibody Sequence
<400> 177
   Met Glu Thr Gly Leu Arg Trp Leu Leu Leu Val Ala Val Leu Lys Gly
   1               5                   10                  15
   Val His Cys Glu Val Gln Leu Leu Glu Ser Gly Gly Gly Leu Val Gln
               20                  25                  30
   Pro Gly Gly Ser Leu Arg Leu Ser Cys Ala Ala Ser Gly Phe Ser Leu
           35                  40                  45
   Ser Ser Tyr Trp Met Asn Trp Val Arg Gln Ala Pro Gly Lys Gly Leu
       50                  55                  60
   Glu Trp Val Gly Thr Ile Asp Ser Gly Gly Arg Thr Asp Tyr Ala Ser
   65                  70                  75                  80
   Trp Ala Lys Gly Arg Phe Thr Ile Ser Arg Asp Asn Ser Lys Asn Thr
                   85                  90                  95
   Met Tyr Leu Gln Met Asn Ser Leu Arg Ala Glu Asp Thr Ala Arg Tyr
               100                 105                 110
   Tyr Cys Ala Arg Asn Trp Asn Leu Trp Gly Gln Gly Thr Leu Val Thr
           115                 120                 125
   Val Ser Ser
       130